# EUROPEAN PATENT SPECIFICATION

(11) **EP 4 171 622 B1**
(45) Date of publication and mention of the grant of the patent: **01.05.2024**
(21) Application number: 22718597.2
(22) Date of filing: 23.03.2022
(51) Int. Cl.: A61K 39/00, C12N 5/10, A61K 35/17, A61P 35/00, C07K 19/00, C12N 15/87

(54) **COMPOSITIONS COMPRISING NKG2D, CXCR2, AND DAP10/DAP12 FUSION POLYPEPTIDES AND METHODS OF USE THEREOF**
ZUSAMMENSETZUNGEN MIT NKG2D, CXCR2 UND DAP10/DAP12 FUSIONSPOLYPEPTIDEN UND VERFAHREN ZUR VERWENDUNG DAVON
COMPOSITIONS COMPRENANT DES POLYPEPTIDES DE FUSION NKG2D, CXCR2 ET DAP10/DAP12 ET LEURS PROCÉDÉS D'UTILISATION

(30) Priority: 23.03.2021 US 202163164959 P; 11.12.2021 US 202163288593 P
(43) Date of publication of application: 03.05.2023
(73) Proprietor: King's College London, London WC2R 2LS (GB)
(72) Inventor: MAHER, John, London WC2R 2LS (GB); DAVIES, David Marc, London WC2R 2LS (GB); LARCOMBE-YOUNG, Daniel, London WC2R 2LS (GB)
(74) Representative: Greaves Brewster LLP
(86) International application number: PCT/EP2022/057653
(87) International publication number: WO 2022/200442

(56) References cited:
- CHANG Y-H ET AL: "A Chimeric Receptor with NKG2D Specificity Enhances Natural Killer Cell Activation and Killing of Tumor Cells", CANCER RESEARCH, vol. 73, no. 6, 15 March 2013 (2013-03-15) , pages 1777-1786, XP055262100, ISSN: 0008-5472, DOI: 10.1158/0008-5472.CAN-12-3558 cited in the application
- LEHNER M ET AL: "Redirecting T Cells to Ewing's Sarcoma Family of Tumors by a Chimeric NKG2D Receptor Expressed by Lentiviral Transduction or mRNA Transfection", PLOS ONE, vol. 7, no. 2, E31210, 15 February 2012 (2012-02-15), XP055262099, DOI: 10.1371/journal.pone.0031210 cited in the application
- SONG D-G ET AL: "Chimeric NKG2D CAR-Expressing T Cell-Mediated Attack of Human Ovarian Cancer Is Enhanced by Histone Deacetylase Inhibition", HUMAN GENE THERAPY, vol. 24, no. 3, March 2013 (2013-03), pages 295-305, XP055370704, ISSN: 1043-0342, DOI: 10.1089/hum.2012.143 cited in the application
- LANIER L L: "DAP10- and DAP12-associated receptors in innate immunity", IMMUNOLOGICAL REVIEWS, vol. 227, no. 1, 19 December 2008 (2008-12-19), pages 150-160, XP071455361, ISSN: 0105-2896, DOI: 10.1111/J.1600-065X.2008.00720.X
- ZHENG L ET AL: "A Humanized Lym-1 CAR with Novel DAP10/DAP12 Signaling Domains Demonstrates Reduced Tonic Signaling and Increased Antitumor Activity in B-Cell Lymphoma Models", CLINICAL CANCER RESEARCH, vol. 26, no. 14, 15 July 2020 (2020-07-15) , pages 3694-3706, XP055879554, ISSN: 1078-0432, DOI: 10.1158/1078-0432.CCR-19-3417
- JIN L ET AL: "CXCR1- or CXCR2-modified CAR T cells co-opt IL-8 for maximal antitumor efficacy in solid tumors", NATURE COMMUNICATIONS, vol. 10, no. 1, 4016, 5 September 2019 (2019-09-05), XP055945908, DOI: 10.1038/s41467-019-11869-4
- WHILDING L ET AL: "CAR T-Cells Targeting the Integrin [alpha]v[beta]6 and Co-Expressing the Chemokine Receptor CXCR2 Demonstrate Enhanced Homing and Efficacy against Several Solid Malignancies", CANCERS, vol. 11, no. 5, 674, 14 May 2019 (2019-05-14), XP055945909, ISSN: 2072-6694, DOI: 10.3390/cancers11050674
- LIU G ET AL: "CXCR2-modified CAR-T cells have enhanced trafficking ability that improves treatment of hepatocellular carcinoma", EUROPEAN JOURNAL OF IMMUNOLOGY, vol. 50, no. 5, 10 February 2020 (2020-02-10), pages 712-724, XP071228440, ISSN: 0014-2980, DOI: 10.1002/EJI.201948457
- LIM S-A ET AL: "Defective Localization With Impaired Tumor Cytotoxicity Contributes to the Immune Escape of NK Cells in Pancreatic Cancer Patients", FRONTIERS IN IMMUNOLOGY, vol. 10, 496, 9 April 2019 (2019-04-09), XP055945898, ISSN: 1664-3224, DOI: 10.3389/fimmu.2019.00496

## Description

### 1. BACKGROUND

Immunotherapy using chimeric antigen receptor (CAR)-engineered T-cells has proven transformative in the management of B-cell malignancy and multiple myeloma. However, application of this technology to solid tumor immunotherapy is impeded by the lack of tumor-selective targets. Most tumor antigens are intracellular and thus cannot easily be recognized by CAR T-cells. Consequently, most solid tumor directed CARs that are currently under development engage targets that are upregulated in tumor cells, but which are found at lower levels in normal tissues.

One of the few target groups that exhibits a high degree of tumor selectivity are the NKG2D ligands. In man, these comprise a group of 8 stress-induced proteins (MICA, MICB, ULBP1-6) that are aberrantly expressed on virtually all tumor cell types. Moreover, NKG2D ligands are also found on tumor associated stromal elements such as endothelium, regulatory T-cells and myeloid derived suppressor cells (Parihar, R., et al., 2019, Cancer Immunol. Res. 7(3):363-375; Schmiedel & Mandelboim, 2018, Front. Immunol. (9)2040). Mice that are genetically deficient in NKG2D demonstrate impaired immunosurveillance for both epithelial and lymphoid malignancies. Evidence that NKG2D ligands are safe therapeutic targets is supported by the fact that they are not found in healthy tissues. Clinical trials using autologous NKG2D-targeted CARs have not revealed any significant safety issues (see https://pubmed.ncbi.nlm.nih.gov/30396908/).

The NKG2D receptor is naturally expressed by natural killer (NK) and some T-cell populations. Each NKG2D homodimer associates with two homodimeric DAP10 adaptor molecules via complementary charged amino acids within the plasma membrane. This interaction is required for cell surface expression and function of NKG2D. DAP10 resembles CD28 in its ability to provide co-stimulation via phosphatidylinositol 3-kinase but, importantly, it lacks a p56lck binding motif that promotes the unwanted recruitment of regulatory T-cells (Kofler, et al., 2011, Mol. Ther. 19:760-767). Potency of DAP10 co-stimulation is underscored by its continued ability to signal following internalization. However, since DAP10 lacks an immunoreceptor tyrosine-based activation motif (ITAM), NKG2D engagement does not lead to full T-cell activation.

A variety of CARs have been developed which use different methods to provide ITAM-dependent signal 1 in addition to co-stimulation (also known as signal 2), as both signal types are necessary to elicit full T-cell activation. The first NKG2D-targeted CAR was developed by Sentman et al. and consists of a fusion of NKG2D to CD3ζ (Zhang et al, 2005, Blood 106:1544-1551). Although nominally a first-generation CAR, it associates with endogenous DAP10 in T-cells, meaning that both signals 1 and 2 are provided. This CAR is currently undergoing clinical development by Celyad Oncology as Cyad-01. More recently, Chang et al. (2013, Cancer Res. 73:1777-1786) engineered NK cells to co-express an identical CAR in addition to exogenous DAP 10. Two further NKG2D CARs have also been described that incorporate either 4-1BB (Song et al., 2013, Hum. Gene Ther. 24:295-305) or CD28 (Lehner et al., 2012, PLoS One 7:e31210) to provide alternative forms of co-stimulation instead of that provided by DAP10. All of these CARs have enabled T-cell mediated tumor cell killing accompanied by cytokine production while the CAR described by Chang et al. also demonstrated transient in vivo anti-tumor activity.

Ligands of the CXCR2 receptor include chemokines of the Cysteine-X-Cysteine (CXC) family that contain an (Glu-Leu-Arg) ELR motif, namely CXCL1-3 and CXCL5-8. Production of these chemokines within tumors is not only undertaken by malignant cells, but also by stromal elements such as fibroblasts and macrophages (Thuwajit et al., 2018, Med Res Rev. 38:1235-54; Thongchot et al, 2021, Int J Oncol. 58:14). Moreover, tumor cells can educate stromal cells to produce these factors, thereby enabling disease progression and resistance to cytotoxic chemotherapy (Le Naour, 2020, J Mol Cell Biol. 12:202-15).

The best studied member of the CXC family is CXCL8, also known as interleukin (IL)-8. Levels of circulating CXCL8 are elevated in patients with certain cancers, including ovarian tumors (Zhang, et al., 2019, Oncol Lett. 17:2365-9), malignant mesothelioma (Judge, et al. 2016, Ann Surg Oncol. 23:1496-500), pancreatic cancer (Hou, et al. 2018, J Clin Med. 7:502, breast cancer (Milovanovic, et al. 2019, Cytokine 118:93-98; Autenshlyus, et al. 2021, 35: 20587384211034089), esophageal cancer (Huang, et al. Cancer Biomark. 29:139-149) and head and neck cancer (Rezaei, et al. 2019, 39:727-739). In addition to CXCL8, high level production of the CXCR2 ligands CXCL1, CXCL3, and CXCL5 has also been described in several cancers.

### 2. SUMMARY

In a first aspect, described herein is an immunoresponsive cell comprising an NKG2D polypeptide, a CXCR2 polypeptide, and a fusion polypeptide comprising a DNAX-activating 10 (DAP10) polypeptide or a functional variant thereof and a DNAX-activating 12 (DAP12) polypeptide or a functional variant thereof. In some embodiments, the NKG2D, CXCR2, DAP10, and DAP12 polypeptides are each mammalian polypeptides. In some embodiments, the NKG2D, CXCR2, DAP10, and DAP12 polypeptides are each human polypeptides. In some embodiments, the sequence of the NKG2D polypeptide has at least about 85% sequence identity to the sequence of SEQ ID NO: 14. In some embodiments, the NKG2D polypeptide is a functional variant of human NKG2D polypeptide and has one or more mutations that add, delete, or substitute at least one of the amino acids of SEQ ID NO: 14. In some embodiments, the functional variant of NKG2D polypeptide is a truncated version of the polypeptide having the sequence of SEQ ID NO: 14. In some embodiments, the functional variant is a chimeric NKG2D polypeptide. In some embodiments, the functional variant is a human-murine chimeric NKG2D polypeptide.

In some embodiments, the DAP10 polypeptide is a functional variant of DAP10 comprising an amino acid sequence having at least about 80%, at least about 85%, at least about 90%, at least about 95%, at least about 97%, at least about 98%, or at least about 99% sequence identity to the DAP10 polypeptide of SEQ ID NO: 1. In some embodiments, the DAP 10 polypeptide is a functional variant of DAP 10 that has one or more point mutations that add, delete, or substitute any of the amino acids of SEQ ID NO: 1. In some embodiments, the DAP10 polypeptide is a functional variant of DAP 10 which is a truncated version of the polypeptide having the amino acid sequence of SEQ ID NO: 1. In some embodiments, the DAP10 polypeptide comprises or consists of the sequence of any one of SEQ ID NOs: 1-8.

In some embodiments, the DAP12 polypeptide is a functional variant of DAP12 comprising an amino acid sequence having at least about 80%, at least about 85%, at least about 90%, at least about 95%, at least about 97%, at least about 98%, or at least about 99% sequence identity to the DAP12 polypeptide of SEQ ID NO: 9. In some embodiments, the DAP12 polypeptide is a functional variant of DAP12 that has one or more point mutations that add, delete, or substitute any of the amino acids of SEQ ID NO: 9. In some embodiments, the DAP12 polypeptide is a functional variant of DAP12 which is a truncated version of the polypeptide having the amino acid sequence of SEQ ID NO: 9. In some embodiments, the DAP12 polypeptide comprises or consists of the sequence of any one of SEQ ID NOs: 9-13.

In some embodiments, the DAP10 polypeptide and the DAP12 polypeptide are joined by a linker. In some embodiments, the linker comprises or consists of the amino acid sequence of any one of SEQ ID NOs: 18-46.

In some embodiments, the fusion polypeptide comprises an N-terminal sequence. In some embodiments, the fusion polypeptide comprises a C-terminal sequence. In some embodiments, the N-terminal or C-terminal sequence comprises one or more of a His-tag, FLAG-tag, Arg-tag, T7-tag, Strep-tag, S-tag, an AviTag^{™}, an aptamer-tag, a myc tag, CD8α leader sequence, a 4-1BB endodomain, a V5 tag, or a CD27 endodomain. In particular embodiments, the fusion polypeptide comprises or consists of the sequence of any one of SEQ ID NOs: 60-63.

In some embodiments, the CXCR2 polypeptide has a sequence that has at least about 85% sequence identity to SEQ ID NO: 87. In particular embodiments, the CXCR2 polypeptide has the sequence of SEQ ID NO: 87.

In some embodiments, the immunoresponsive cell of the disclosure is a T cell or a Natural Killer (NK) cell. In some embodiments, the immunoresponsive cell is an αβ T cell, a γδ T cell, a CD4⁺ T cell, a CD8⁺ T cell, a Natural Killer T (NKT) cell, or any combination thereof.

In a second aspect, described herein is an immunoresponsive cell comprising a chimeric NKG2D polypeptide and a CXCR2 polypeptide. In some embodiments, the chimeric NKG2D polypeptide comprises a human NKG2D extracellular domain or a variant thereof and a murine NKG2D transmembrane domain or a variant thereof. In particular embodiments, the human NKG2D extracellular domain has the sequence set forth in any one of SEQ ID NOs: 95-97. In some embodiments, the chimeric NKG2D polypeptide comprises a variant of the human NKG2D extracellular domain that has at least 80% sequence identity to the sequence of any one of SEQ ID NOs: 95-97. In some embodiments, the murine NKG2D transmembrane domain has the sequence set forth in any one of SEQ ID NOs: 98-101. In some embodiments, the chimeric NKG2D polypeptide comprises a variant of the murine NKG2D transmembrane domain that has at least 80% sequence identity to the sequence of any one of SEQ ID NOs: 98-101. In some embodiments, the immunoresponsive cell further comprises at least one DAP12 polypeptide or a variant thereof. In some embodiments, the immunoresponsive cell further comprises at least one DAP10 polypeptide or a variant thereof. In a preferred embodiment, the cell comprises a polypeptide that has at least 90% sequence identity to SEQ ID NO: 102.

Also described herein is a nucleic acid molecule encoding a NKG2D polypeptide, a CXCR2 polypeptide, and, optionally, a DNAX-activating (DAP 10) polypeptide, a DNAX-activating (DAP12) polypeptide, and/or a fusion polypeptide.

Also described herein is a method of making an immunoresponsive cell comprising the steps of (i) transducing a T-cell or a natural killer (NK) cell with a nucleic acid molecule or vector as described herein, and (ii) culturing the T-cell or natural killer (NK) cell such that the transduced cell expresses an NKG2D polypeptide, a CXCR2 polypeptide, and, optionally, a DAP10/DAP12 fusion polypeptide. In preferred embodiments, the NKG2D polypeptide associates with the cell membrane.

Also described herein is a method of treating a subject who has cancer, comprising administering to the subject a therapeutically effective amount of an immunoresponsive cell described herein. In some embodiments, the immunoresponsive cells are manufactured from T or natural killer (NK) cells autologous to the subject. In some embodiments, the cancer is a solid tumor cancer. In some embodiments, the solid tumor cancer is liver cancer, lung cancer, breast cancer, prostate cancer, lymphoid cancer, colon cancer, renal cancer, bone cancer, pancreatic cancer, skin cancer, cancer of the head and neck, cutaneous or intraocular malignant melanoma, uterine cancer, ovarian cancer, rectal cancer, cancer of the anal region, stomach cancer, testicular cancer, uterine cancer, thyroid cancer, cancer of the esophagus, cancer of the small intestine, or any combination thereof.

### 3. BRIEF DESCRIPTION OF THE DRAWINGS

These and other features, aspects, and advantages of the present invention will become better understood with regard to the following description, and accompanying drawings, where:

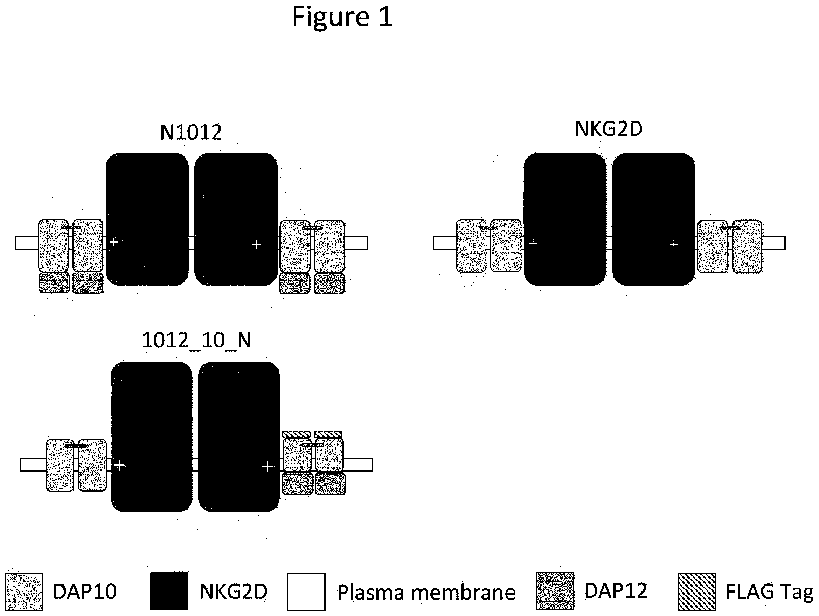

**Figure 1** is a schematic demonstrating the structure of each of the constructs that have been generated. N1012 comprises a complex comprising an exogenous human NKG2D protein and fused exogenous DAP10/12 homodimers according to the invention. N1012 comprises SEQ ID NO: 64, which is described in Table 2. 1012_10_N comprises NKG2D co-expressed with DAP 10 and a FLAG-tagged fusion of the DAP 10 extracellular and transmembrane domain fused to the DAP12 endodomain. The NKG2D complex comprises an exogenous human NKG2D protein which interacts with endogenous DAP 10 already present in the cell and is provided for comparative purposes only.

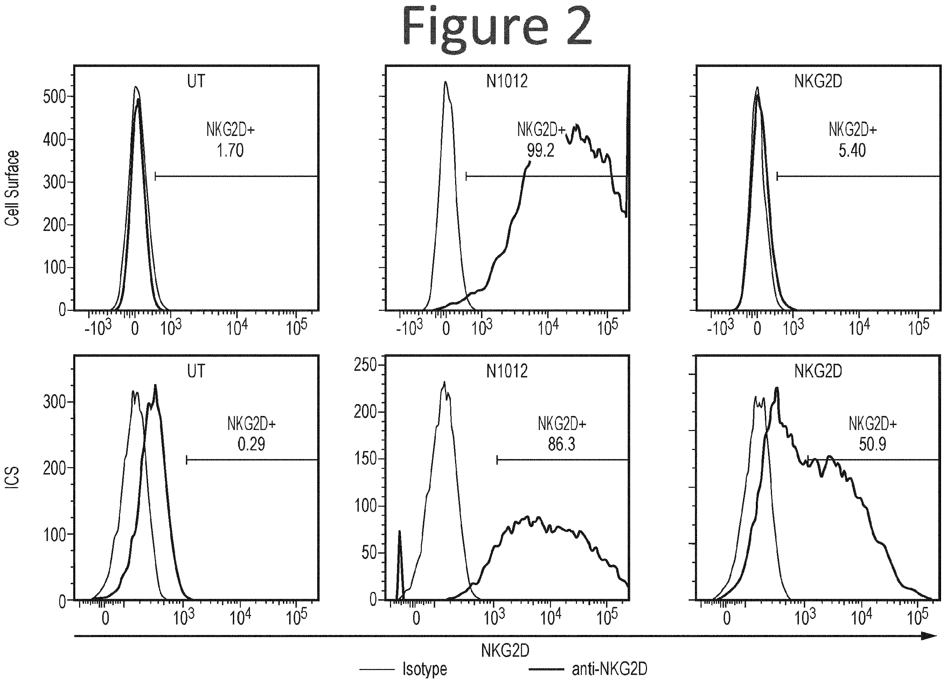

**Figure 2** shows the expression of NKG2D at both the cell surface and intracellularly (ICS) in 293T cells three days after transfection with either the retroviral plasmid expressing N1012 (shown schematically in Figure 1) or NKG2D alone, making comparison with untransduced (UT) 293T cells.

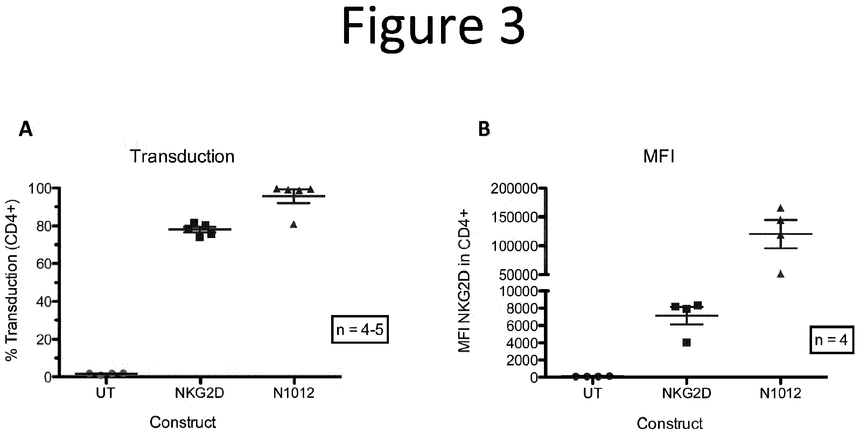

**Figures 3A-3B** show the transduction percentage **(****Figure 3A****)** and median fluorescence intensity (MFI - **Figure 3B****)** of cell surface NKG2D expression in the CD4⁺ subset of T-cells, following transduction of activated unfractionated human T-cells with retroviral vectors that encode for NKG2D alone or N1012. The expression and MFI in UT T-cells is also shown for comparison.

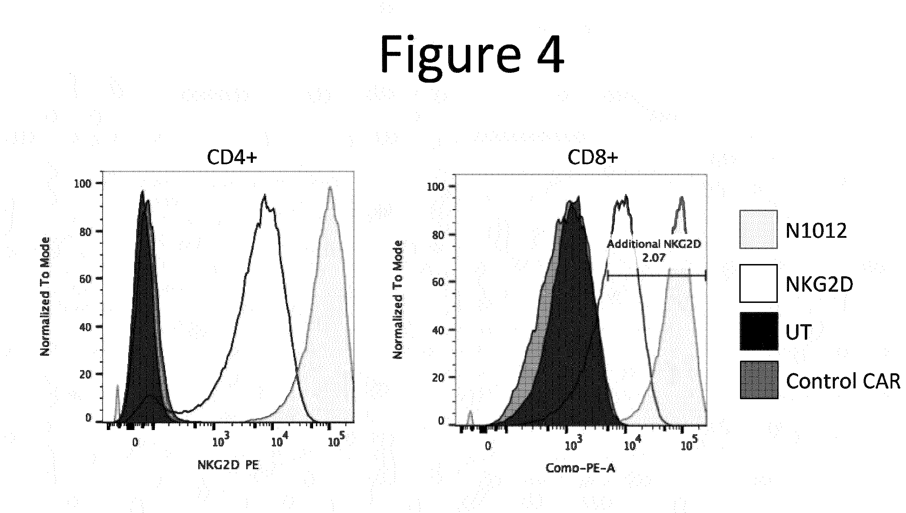

**Figure 4** shows a representative expression pattern of cell surface NKG2D within the CD4⁺ and CD8⁺ subset of activated unfractionated T-cells transduced to express N1012, NKG2D or a control CAR that lacks NKG2D. NKG2D expression within UT T-cells is provided as a comparison.

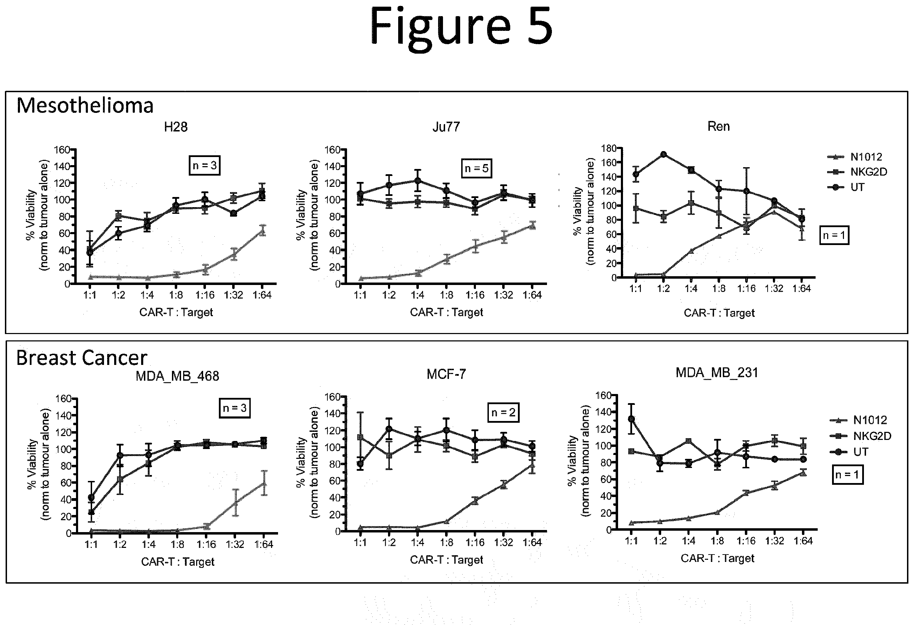

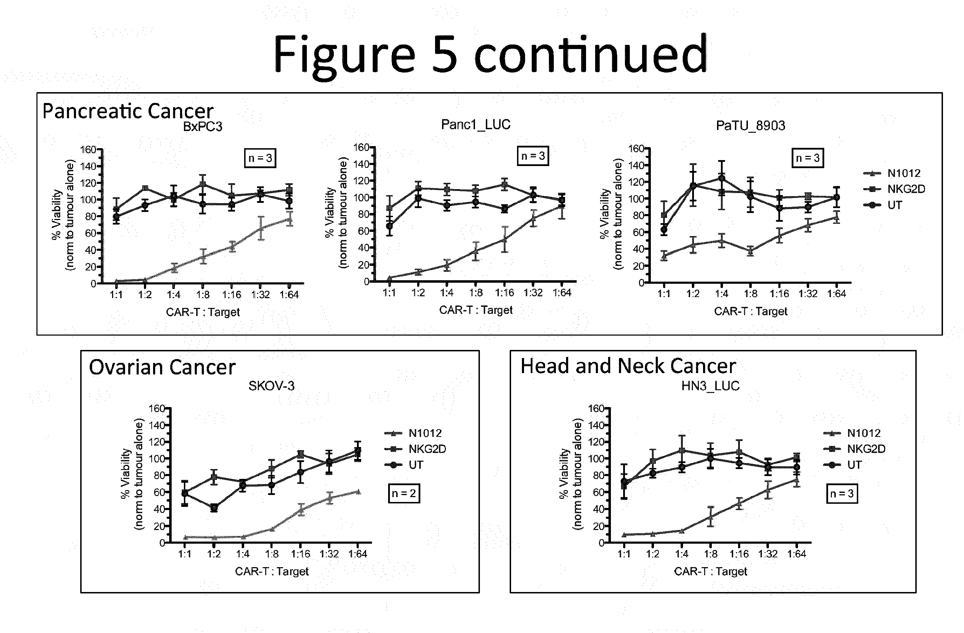

**Figure 5** shows the viability of 11 different tumor cell lines after co-culture with N1012⁺, NKG2D⁺ or UT T-cells at varying CAR T-cell: target ratios. Tumor cell viability was assessed after 72 hours using an MTT assay and is expressed as a percentage of that observed in the absence of T-cell co-culture.

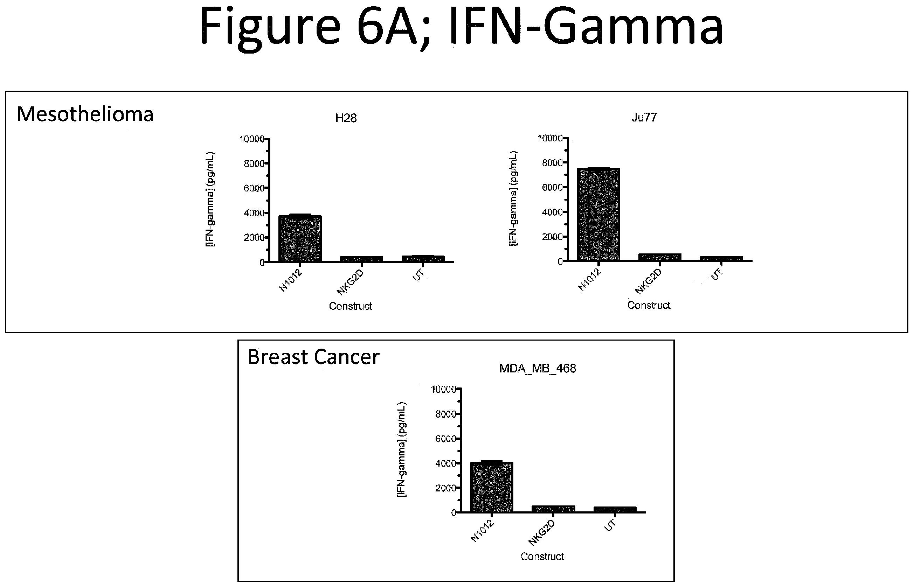

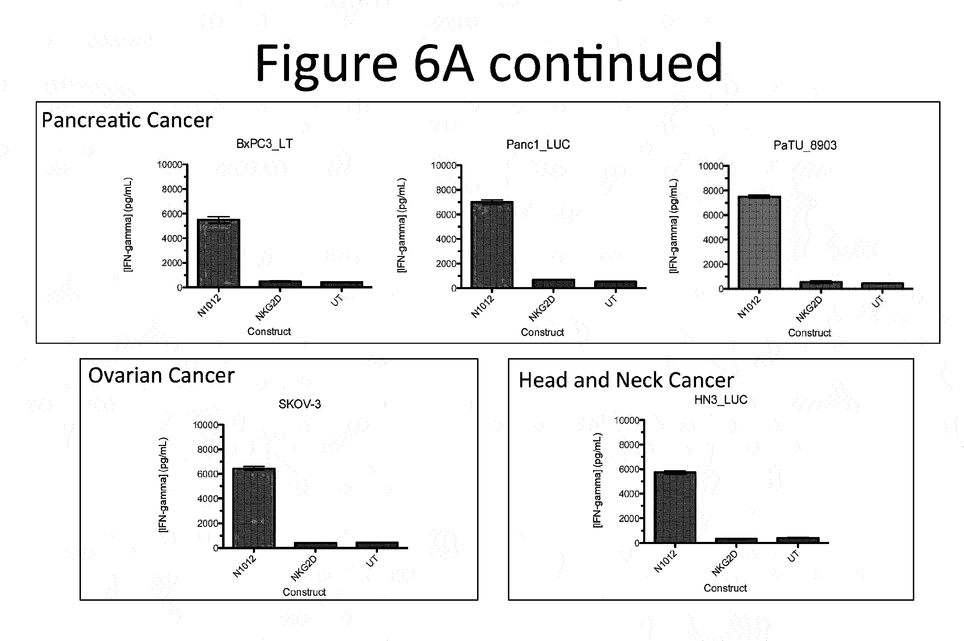

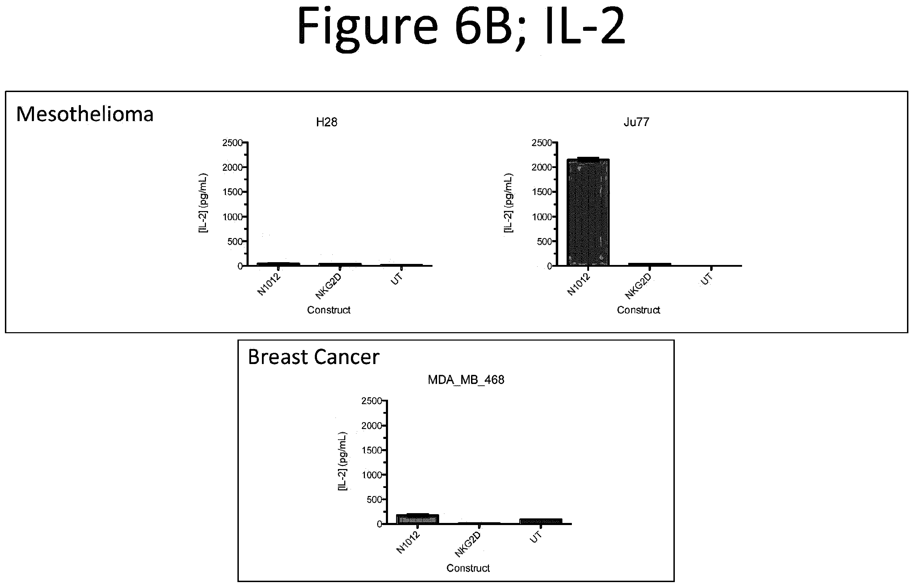

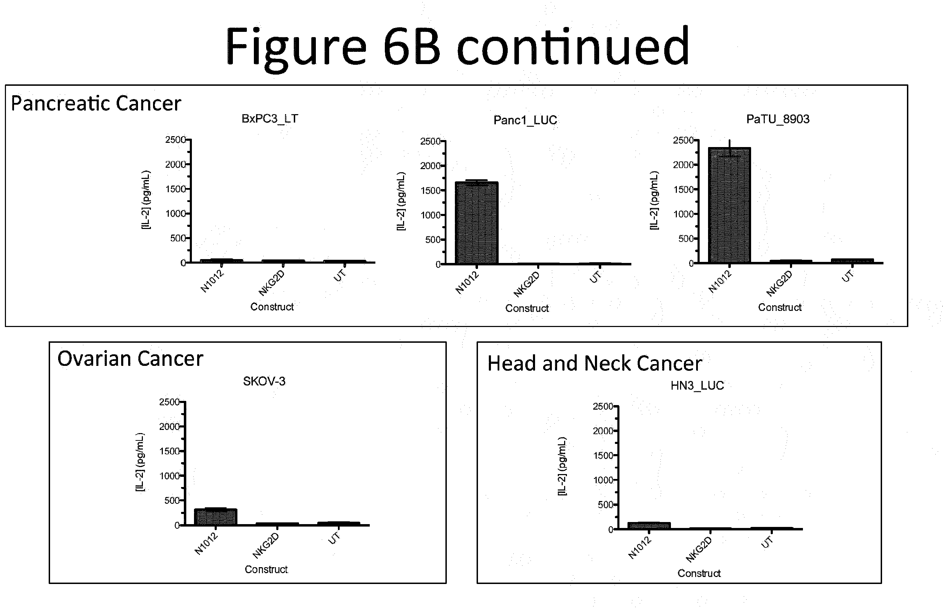

**Figures 6A-6B** show the levels of cytokine (IFN-γ **(****Figure 6A****)** and IL-2 **(****Figure 6B****)** secreted by N1012, NKG2D and UT T-cells during co-culture with 8 different tumor cell lines at a 1:1 CAR T-cell: target ratio. Co-culture supernatants were removed after 72 hours and assessed for cytokine presence by enzyme-linked immunosorbent assay (ELISA).

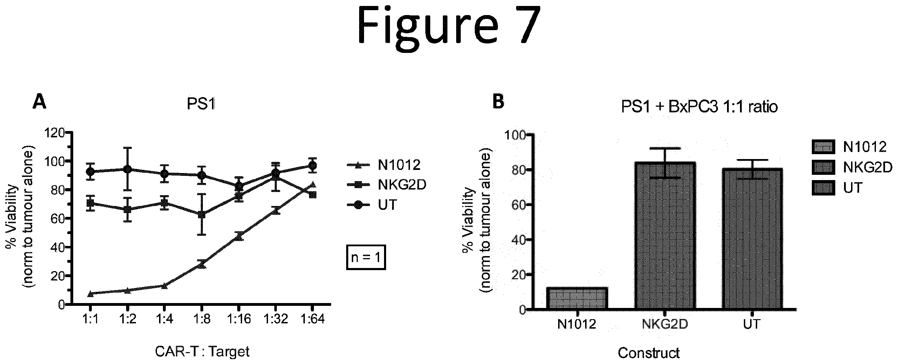

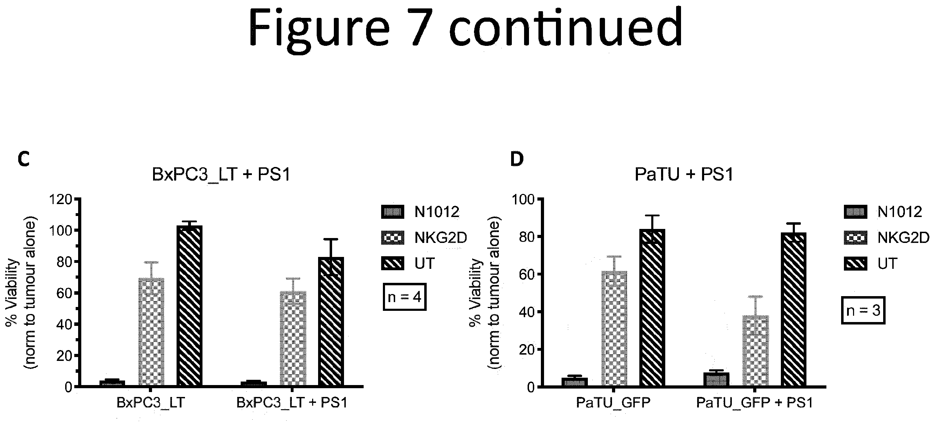

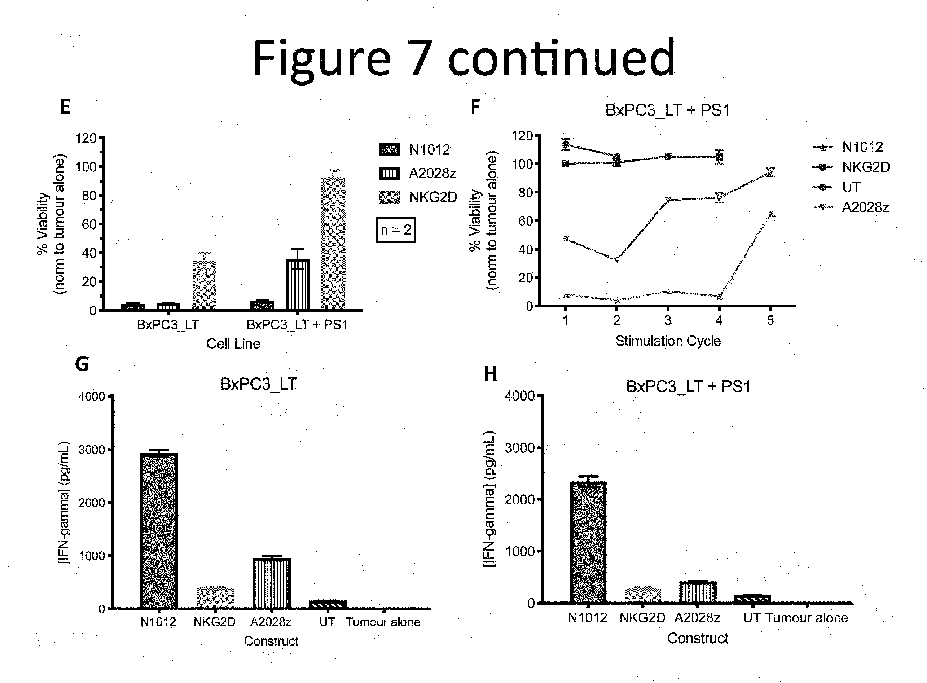

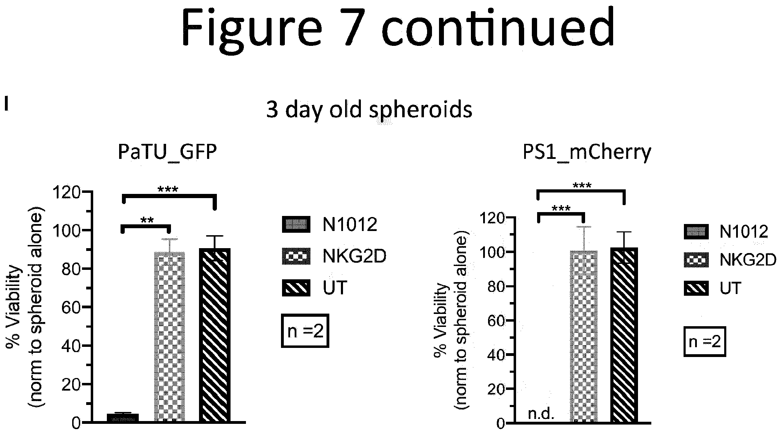

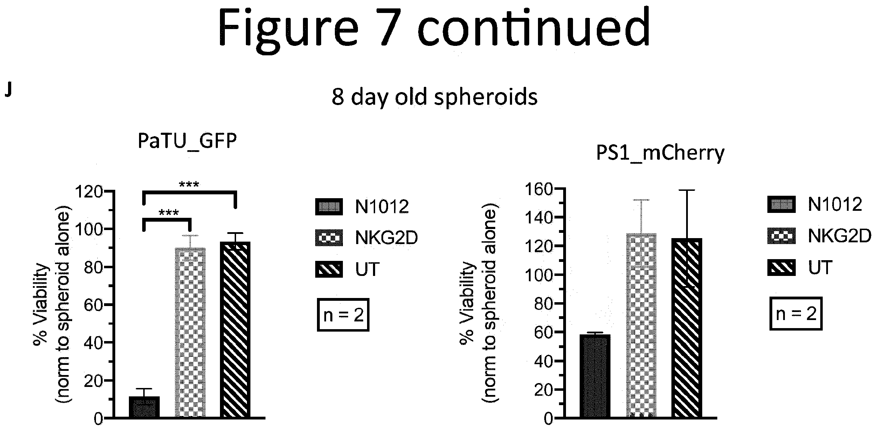

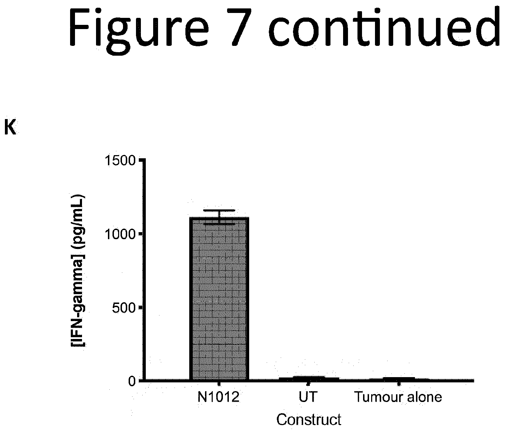

**Figures 7A-7F** show the viability of tumor and/or pancreatic stellate PS1 after co-culture with N1012⁺, NKG2D⁺ or UT T-cells at varying CAR T-cell: target ratios. **Figure 7A** shows the viability of pancreatic stellate PS1 cells after co-culture with N1012⁺, NKG2D⁺ or UT T-cells at varying CAR T-cell: target ratios. When added at a 1:1 CAR T-cell: target ratio, N1012⁺ T-cells also demonstrate potent lysis of monolayers consisting of both PS1 and BxPC3 cells grown together at a 1:1 ratio **(****Figure 7B****).** This contrasts with the lack of efficacy observed upon the addition of either NKG2D⁺ or UT T-cells. In both cases, target cell viability was assessed after 48 hours by MTT assay and is expressed as a percentage of that observed in the absence of T-cell co-culture. The viability of tumor cell and stromal cell monolayers after co-culture with N1012⁺, NKG2D⁺ or UT T-cells at a 1:1 CAR T-cell: target ratio is shown in **Figure 7C** (BxPC3_LT + PS1) and **Figure 7D** (PaTU + PS1). Whereas potent lysis of both tumor and stromal cells was observed with N1012⁺ T-cells, a minimal reduction in target cell viability was observed when co-cultured with either NKG2D or UT T-cells **(****Figure 7C-7D****).** The viability of tumor cells alone following co-culture with N1012⁺, NKG2D⁺ or UT T-cells at a 1:1 CAR T-cell: target ratio is shown in **Figure 7C** (BxPC3 _LT) and **Figure 7D** (PaTU_GFP). The viability of tumor cell and stromal cell monolayers after co-culture with N1012⁺, A2028z⁺, NKG2D⁺ or UT T-cells at a 1:1 CAR T-cell: target ratio is shown in **Figures 7E and Figure 7F** (after up to 5 re-stimulations). The viability of tumor cells alone following co-culture with N1012⁺, A2028z⁺, NKG2D⁺ or UT T-cells at a 1:1 CAR T-cell: target ratio is also shown in **Figure 7E** (BxPC3_LT). A2028z is a CAR targeted against αvβ6 integrin which kills tumor cells, but not PS1 stromal cells (Whilding, et al, 2017, Mol Ther. 25:2427). **Figures 7G and 7H** show the level of IFN-γ secreted by N1012⁺, A2028z⁺, NKG2D⁺ and UT T-cells during the co-cultures described above. The level of IFN-γ secreted from the tumor alone was determined as a negative control. **Figures 7I** **and** **7J** show the viability of tumor spheroids in which GFP-expressing PaTU tumor cells were mixed with mCherry-expressing PS1 cells (1:1 ratio for 3 or 8 days respectively) following co-culture with N1012⁺, NKG2D⁺ or UT T-cells..n.d. - not detected. **Figure 7K** shows the levels of IFN-γ secreted after 72 hours by N1012⁺, UT T-cells and tumor alone during the co-cultures with tumor spheroids.

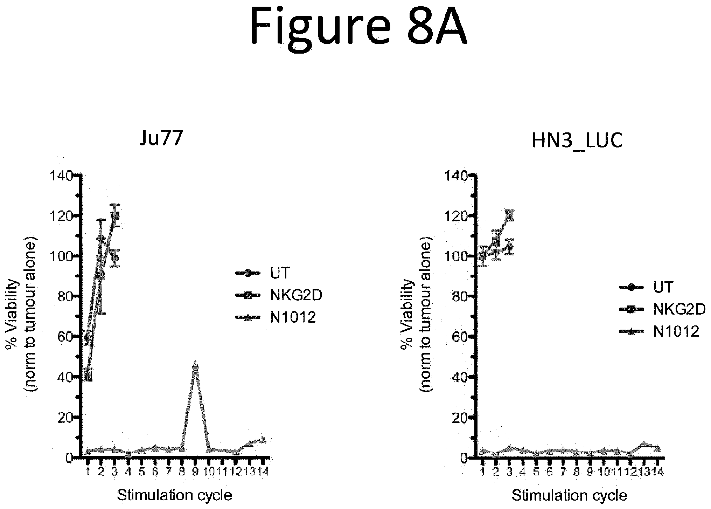

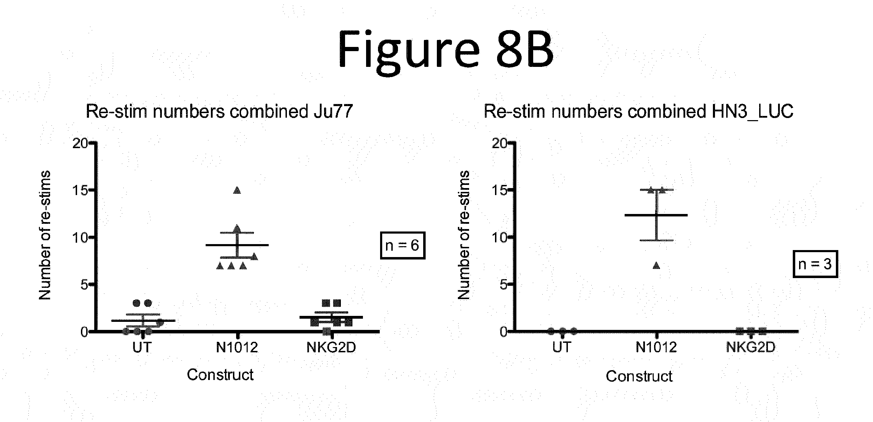

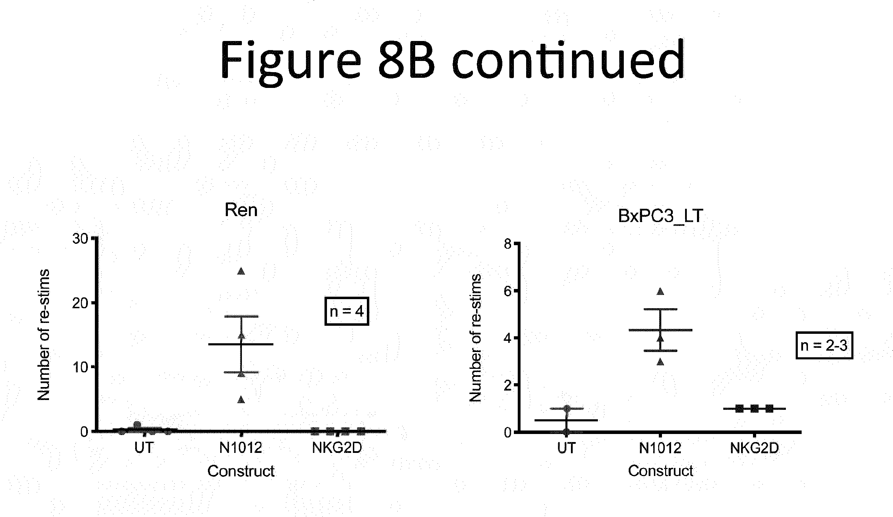

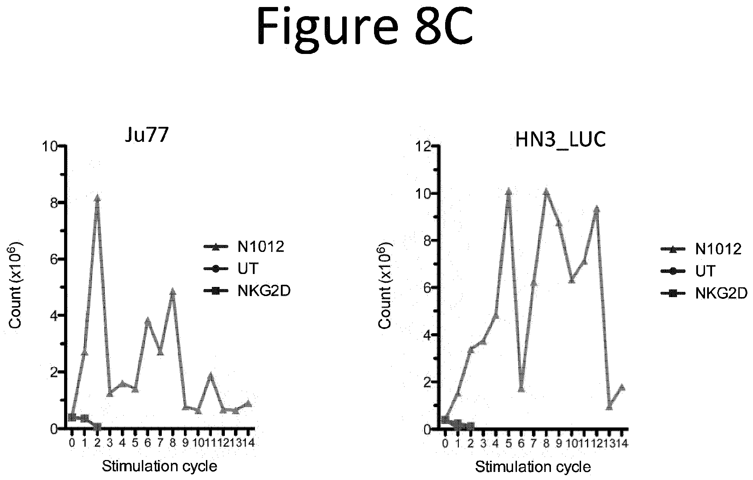

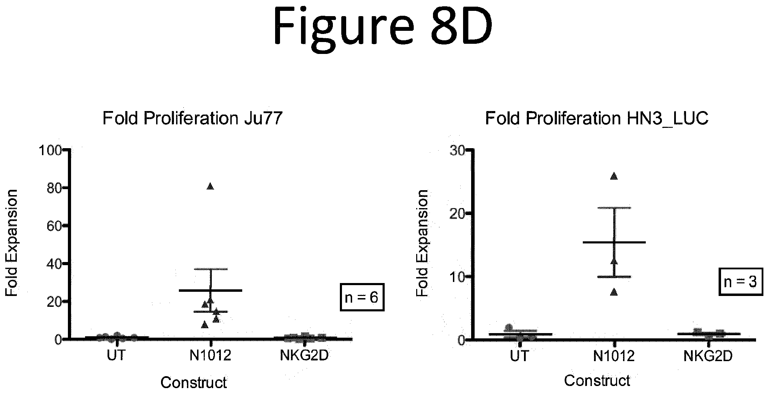

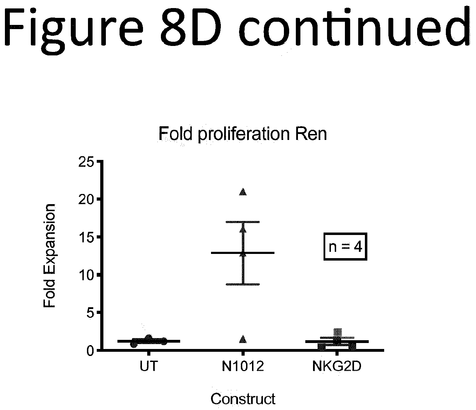

**Figures 8A-8D** show the ability of T-cells expressing N1012 to undergo multiple rounds of repeated stimulation (`re-stimulation'), when compared to UT T-cells, or those expressing NKG2D alone. T-cells expressing N1012 mediate lysis of both Ju77 mesothelioma Ju77 cells and HN3_LUC head and neck squamous cell carcinoma (HNSCC) cells through multiple cycles of stimulation **(****Figure 8A****).** Total number of successful re-stimulation cycles when co-cultured with mesothelioma (Ju77, Ren), HNSCC (HN3_LUC) or pancreatic cancer (BxPC3 _LT) cells are shown in **Figure 8B****.** Proliferation of T-cells upon repeated stimulation by Ju77 or HN3 _LUC tumor cells is shown in **Figure 8C****.** Maximum fold expansion of T-cells when compared to day 1 of culture with Ju77, Ren or HN3 _LUC tumor cells is shown in **Figure 8D****.** In contrast, UT T-cells or those expressing NKG2D alone demonstrate minimal target cell lysis or proliferation.

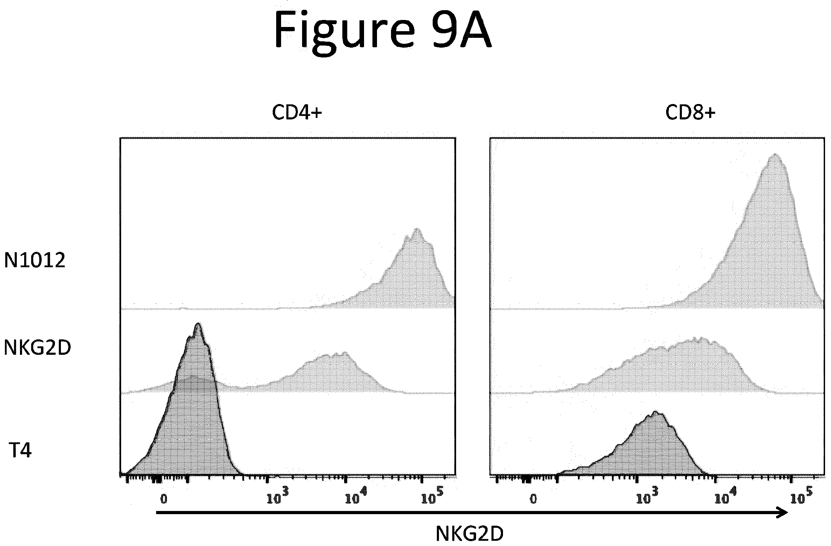

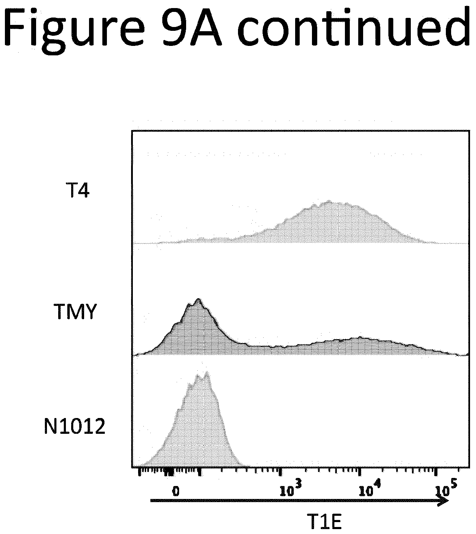

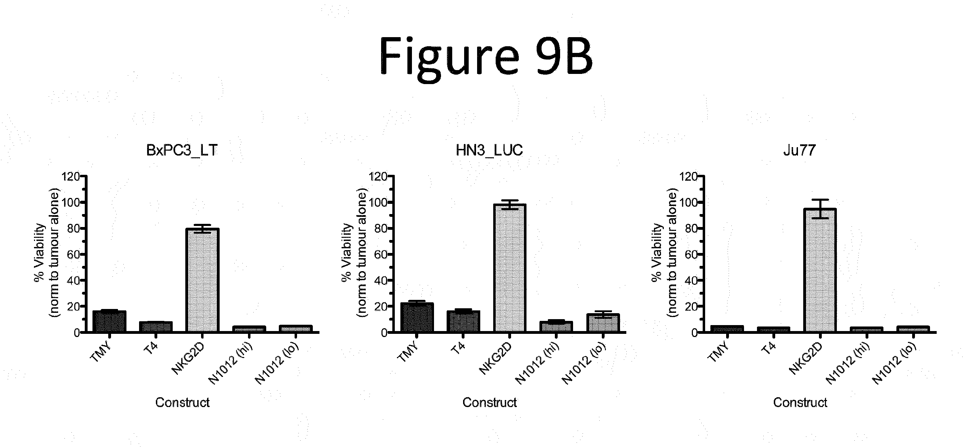

**Figures 9A-9B** show the engineering by retroviral transduction of CAR T-cells for *in vivo* evaluation in tumor-bearing mice. **Figure 9A** shows cell surface expression of NKG2D in CD4⁺ and CD8⁺ T-cells following transduction with either N1012 or NKG2D. Comparison was made with T-cells from the same donor expressing the pan-ErbB targeting CAR, T4, as described by Davies et al., 2012, Mol. Med. 18:565-576. A second pan-ErbB targeting CAR, designated TMY, that lacks the 4αβ domain contained within T4 and also has a slightly altered CD28 hinge (in which the MYPPPY sequence is replaced with the 10 amino acid linear myc tag sequence) was also used. **Figure 9B** shows the *in vitro* cytotoxic function of residual T-cells against the indicated tumor cell lines following adoptive transfer to mice.

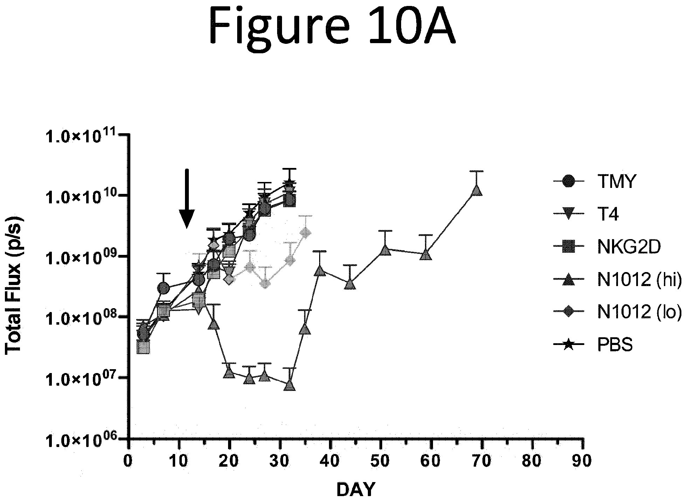

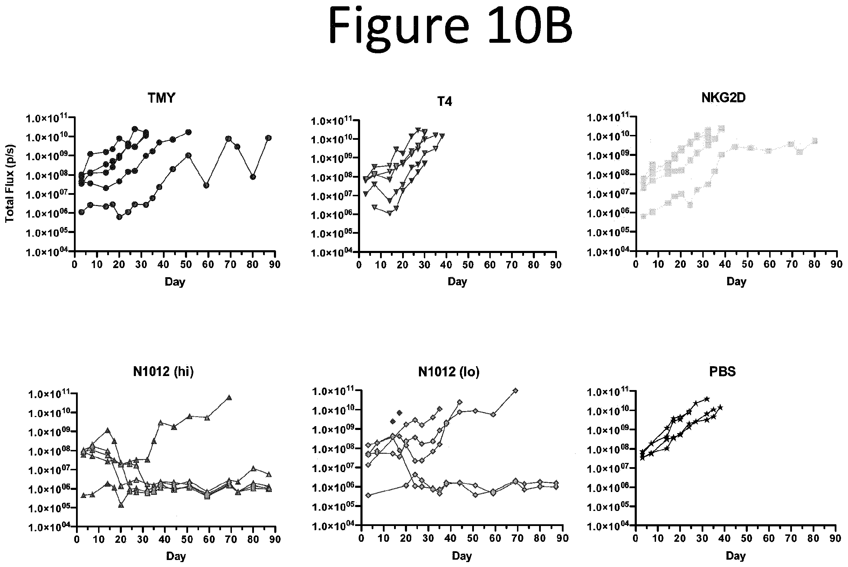

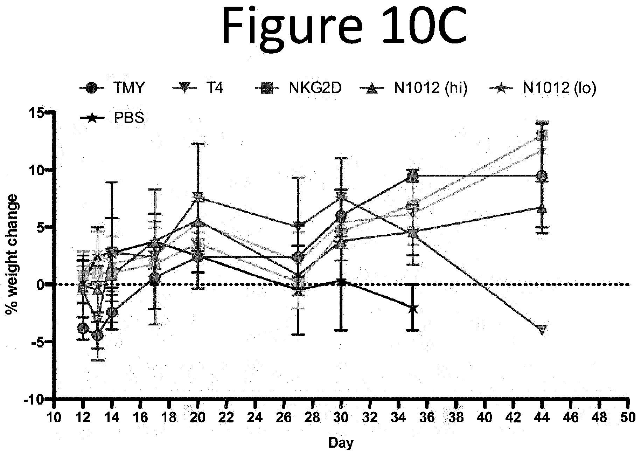

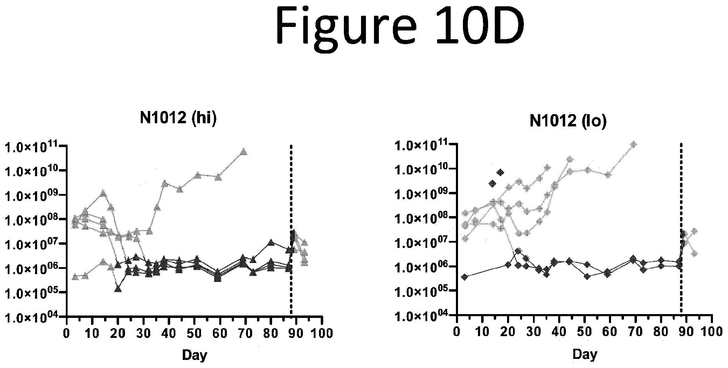

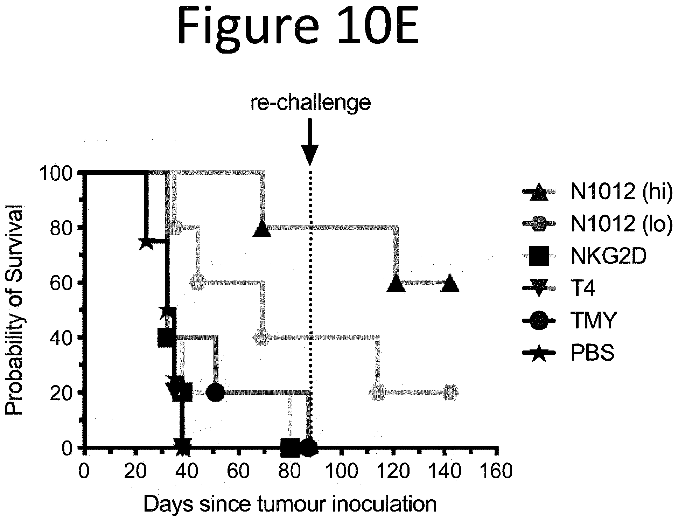

**Figures 10A-10E** show the intraperitoneal (i.p.) growth of ffLUC-tagged BxPC3 cells *in vivo* in NSG mice, as ascertained by bioluminescence imaging (BLI) before and after i.p. treatment with 10 × 10⁶ of the indicated CAR T-cells. In the case of N1012, this dose was also used (denoted `hi') or a lower dose administered ('lo' - 4 × 10⁶ cells). Pooled BLI emission from each group of mice is shown in **Figure 10A****.** Serial bioluminescence emission from individual mice is shown in **Figure 10B****.** Weight of mice is shown in **Figure 10C****.** Serial bioluminescence emission from individual mice following i.p. ffLUC BxPC3 tumor re-challenge on day 88 is shown in **Figure 10D****.** A survival curve of the experiment, which was ended after 145 days, is shown in **Figure 10E****.**

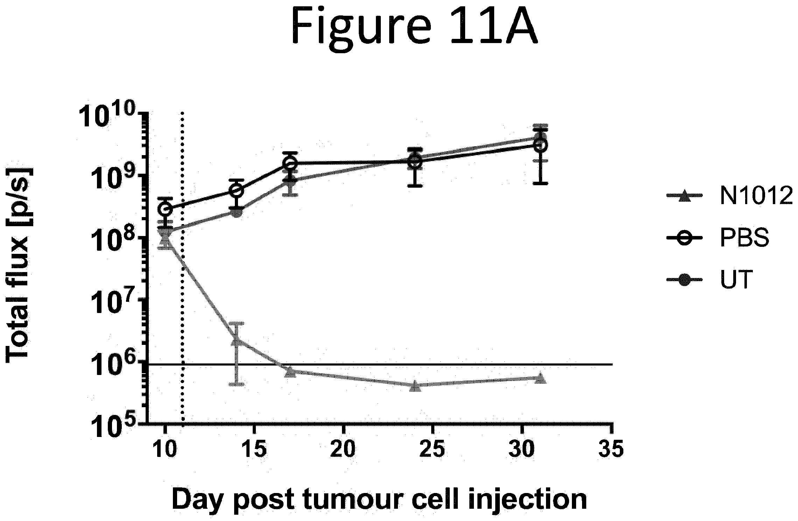

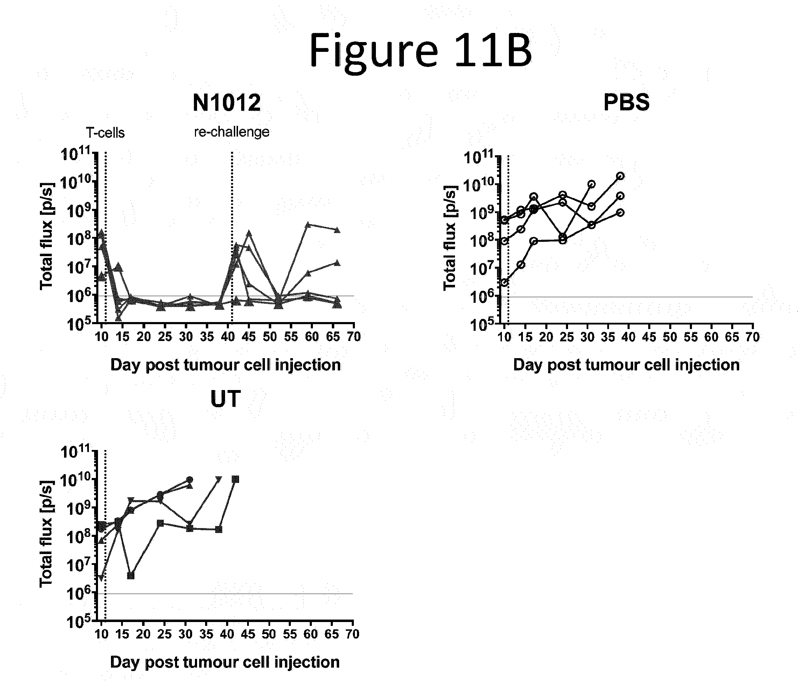

**Figures 11A-11B** show the results of another experiment demonstrating the i.p. growth of ffLUC-tagged BxPC3 cells *in vivo* in NSG mice before and after treatment with the indicated T-cells or PBS, as ascertained by bioluminescence imaging (BLI). BLI average total flux (photons/second) per treatment group is shown in **Figure 11A****,** and BLI total flux (photons/second) per individual mouse is shown in **Figure 11B****.** Mice that were tumor free at day 41 (29 days after T-cell infusion) were re-challenged i.p. (shown as a dotted line) with ffLUC-tagged BxPC3 cells.

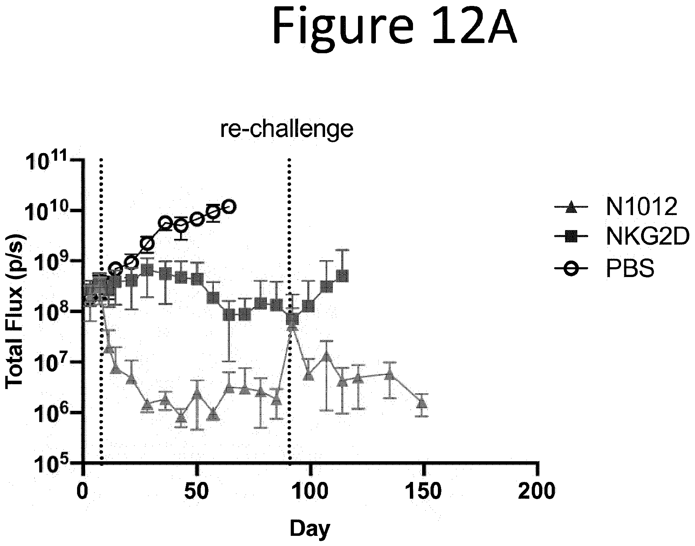

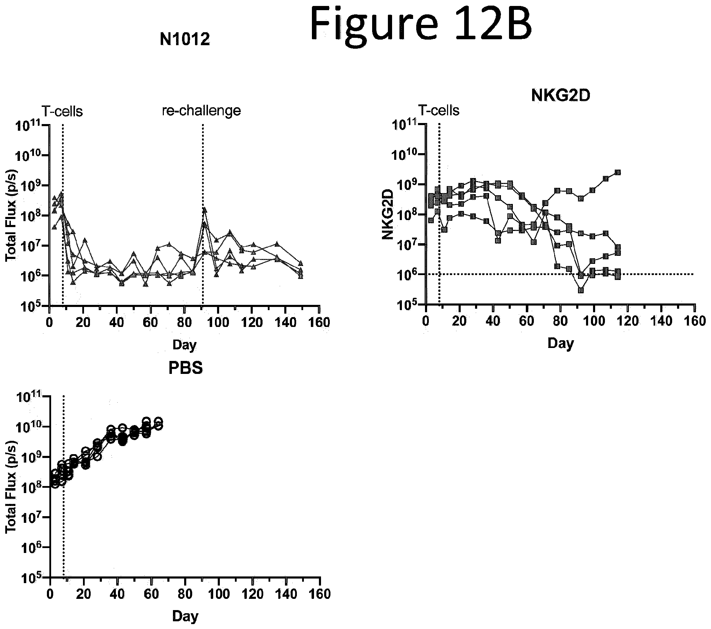

**Figures 12A-12B** show the i.p. growth of ffLUC-tagged H226 malignant mesothelioma cells *in vivo* in NSG mice before and after treatment with the indicated T-cells or PBS, as ascertained by bioluminescence imaging (BLI). BLI average total flux (photons/second) per treatment group is shown in **Figure 12A****,** and BLI total flux (photons/second) per individual mouse is shown in **Figure 12B****.** To confirm T-cell persistence and maintenance of function, all tumor-free mice were inoculated i.p. with an additional 1×10⁶ ffLUC-tagged H226 cells, 91 days after initial tumor inoculation. Timing of tumor rechallenge is shown as a second dotted line in the N1012 graph.

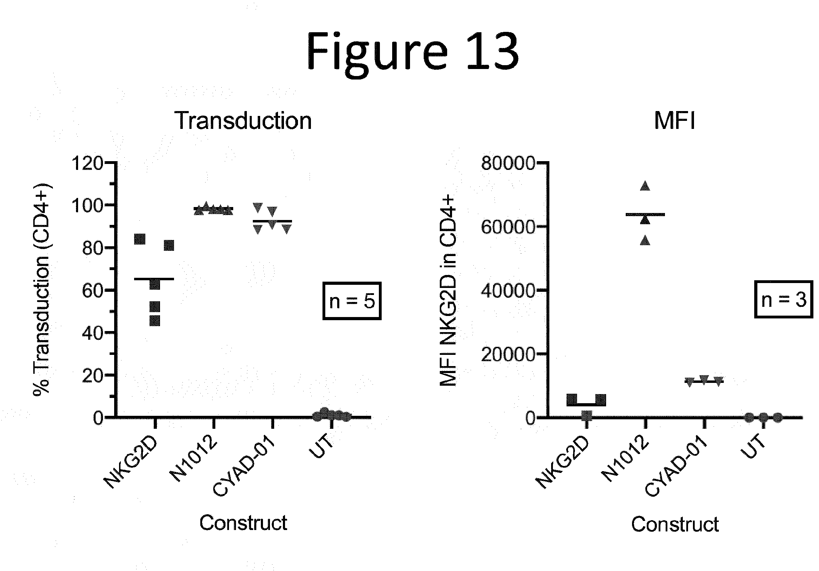

**Figure 13** shows the transduction percentage (left panel) and median fluorescence intensity (MFI) (right panel) of cell surface expression of NKG2D, N1012 and a replica of the Cyad-01 CAR when expressed in CD4⁺ T-cells.

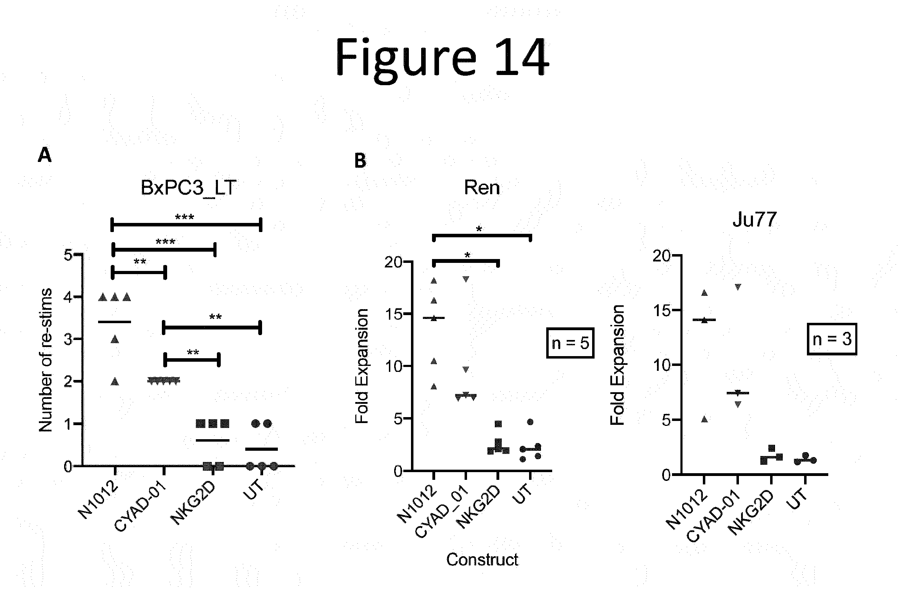

**Figures 14A-14B** compare the restimulation **(****Figure 14A****)** and maximum fold expansion **(****Figure 14B****)** of N1012⁺ T-cells to NKG2D T-cells or those expressing a replica of Cyad-01, when co-cultured with BxPC3-LT **(****Figure 14A****),** Ren or Ju77 cells **(****Figure 14B****).**

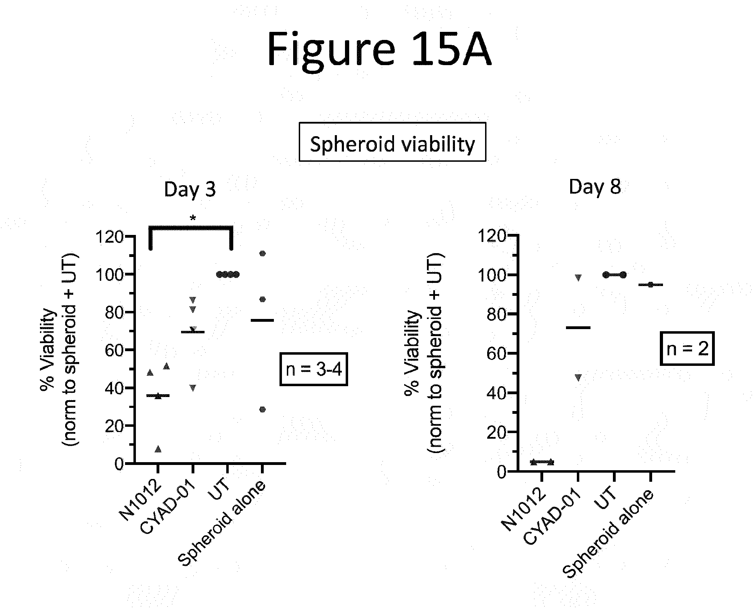

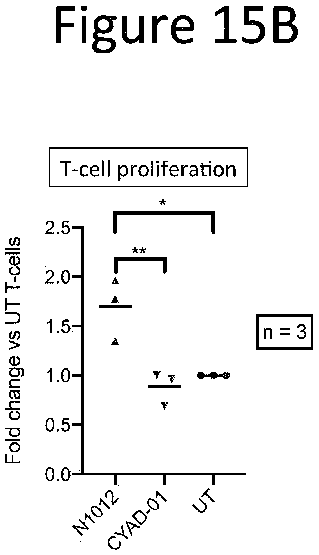

**Figure 15A** shows the viability of tumor spheroids after co-culture with N1012, a Cyad-01 replica, or untransduced T-cells for either 3 or 8 days. The tumor spheroids consisted of GFP-expressing PaTU tumor cells and mCherry-expressing PS1 cells co-cultured at a 1:1 ratio for 3 days prior to T-cell addition. The number of T-cells added reflected a final T-cell:GFP PaTU:mCherry PS1 ratio of **2:1:1.****Figure 15B** shows the level of proliferation of the T-cells during the co-cultures with tumor spheroids.

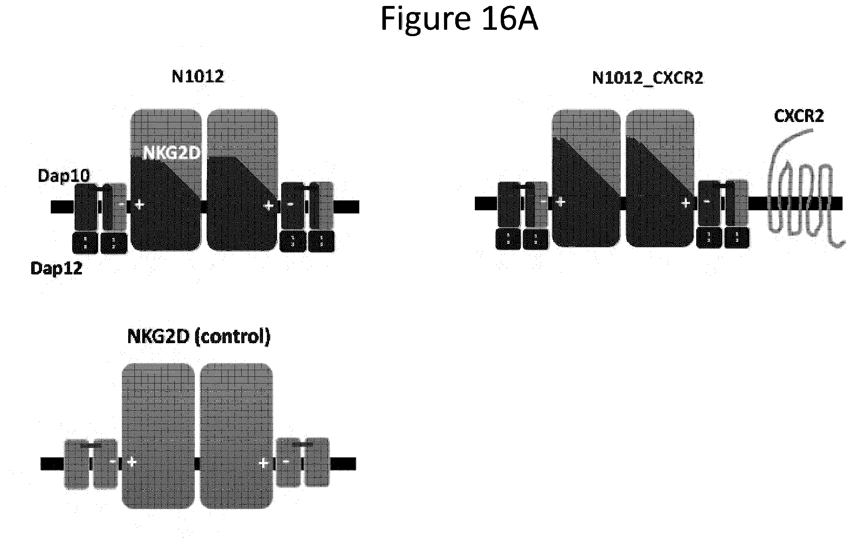

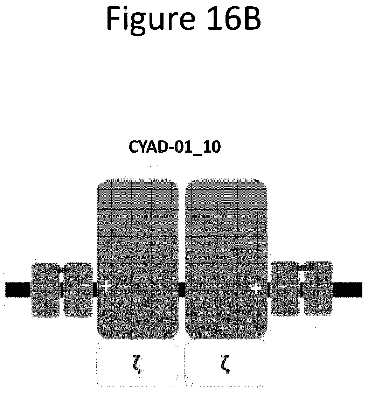

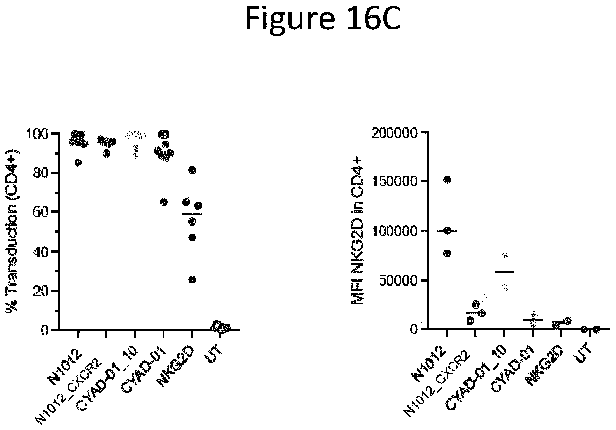

**Figures 16A-16C** are schematics of the CAR constructs N1012, N1012_CXCR2, and NKG2D **(****Figure 16A****)** and CYAD-01_10 **(****Figure 16B****).** In CYAD-01_10, a replica of Cyad-01 has been co-expressed with additional DAP10. **Figure 16C** shows the transduction percentage (left panel) and median fluorescence intensity (MFI) (right panel) of cell surface NKG2D expression in the CD4⁺ subset of T-cells following transduction of activated unfractionated human T-cells with retroviral vectors that encode N1012, N1012_CXCR2, CYAD-01_10, CYAD-01 replica, or NKG2D. Results for untransduced T-cells are shown for comparison.

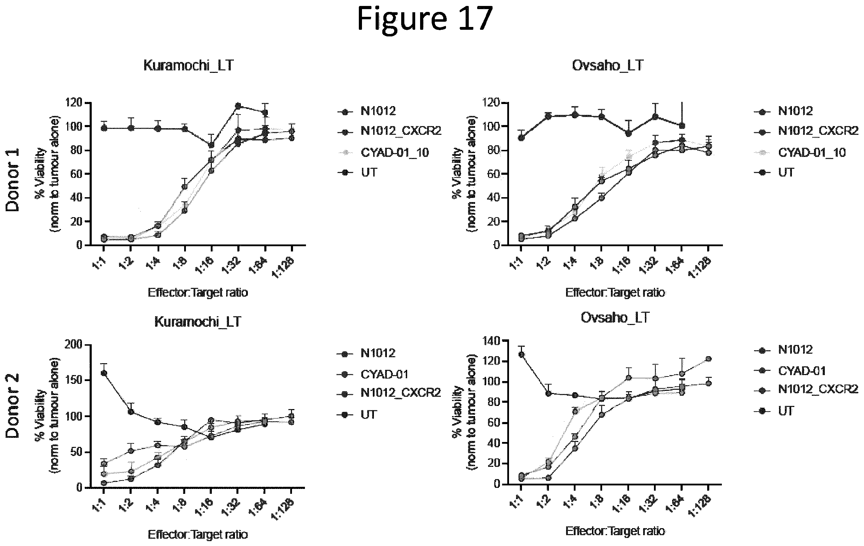

**Figure 17** shows viability of two ovarian cancer cell lines, Kuramochi_LT (left) and Ovsaho_LT (right) following co-culture with N1012, N1012_CXCR2, CYAD-01_10, and untransduced T-cells at varying CAR T-cell: target ratios. Data are shown for two independent donors. Tumor cell viability was assessed by MTT assay after 72 hours and is expressed as a percentage viable cells cultured in the absence of T-cells.

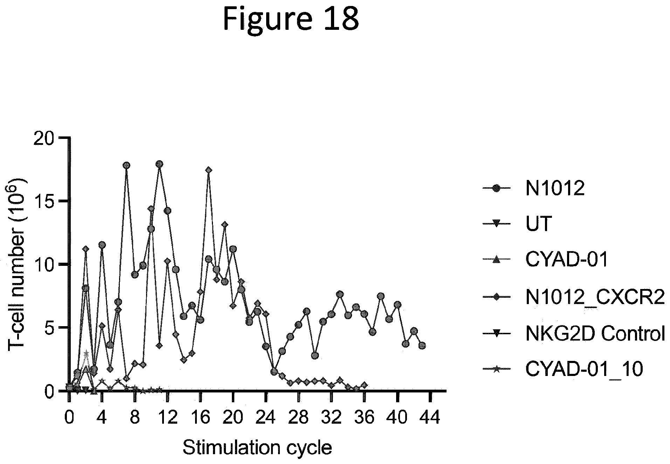

**Figure 18** shows the improved ability of N1012 and N1012_CXCR2 T-cells to persist and proliferate following repeated rounds of stimulation, compared to a replica of CYAD-01 or CYAD-01_10 T-cells.

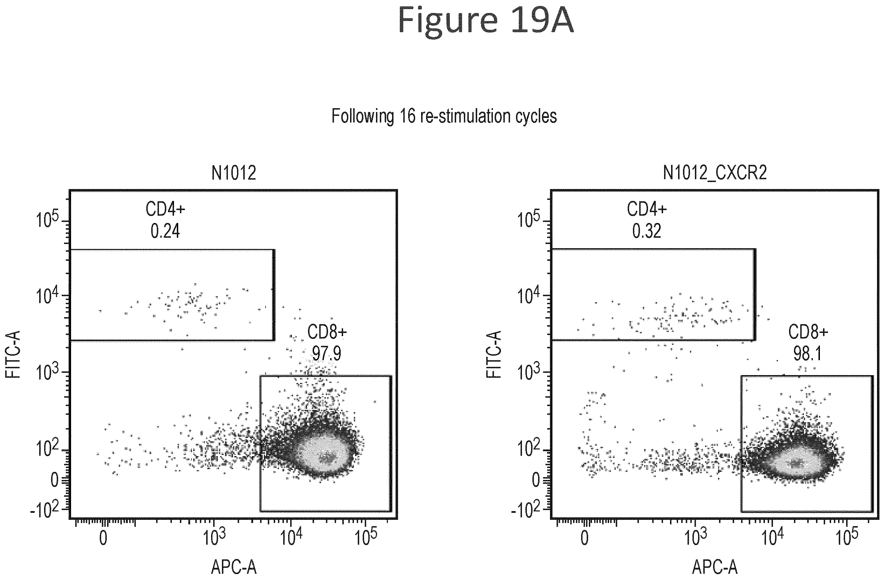

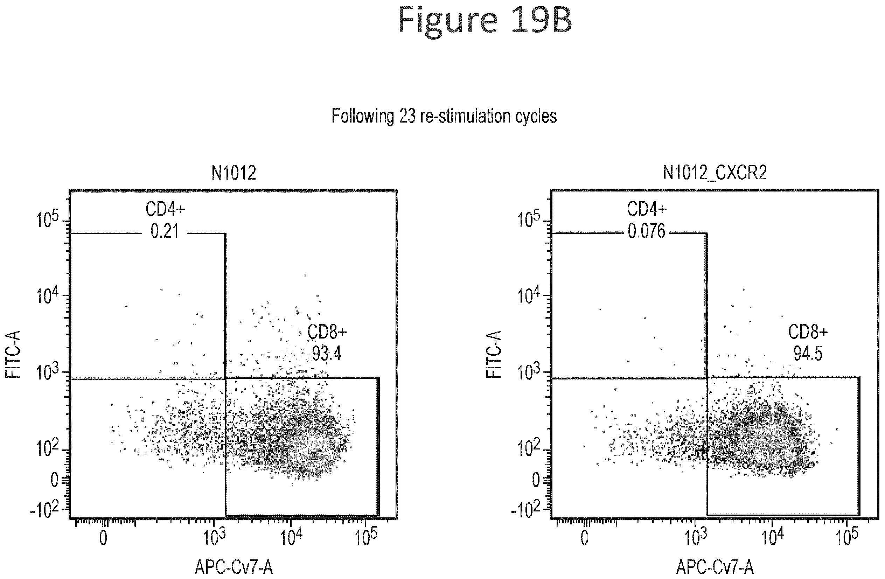

**Figures 19A-19B** are flow cytometry plots showing the nearly complete shift in N1012 and N1012_CXCR2 T-cells from a CD4⁺ to CD8⁺ population following 16 re-stimulation cycles **(****Figure 19A****)** and 23 re-stimulation cycles **(****Figure 19B****)** on Ren tumor cell monolayers.

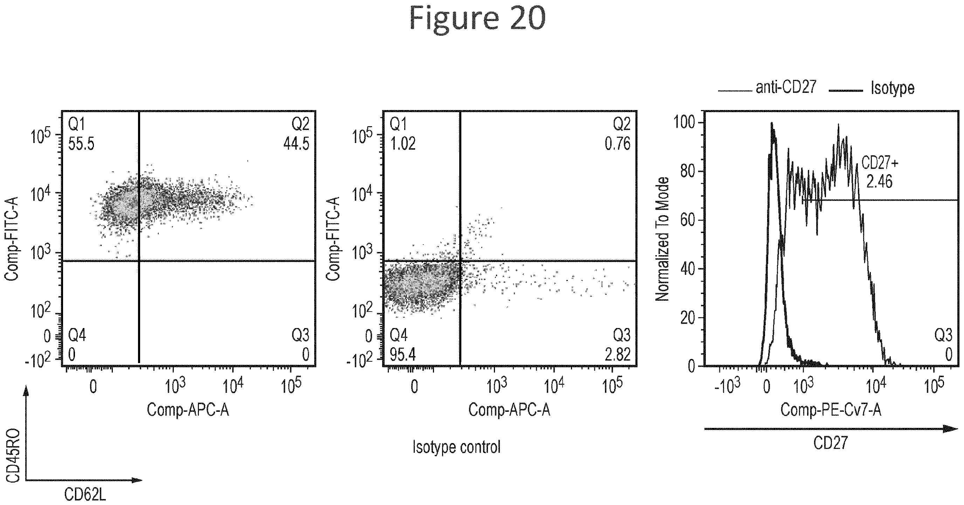

**Figure 20** shows flow cytometry results of N1012 T-cells assessed for CD45RO, CD62L and CD27 expression following 28 rounds of stimulation on Ren tumor cell monolayers.

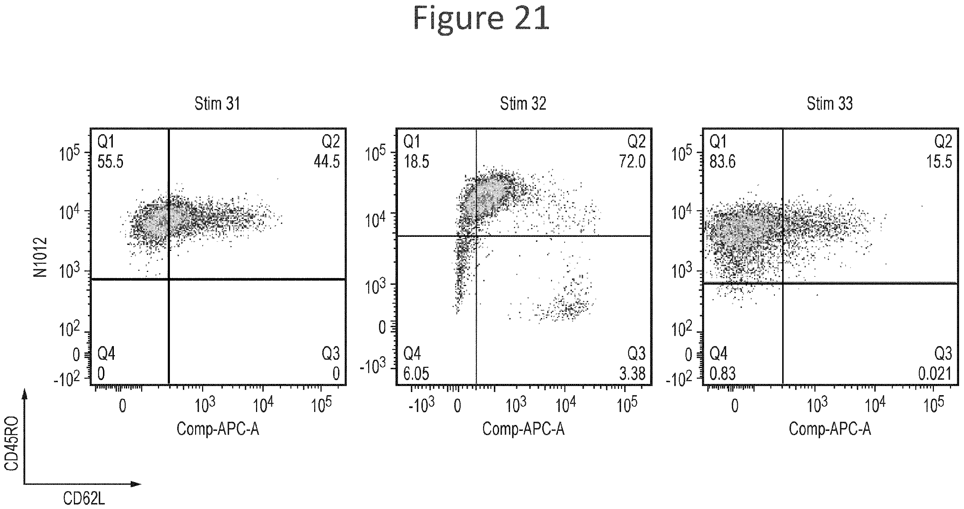

**Figure 21** shows results of N1012 T-cells assessed for expression of CD45RO and CD62L following 31, 32, and 33 rounds of stimulation on Ren tumor cell monolayers.

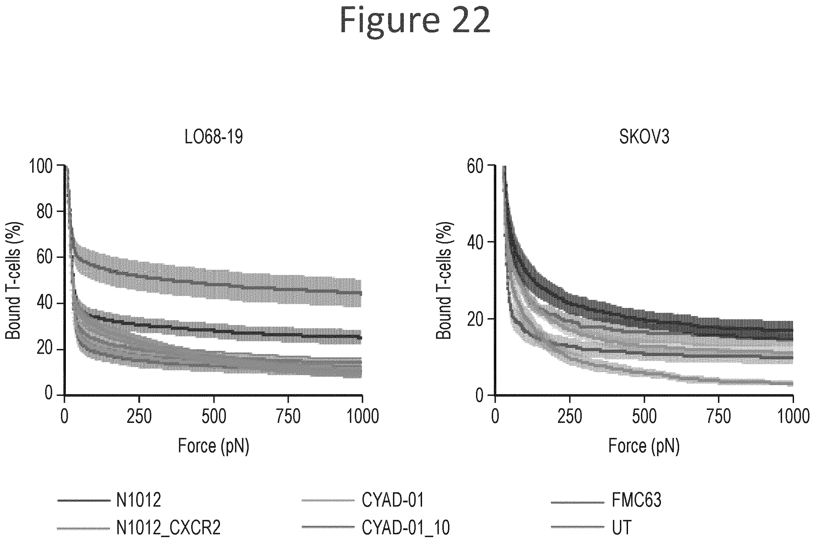

**Figure 22** shows avidity plots of the indicated CAR T-cells for Lo68_CD19 cells (left panel) and SKOV-3 cells (right panel) upon application of an acoustic force ramp.

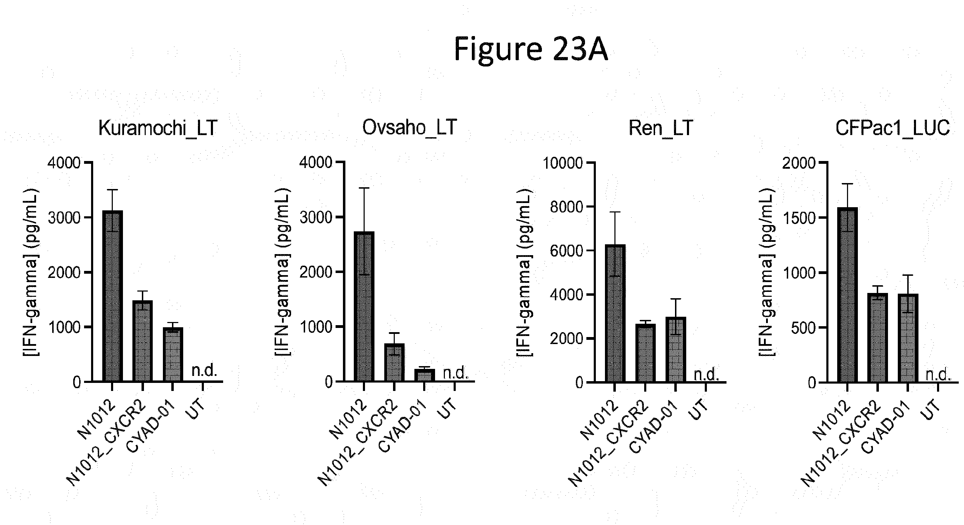

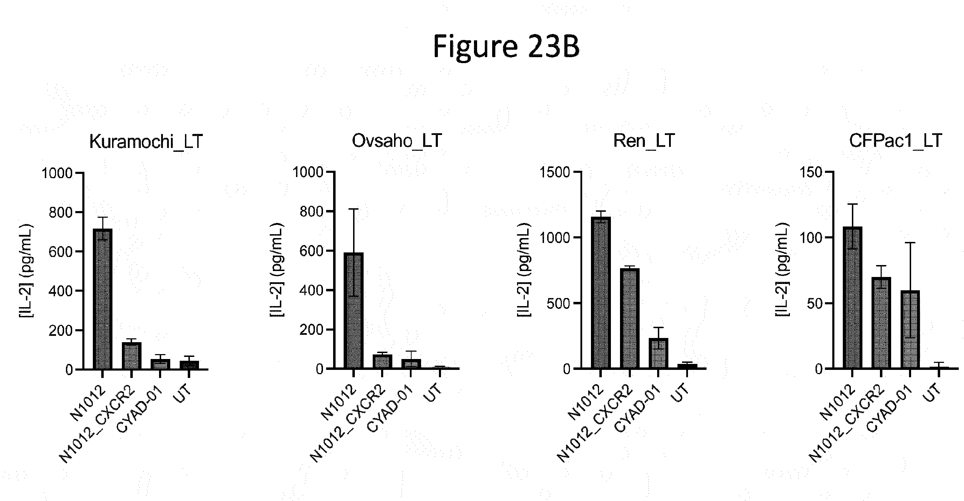

**Figures 23A-23B** show the levels of cytokines IFN-γ **(****Figure 23A****)** and IL-2 **(****Figure 23B****)** secreted by N1012, N1012_CXCR2, CYAD-01 replica and untransduced T-cells when co-cultured with four different tumor cell lines. Supernatants were removed from the co-cultured cells and assessed for cytokine presence by ELISA.

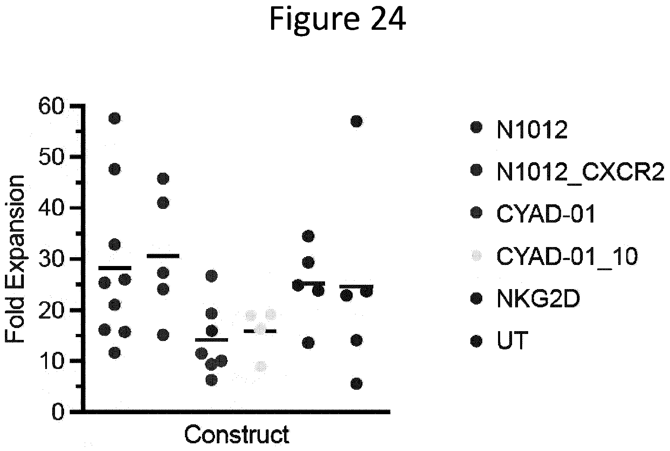

**Figure 24** shows that expansion of unstimulated N1012 and N1012_CXCR2 T-cells over 12 days exceeds expansion of NKG2D and untransduced T-cells whereas CYAD-01 replica and CYAD-01_10 T-cells demonstrate substantially poorer expansion compared to control T-cells.

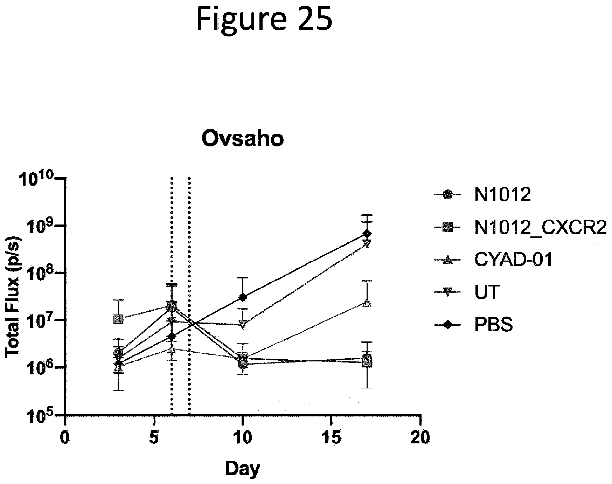

**Figure 25** shows bioluminescence emission from firefly luciferase (ffLuc)-expressing Ovsaho tumor xenografts in mice treated with PBS or CAR-T cells (N1012, N1012_CXCR2, CYAD-01 replica, or untransduced). T-cells were administered i.p. on day 6 and intravenously (i.v.) on day 7 after tumor inoculation (indicated by vertical dotted lines). Tumor development is measured as total flux (photons/sec).

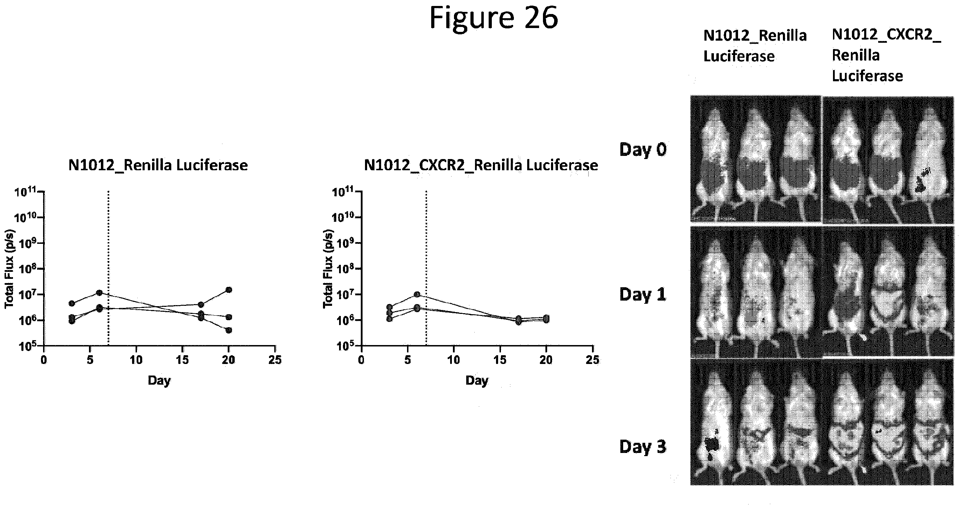

**Figure 26** shows results of bioluminescence imaging demonstrating anti-tumor activity in ffLuc Ovsaho xenograft-containing mice treated with N1012 and N1012_CXCR2 T-cells (left and middle panels) and improved trafficking of N1012_CXCR2 T-cells to tumor sites compared to N1012 T-cells (right panel). Tumor development is measured by firefly luciferase expression as total flux (photons/sec). T-cell trafficking is monitored by expression of renilla luciferase, quantified once again as total flux (photons/sec).

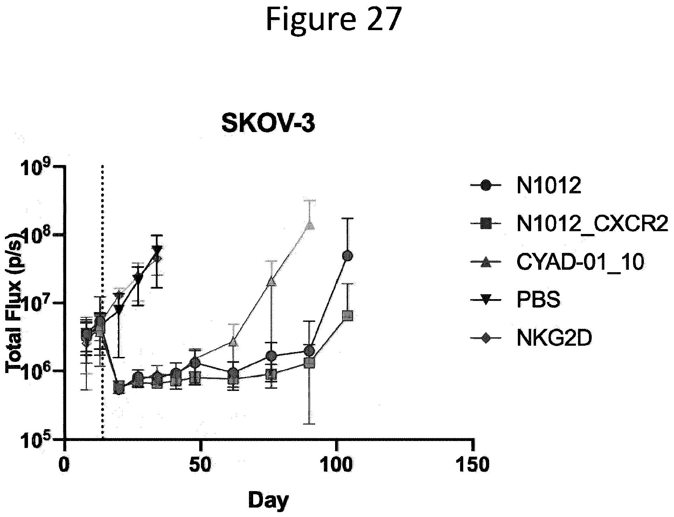

**Figure 27** shows development of ffLuc-expressing SKOV-3 tumor xenografts in mice treated with PBS or CAR-T cells (N1012, N1012_CXCR2, CYAD-01_10, or control NKG2D construct) 7 days after tumor inoculation. Tumor development is measured by firefly luciferase expression as total flux (photons/sec).

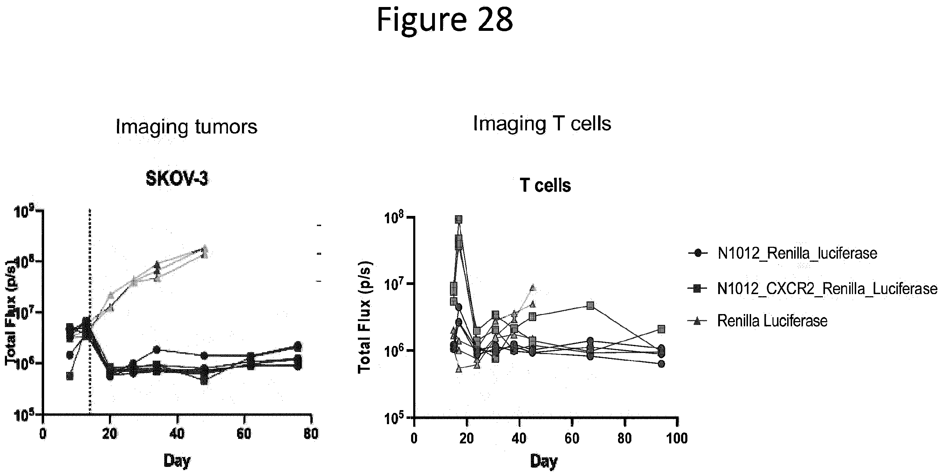

**Figure 28** shows results of tumor growth (left panel) and T-cell trafficking (right panel) assessments in SKOV-3 xenograft-containing mice treated with CAR T-cells (N1012, N1012_CXCR2, or reporter control "Renilla Luciferase").

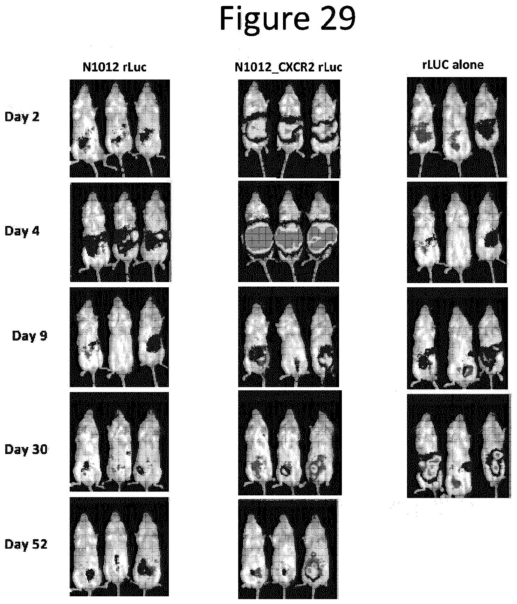

**Figure 29** shows results of bioluminescence imaging of the indicated renilla luciferase-expressing CAR T-cells following i.v. administration to NSG mice with an i.p. SKOV-3 tumor xenograft. Imaging of T-cells that express rLuc N1012, rLuc N1012_CXCR2,or rLuc alone at various time points is shown.

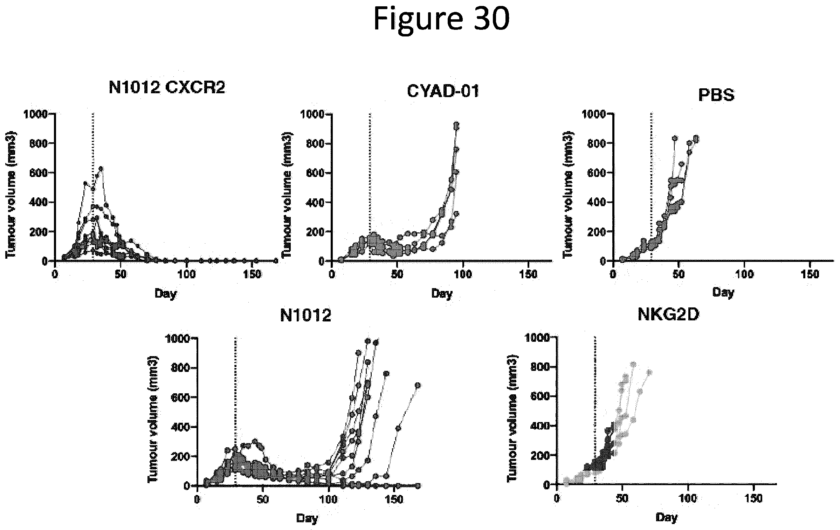

**Figure 30** shows development of subcutaneous (s.c.) CFPac-1 pancreatic tumor xenografts in mice following administration of PBS or CAR T-cells (N1012_CXCR2, CYAD-01 replica, N1012 or NKG2D).

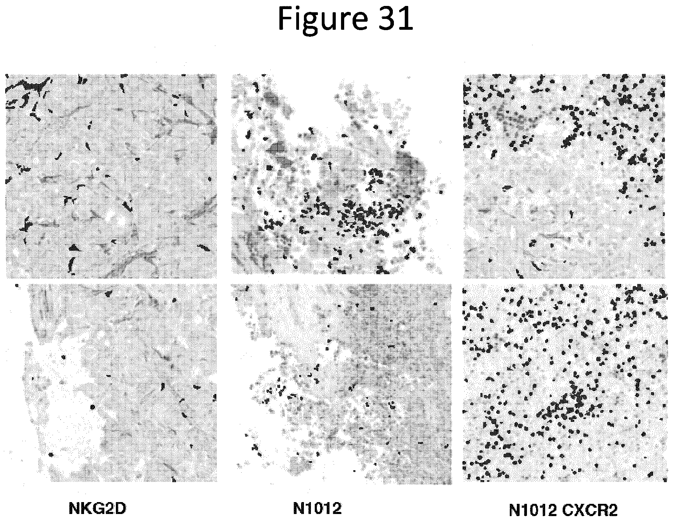

**Figure 31** shows CD3 immunohistochemistry images of s.c. CFPac-1 tumors demonstrating improved infiltration of N1012_CXCR2 T-cells compared to N1012 or NKG2D T-cells.

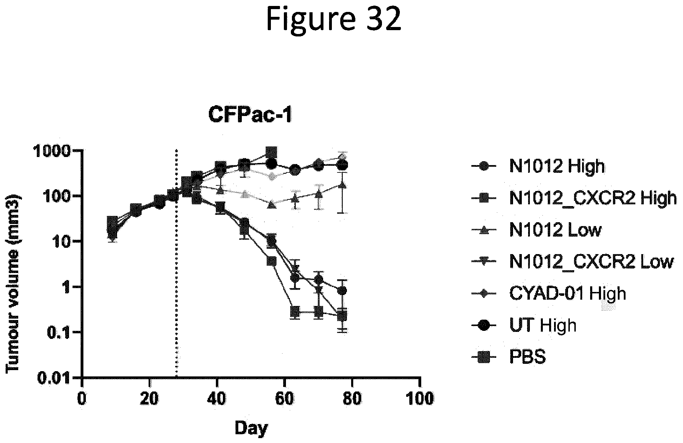

**Figure 32** shows development over time of s.c. CFPac-1 pancreatic tumor xenografts in mice treated 28 days after tumor inoculation with PBS, high dose (10×10⁶) CAR T-cells (N1012, N1012_CXCR2, CYAD-01 replica or untransduced) or low dose (4×10⁶) CAR T-cells (N1012, or N1012_CXCR2).

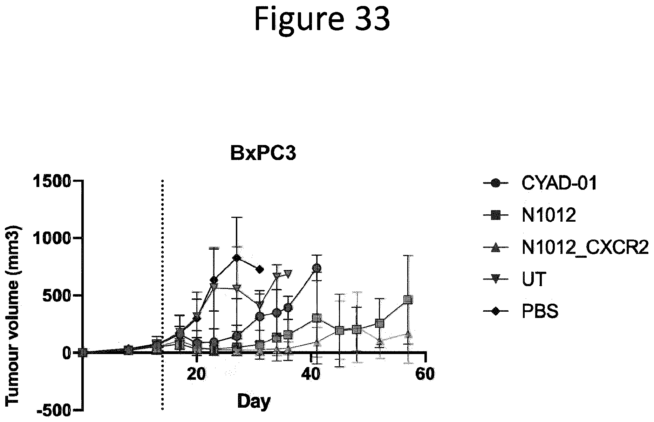

**Figure 33** shows development of s.c. BxPC3 pancreatic tumor xenografts in mice following administration of PBS or 10 × 10⁶ CAR T-cells (CYAD-01 replica, N1012, N1012_CXCR2,or untransduced) 14 days following tumor inoculation.

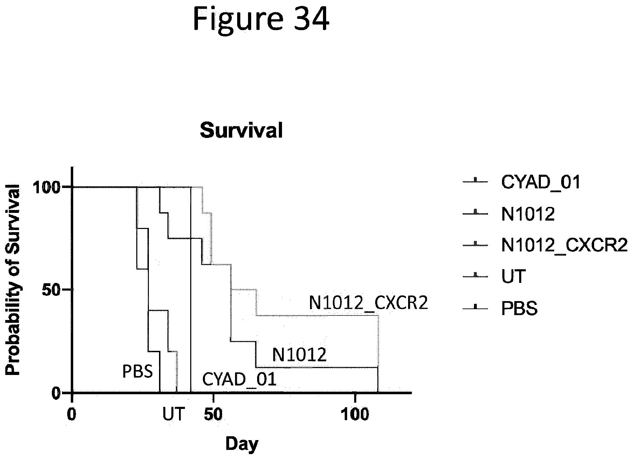

**Figure 34** is a survival curve of s.c. BxPC3 tumor xenograft-containing mice treated with PBS or 10 × 10⁶ CAR T-cells (CYAD-01 replica, N1012, N1012_CXCR2, or untransduced).

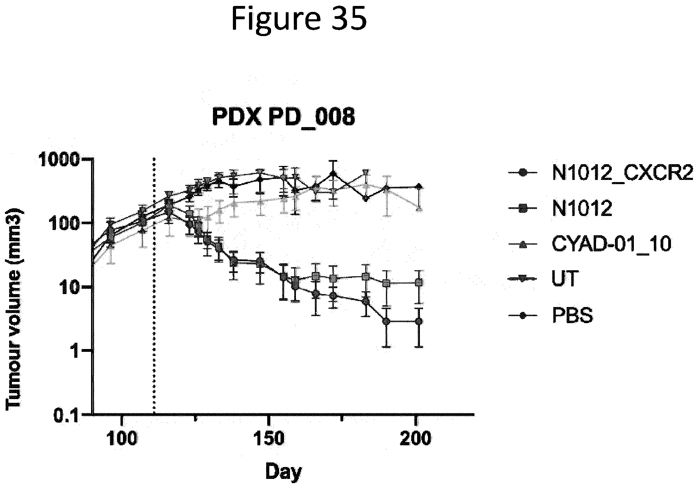

**Figure 35** shows development of a s.c. mesothelioma patient derived xenograft tumor in mice (PDX PD_008) following administration of PBS or 10 × 10⁶ CAR T-cells (N1012_CXCR2, N1012, CYAD-01_10, or untransduced) 111 days following tumor engraftment.

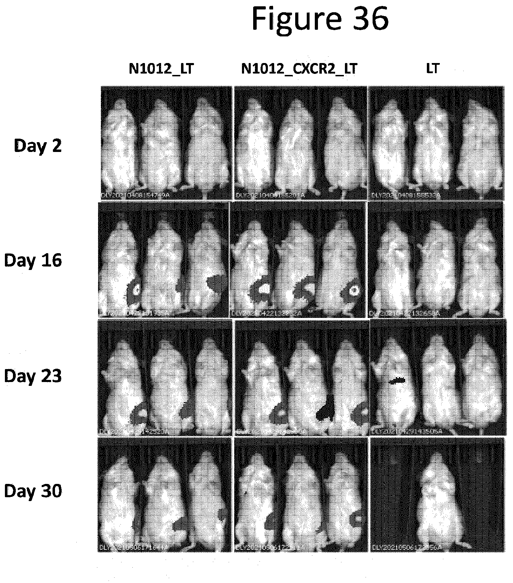

**Figure 36** shows results of bioluminescence imaging of ffLuc/ dsTomato (LT)-expressing CAR T-cells following administration to mesothelioma patient derived xenograft tumor-containing NSG mice. Mice were treated with 10 × 10⁶ CAR T-cells (N1012_LT, N1012_CXCR2_LT, or LT) at various time points following T-cell administration.

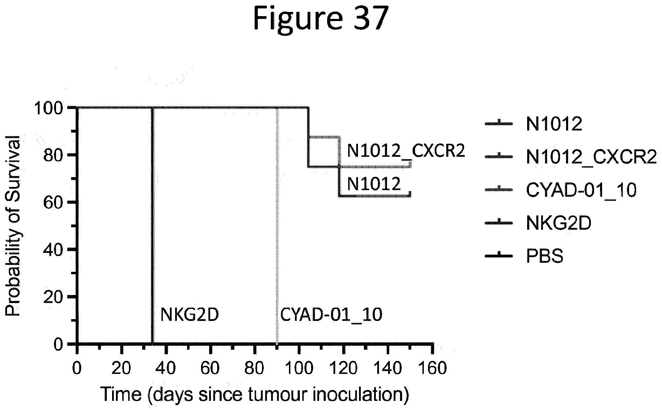

**Figure 37** shows a Kaplan-Meier survival curve of NSG mice bearing i.p. SKOV-3 tumors that were treated i.p. with either PBS or 10 × 10⁶ CAR-T cells (N1012, N1012_CXCR2, CYAD-01_10, or control NKG2D) 7 days after tumor inoculation.

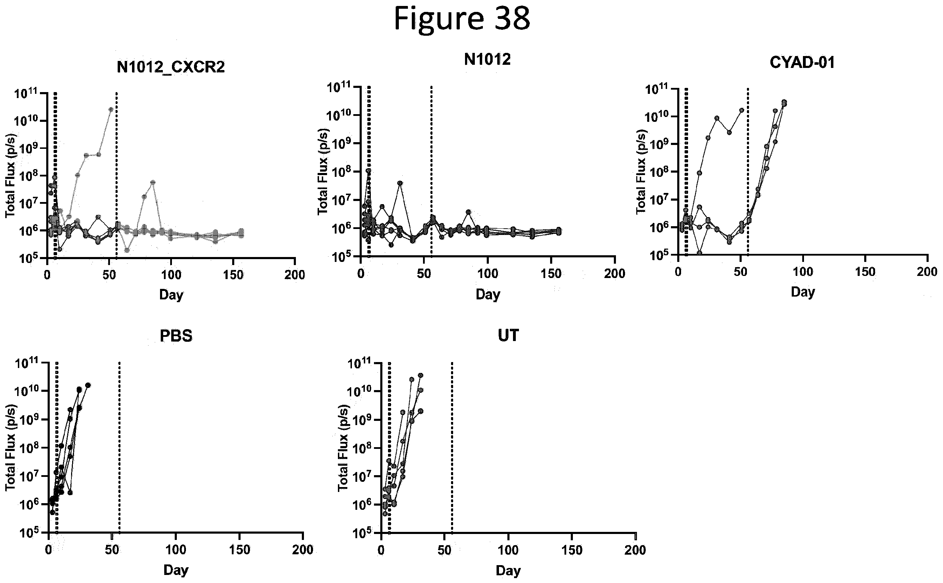

**Figure 38** shows development of i.p. ffLuc Ovsaho tumor xenografts in NSG mice treated with PBS or CAR-T cells (N1012, N1012_CXCR2, CYAD-01 replica, or untransduced). 5 × 10⁶ T-cells were administered i.p. on day 6 post tumor inoculation and a further dose of 5 × 10⁶ T-cells were administered i.v. on the following day. Mice were re-challenged with ffLuc Ovsaho cells 56 days after initial tumor inoculation.

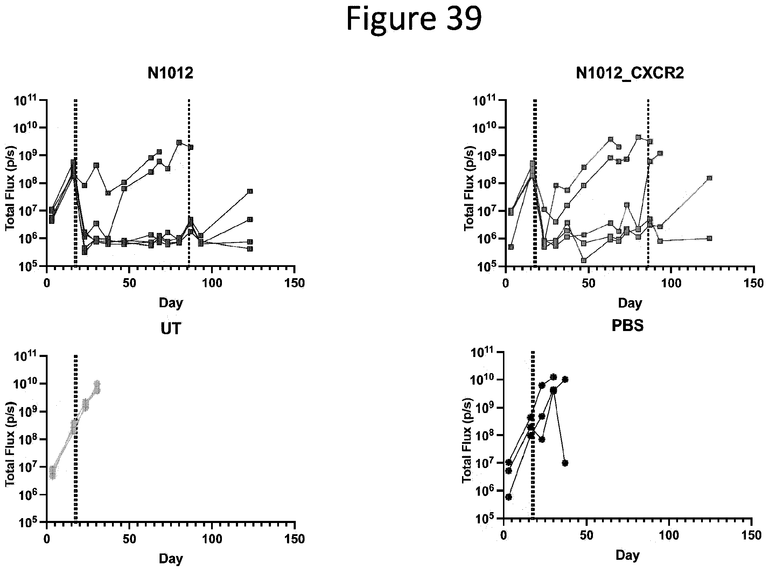

**Figure 39** shows development of i.p. ffLuc Kuramochi tumor xenografts in NSG mice treated with PBS or CAR-T cells (N1012, N1012_CXCR2, or untransduced). 5 × 10⁶ T-cells were administered i.p. on day 17 post tumor inoculation and a further dose of 5 × 10⁶ T-cells were administered i.v. on the following day. Tumor-free mice were re-challenged with ffLuc Kuramochi cells 86 days after initial tumor inoculation.

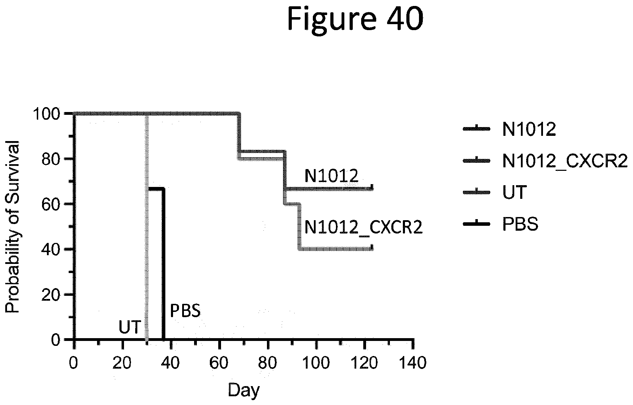

**Figure 40** is a Kaplan-Meier survival curve of mice bearing i.p. ffLuc Kuramochi tumors that were treated with PBS or CAR-T cells (N1012, N1012_CXCR2, or untransduced) 17 and 18 days after tumor inoculation, as described in Figure 39.

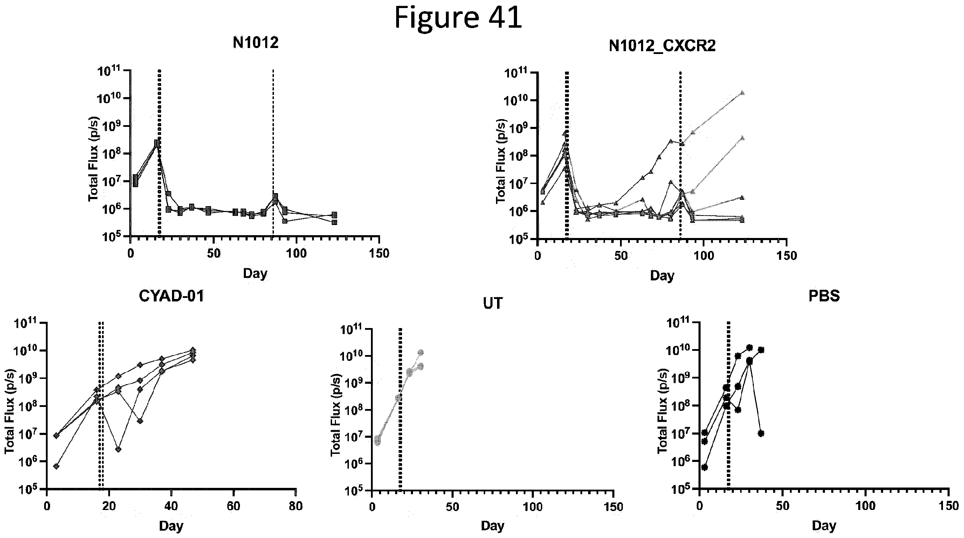

**Figure 41** shows development of i.p. ffLuc Kuramochi tumor xenografts in NSG mice treated with PBS or CAR-T cells (N1012, N1012_CXCR2, or untransduced). 5 × 10⁶ T-cells were administered i.p. on day 17 post tumor inoculation and a further dose of 5 × 10⁶ T-cells were administered i.v. on the following day. Tumor-free mice were re-challenged with ffLuc Kuramochi cells 86 days after initial tumor inoculation.

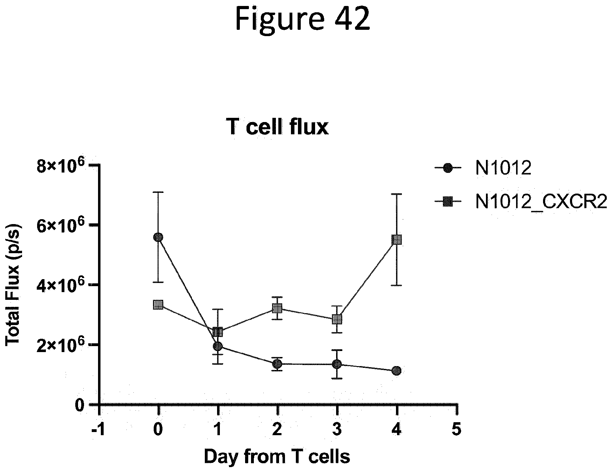

**Figure 42** shows results of bioluminescence imaging of Kuramochi tumor xenograft-containing NSG mice treated with rLuc labeled CAR T-cells (N1012_rLuc, N1012_CXCR2_rLuc). T-cell infiltration was assessed over 4 days by bioluminescence imaging following coelenterazine administration.

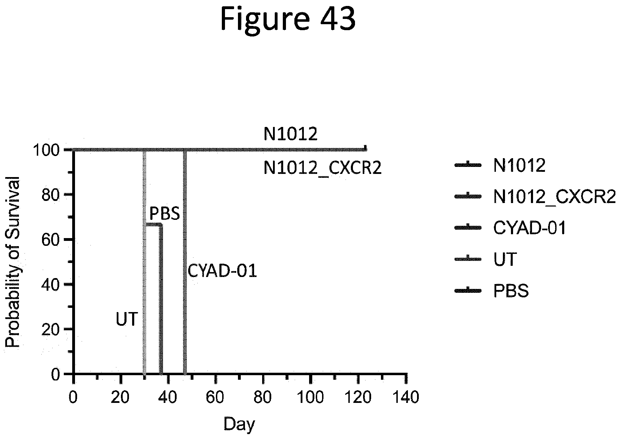

**Figure 43** is a Kaplan-Meier survival curve of NSG mice bearing Kuramochi tumors that were treated with PBS or CAR-T cells (N1012, N1012_CXCR2, Cyad-01 replica or untransduced T-cells). 5 × 10⁶ T-cells were administered i.p. on day 17 post tumor inoculation and a further dose of 5 × 10⁶ T-cells were administered i.v. on the following day.

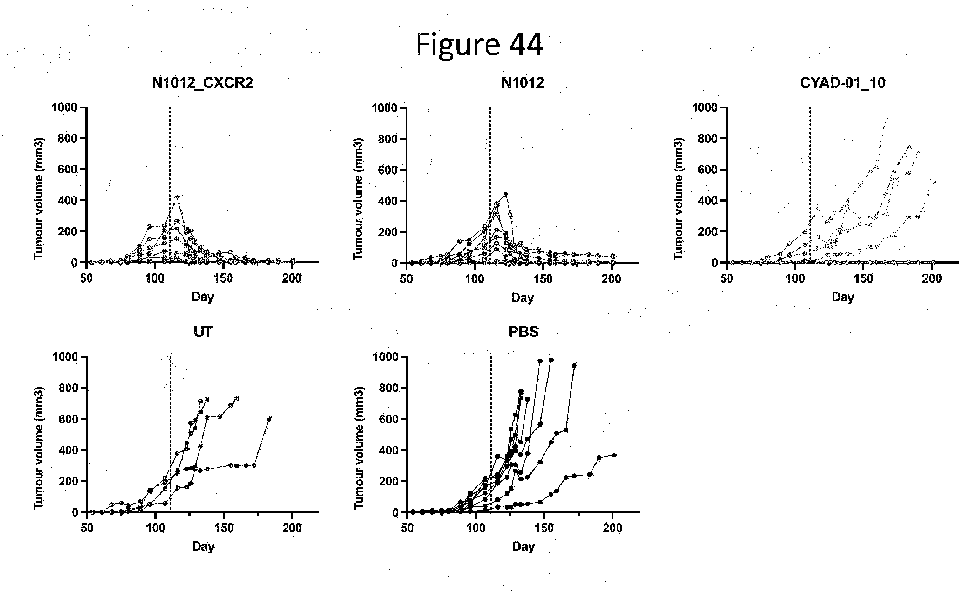

**Figure 44** shows tumor volumes as assessed by caliper measurements in individual NSG mice with a S.C. mesothelioma patient derived xenograft following i.v. administration of PBS or 10 × 10⁶ CAR T-cells (N1012_CXCR2, N1012, CYAD-01_10, or untransduced T-cells) 111 days following tumor engraftment.

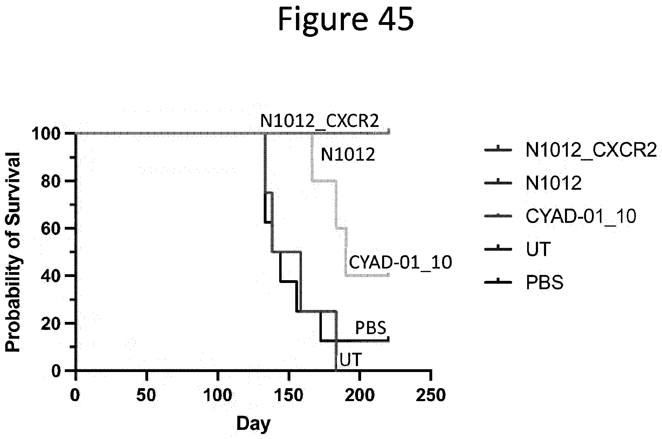

**Figure 45** is a Kaplan-Meier survival curve of NSG mice with a S.C. mesothelioma patient derived xenograft that were treated i.v. with PBS or 10 × 10⁶ CAR T-cells (N1012_CXCR2, N1012, CYAD-01_10, or untransduced T-cells) 111 days following tumor engraftment.

### 4. DETAILED DESCRIPTION

### 4.1. Definitions

Terms used in the claims and specification are defined as set forth below unless otherwise specified.

The term "mammal" as used herein includes both humans and non-humans and include but is not limited to humans, non-human primates, canines, felines, murines, bovines, equines, and porcines.

The term "murine" refers to a rodent of the subfamily Murinae. The term "murine" comprises rat and mouse.

The term "chimeric NKG2D polypeptide" refers to an NKG2D receptor formed of domains from two or more different organisms. Chimeric NKG2D polypeptides are described in more detail in WO 2021/234163.

The term percent "identity," in the context of two or more nucleic acid or polypeptide sequences, refers to two or more sequences or subsequences that have a specified percentage of nucleotides or amino acid residues that are the same, when compared and aligned for maximum correspondence, as measured using one of the sequence comparison algorithms described below (e.g., BLASTP and BLASTN or other algorithms available to persons of skill) or by visual inspection. Depending on the application, the percent "identity" can exist over a region of the sequence being compared, e.g., over a functional domain, or, alternatively, exist over the full length of the two sequences to be compared.

For sequence comparison, typically one sequence acts as a reference sequence to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

For purposes herein, percent identity and sequence similarity is performed using the BLAST algorithm, which is described in Altschul et al., J. Mol. Biol. 215:403-410 (1990). Software for performing BLAST analyses is publicly available through the National Center for Biotechnology Information (www.ncbi.nlm.nih.gov/).

As used herein, the term "subject" broadly refers to any animal, including but not limited to, human and non-human animals (e.g., dogs, cats, cows, horses, sheep, pigs, poultry, fish, crustaceans, etc.).

As used herein, the term "effective amount" refers to the amount of a composition (e.g., a synthetic peptide) sufficient to effect beneficial or desired results. An effective amount can be administered in one or more administrations, applications or dosages and is not intended to be limited to a particular formulation or administration route.

The term "therapeutically effective amount" is an amount that is effective to ameliorate a symptom of a disease. A therapeutically effective amount can be a "prophylactically effective amount" as prophylaxis can be considered therapy.

As used herein, the terms "administration" and "administering" refer to the act of giving a drug, prodrug, or other agent, or therapeutic treatment (e.g., peptide) to a subject or *in vivo, in vitro,* or *ex vivo* cells, tissues, and organs. Exemplary routes of administration to the human body can be through space under the arachnoid membrane of the brain or spinal cord (intrathecal), the eyes (ophthalmic), mouth (oral), skin (topical or transdermal), nose (nasal), lungs (inhalant), oral mucosa (buccal or lingual), ear, rectal, vaginal, by injection (e.g., intravenously, subcutaneously, intratumorally, intraperitoneally, etc.) and the like.

As used herein, the term "treatment" means an approach to obtaining a beneficial or intended clinical result. The beneficial or intended clinical result can include alleviation of symptoms, a reduction in the severity of the disease, inhibiting an underlying cause of a disease or condition, steadying diseases in a non-advanced state, delaying the progress of a disease, and/or improvement or alleviation of disease conditions.

As used herein, the term "pharmaceutical composition" refers to the combination of an active ingredient with a carrier, inert or active, making the composition especially suitable for therapeutic or diagnostic use *in vitro, in vivo* or *ex vivo.*

The terms "pharmaceutically acceptable" or "pharmacologically acceptable," as used herein, refer to compositions that do not substantially produce adverse reactions, e.g., toxic, allergic, or immunological reactions, when administered to a subject.

As used herein, the words "comprise" and "contain" and variations of the words, for example, "comprising" and "comprises", mean "including but not limited to", and do not exclude other components, integers or steps. Moreover, the singular encompasses the plural unless the context otherwise requires: in particular, where the indefinite article is used, the specification is to be understood as contemplating plurality as well as singularity, unless the context requires otherwise.

It must be noted that, as used in the specification and the appended claims, the singular forms "a," "an" and "the" include plural referents unless the context clearly dictates otherwise.

### 4.2. Immunoresponsive cells comprising NKG2D, CXCR2, and, optionally, DAP10, DAP12, or DAP10/DAP12 fusion polypeptides

Immunoresponsive cells of the disclosure comprise an NKG2D polypeptide, a CXCR2 polypeptide, and, optionally, a DNAX-activating protein 10 (DAP10) polypeptide or functional variant thereof, a DNAX-activating protein 12 (DAP12) polypeptide or functional variant thereof, and/or a fusion polypeptide comprising a DAP10 polypeptide or functional variant thereof and a DAP12 polypeptide or functional variant thereof.

### 4.2.1. DAP10 polypeptides and functional variants thereof

DAP10 polypeptide may be endogenously expressed in certain organisms and certain cell types. In an embodiment, the DAP10 polypeptide or variant thereof is endogenous. Alternatively, the DAP10 polypeptide or variant thereof may be exogenous.

The DAP10 polypeptide of the disclosure may be mammalian, for example human. Wild-type human DAP10 is encoded by the amino acid sequence having UniProt accession no: Q9UBK5 (SEQ ID NO: 1). This is a 93 amino acid polypeptide. The first 18 amino acids are considered to be a signal/leader sequence, amino acids 19-48 the extracellular domain, amino acids 49-69 the transmembrane domain, and amino acids 70-93 the cytoplasmic/intracellular domain.

In one embodiment, a DAP10 polypeptide used in the fusion polypeptide of the disclosure comprises an amino acid sequence having at least about 80%, at least about 85%, at least about 90%, at least about 95%, at least about 97%, at least about 98% or at least about 99% sequence identity to the DAP10 polypeptide of SEQ ID NO: 1. In some embodiments, a DAP10 polypeptide used in the fusion polypeptide of the disclosure comprises an amino acid sequence of SEQ ID NO: 1.

In another embodiment, a functional variant DAP10 polypeptide used in the fusion polypeptide of the disclosure may comprise one or more (i.e. 1, 2, 3, 4, 5, 6, 7, 8, 9, 10 or more) point mutations that add, delete or substitute any of the amino acids of the amino acids of DAP 10 (such as that of wild-type human DAP 10 (SEQ ID NO: 1)).

Truncated versions of a DAP10 polypeptide may also be used in fusion polypeptides of the disclosure. For example, a truncated version of DAP10 comprising only amino acids 19-93 of SEQ ID NO: 1 (i.e. lacking amino acids 1-18, the signal/leader sequence) may be used in the fusion polypeptide of the disclosure. Such a sequence is referred to as SEQ ID NO: 2 herein. Other truncated versions may comprise amino acids 19-69 of SEQ ID NO: 1, such a sequence comprising merely the extracellular and transmembrane domains of DAP10, and referred to herein as SEQ ID NO: 3. A further truncated version of DAP10 used in the invention may comprise amino acids 1-71 of SEQ ID NO: 1 (i.e. the signal/leader sequence, extracellular domain, transmembrane domain and 2 amino acids from the cytoplasmic/intracellular domain), referred to as SEQ ID NO: 4 herein. A further truncated version of DAP10 used in the invention may comprise amino acids 19-71 of SEQ ID NO: 1 (i.e. the extracellular domain, transmembrane domain and 2 amino acids from the cytoplasmic/intracellular domain), referred to as SEQ ID NO: 5 herein. A further truncated version of DAP10 used in the invention may comprise amino acids 70-93 of SEQ ID NO: 1 (i.e. the intracellular domain), referred to as SEQ ID NO: 6 herein. A yet further truncated version of DAP10 used in the invention may comprise amino acids 49-93 of SEQ ID NO: 1 (i.e. the transmembrane and cytoplasmic/intracellular domains), referred to as SEQ ID NO: 7 herein. A yet further truncated version of DAP10 used in the invention may comprise amino acids 49-69 of SEQ ID NO: 1 (i.e. the transmembrane domain), referred to as SEQ ID NO: 8 herein.

Other mutated versions or truncated versions of the DAP10 polypeptide are also suitable for use in the disclosure. In some embodiments, mutated versions or truncated versions that are used as functional variants of DAP10 polypeptides in the disclosure retain the activity of the wild type polypeptide shown in SEQ ID NO: 1.

In one embodiment, a functional variant of a DAP10 polypeptide of the disclosure retains at least 10% (for example 20%, 30%, 40%, 50%, 60%, 70%, 80%, 90%, 95%, 97%, 98%, 99% or more) of the activity of the wild type polypeptide shown in SEQ ID NO: 1. In one embodiment, the activity may be measured by assessment of tyrosine phosphorylation of DAP10 and/or recruitment and activation of the p85 subunit of phosphatidylinositol 3-kinase and the downstream anti-apoptotic kinase, AKT.

As the skilled person will appreciate, DAP10 typically exists as a homodimer. Thus, in an embodiment, the immunoresponsive cell comprises a DAP10 homodimer comprising two DAP10 polypeptides according to the disclosure. In an embodiment, the immunoresponsive cell comprises a DAP10 heterodimer, each peptide of the DAP10 heterodimer comprising a different DAP10 polypeptide of the disclosure.

### 4.2.2. DAP12 polypeptides and functional variants thereof

DAP12 polypeptide may be endogenously expressed in certain organisms and certain cell types. In an embodiment, the DAP12 polypeptide or variant thereof is endogenous. Alternatively, the DAP12 polypeptide or variant thereof may be exogenous.

The DAP12 polypeptide of the disclosure may be mammalian, for example human. Wild-type human DAP12 is encoded by the amino acid sequence having UniProt accession no: 043914 (SEQ ID NO: 9). The first 21 amino acids are considered to be a signal/leader sequence, amino acids 22-40 the extracellular domain, amino acids 41-61 the transmembrane domain, and amino acids 62-113 the cytoplasmic/intracellular domain.

In one embodiment, a DAP12 polypeptide used in the fusion polypeptide of the disclosure comprises an amino acid sequence having at least about 80%, at least about 85%, at least about 90%, at least about 95%, at least about 97%, at least about 98% or at least about 99% sequence identity to the DAP12 polypeptide of SEQ ID NO: 9. In some embodiments, a DAP12 polypeptide used in the fusion polypeptide of the disclosure comprises an amino acid sequence of SEQ ID NO: 9.

In another embodiment, a functional variant DAP12 polypeptide used in the fusion polypeptide of the disclosure may comprise one or more (i.e. 1, 2, 3, 4, 5, 6, 7, 8, 9, 10 or more) point mutations that add, delete or substitute any of the amino acids of the amino acids of DAP12 (such as that of wild-type human DAP12 (SEQ ID NO: 9)).

Truncated versions of a DAP12 polypeptide may also be used in fusion polypeptides of the disclosure. For example, a truncated version of DAP12 comprising only amino acids 22-113 of SEQ ID NO: 9 (i.e. lacking amino acids 1-21, the signal/leader sequence) may be used in the fusion polypeptide of the disclosure. Such a sequence is referred to as SEQ ID NO: 10 herein. Other truncated versions may comprise amino acids 62-113 of SEQ ID NO: 9, such a sequence comprising merely the cytoplasmic/intracellular domain of DAP12, and referred to herein as SEQ ID NO: 11. Other truncated versions may comprise amino acids 41-61 of SEQ ID NO: 9 (i.e. the transmembrane domain), referred to as SEQ ID NO: 12 herein. Another truncated version may comprise amino acids 22-61 of SEQ ID NO: 9 (i.e. the extracellular and transmembrane domains), referred to as SEQ ID NO: 13 herein.

Other mutated versions or truncated versions of the DAP12 polypeptide are also suitable for use in the disclosure. In some embodiments, mutated versions or truncated versions that are used as functional variants of DAP12 polypeptides in the disclosure retain the activity of the wild type polypeptide shown in SEQ ID NO: 9.

In one embodiment, a functional variant of a DAP12 polypeptide of the disclosure retains at least 10% (for example 20%, 30%, 40%, 50%, 60%, 70%, 80%, 90%, 95%, 97%, 98%, 99% or more) of the activity of the wild type polypeptide shown in SEQ ID NO: 9. In one embodiment, the activity may be measured using functional assays, such as MTT and measuring cytokine secretion by ELISA.

The skilled person will appreciate that DAP12 typically exists as a homodimer. Thus, in an embodiment, the immunoresponsive cell comprises a DAP12 homodimer comprising two DAP12 polypeptides according to the disclosure. In an embodiment, the immunoresponsive cell comprises a DAP12 heterodimer, each peptide of the DAP12 heterodimer comprising a different DAP12 polypeptide of the disclosure.

### 4.2.3. Polypeptides that associate with DAP10/DAP12 fusion polypeptides

The fusion polypeptide of the invention may associate with other polypeptides. Such association may be due to electrostatic forces, such as provided by complementary charged amino acids.

One example of such a polypeptide that may associate with a fusion polypeptide of the invention is the NKG2D polypeptide (Wu et al., 2000, J. Exp. Med., 192(7):1059-1067 and Rosen et al., 2004, J. Immunol., 173(4):2470-2478).

In one embodiment, such polypeptides may be genetically encoded as part of a contiguous chimeric construct with the gene that encodes for the fusion polypeptide of the disclosure. The fusion polypeptide and other polypeptide may then be separated during translation (e.g. using a ribosomal skip peptide) or by post translation cleavage (e.g. using a furin cleavage site). The fusion polypeptide and other polypeptide may therefore be joined by an optional linker. Such a linker may comprise a cleavage site to facilitate cleavage.

### 4.2.3.1. NKG2D polypeptides and functional variants thereof

The NKG2D polypeptide of the disclosure may be mammalian, for example human. Wild-type human NKG2D is encoded by the amino acid sequence having UniProt accession no: P26718 (SEQ ID NO: 14). The polypeptide is considered to comprise a cytoplasmic domain (amino acids 1-51), a transmembrane domain (amino acids 52-72) and an extracellular domain (amino acids 73-216).

In one embodiment, a NKG2D polypeptide used in the disclosure comprises an amino acid sequence having at least about 80%, at least about 85%, at least about 90%, at least about 95%, at least about 97%, at least about 98% or at least about 99% sequence identity to the NKG2D polypeptide of SEQ ID NO: 14. In some embodiments, a NKG2D polypeptide used in the disclosure comprises an amino acid sequence of SEQ ID NO: 14.

In another embodiment, a functional variant NKG2D polypeptide used in the disclosure may comprise one or more (i.e., 1, 2, 3, 4, 5, 6, 7, 8, 9, 10 or more) point mutations that add, delete or substitute any of the amino acids of the amino acids of NKG2D (such as that of wild-type human NKG2D (SEQ ID NO: 14)).

Truncated versions of a NKG2D polypeptide may also be used in polypeptides of the disclosure. For example, a truncated version of NKG2D comprising only amino acids 73-216 of SEQ ID NO: 14 (i.e., the extracellular domain) may be used in the disclosure. Such a sequence is referred to as SEQ ID NO: 15 herein. Other truncated versions may comprise amino acids 82-216 of SEQ ID NO: 14, such a sequence comprising part of the extracellular domain of NKG2D and referred to herein as SEQ ID NO: 16. A further truncated version comprises amino acids 52-216 of SEQ ID NO: 14 (i.e. the transmembrane and extracellular domains), referred to as SEQ ID NO: 17 herein.

Other mutated versions or truncated versions of the NKG2D polypeptide are also suitable for use in the disclosure. Of course, any such mutated versions or truncated versions that are used as functional variants of NKG2D polypeptides in the disclosure should preferably retain the activity of the wild type polypeptide shown in SEQ ID NO: 14.

In one embodiment, a functional variant of a NKG2D polypeptide of the disclosure retains at least 10% (for example 20%, 30%, 40%, 50%, 60%, 70%, 80%, 90%, 95%, 97%, 98%, 99% or more) of the activity of the wild type polypeptide shown in SEQ ID NO: 14. In one embodiment, the activity may be measured using flow cytometry to confirm continued binding to NKG2D ligands and through various cell culture assays (such as MTT and ELISA) aimed at confirming target cell lysis, cytokine secretion and co-stimulation.

In some embodiments, the NKG2D polypeptide is a chimeric polypeptide. In particular embodiments, the NKG2D polypeptide is a human-murine chimeric polypeptide. In an embodiment, the chimeric NKG2D polypeptide comprises from N terminus to C terminus the murine NKG2D transmembrane domain or a variant thereof and a human NKG2D extracellular domain or a variant thereof. Chimeric NKG2D polypeptides are described in more detail in WO 2021/234163, the disclosure of which is herein incorporated by reference in its entirety.

### 4.2.3.2. CXCR2 polypeptides and functional variants thereof

The CXCR2 polypeptide of the present disclosure may be mammalian, for example human. Wild-type human CXCR2 is encoded by the amino acid sequence having UniProt accession no: P25025 (SEQ ID NO: 87).

In one embodiment, the CXCR2 polypeptide of the disclosure comprises an amino acid sequence having at least about 80%, at least about 85%, at least about 90%, at least about 95%, at least about 97%, at least about 98%, or at least about 99% sequence identity to the CXCR2 polypeptide of SEQ ID NO: 87. In one embodiment, the CXCR2 polypeptide of the disclosure has the amino acid sequence of SEQ ID NO: 87.

In one embodiment, the CXCR2 polypeptide of the disclosure is a functional variant of the CXCR2 polypeptide of SEQ ID NO: 87. In one embodiment, a functional variant CXCR2 polypeptide comprises one or more (e.g., 1, 2, 3, 4, 5, 6, 7, 8, 9, 10 or more) point mutations that add, delete, or substitute amino acids of CXCR2 (e.g., SEQ ID NO: 87).

In one embodiment, a functional variant CXCR2 polypeptide of the disclosure retains at least 10% (for example, 20%, 30%, 40%, 50%, 60%, 70%, 80%, 90%, 95%, 97%, 98%, 99% or more) of the activity of wild-type human CXCR2 (SEQ ID NO: 87). In one embodiment, activity of the polypeptide is measured using flow cytometry to confirm binding of CXCR2 to ligand (e.g., IL-8). In one embodiment, activity of the polypeptide is measured through cell culture assays known in the art.

### 4.2.4. Linkers

The DAP10 and DAP12 moieties of the DAP10/DAP12 fusion polypeptides described herein can be directly bonded to each other in a contiguous polypeptide chain, or may be indirectly bonded to each other through a suitable linker. The linker may be a peptide linker. Peptide linkers are commonly used in fusion polypeptides and methods for selecting or designing linkers are well-known. (See, e.g., Chen X et al., 2013, Adv. Drug Deliv. Rev. 65(10):135701369 and Wriggers W et al., 2005, Biopolymers 80:736-746.). Linkers may also be used to join the fusion polypeptide of the disclosure to another polypeptide (such as a NKG2D polypeptide) in a chimeric construct as described above.

Peptide linkers generally are categorized as i) flexible linkers, ii) helix forming linkers, and iii) cleavable linkers, and examples of each type are known in the art. In one example, a flexible linker is included in the fusion polypeptides described herein. Flexible linkers may contain a majority of amino acids that are sterically unhindered, such as glycine and alanine. The hydrophilic amino acid Ser is also conventionally used in flexible linkers. Examples of flexible linkers include, without limitation: polyglycines (e.g., (Gly)₄ and (Gly)s), polyalanines poly(Gly-Ala), and poly(Gly-Ser) (e.g., (Glyₙ-Serₙ)ₙ or (Serₙ-Glyₙ)ₙ, wherein each n is independently an integer equal to or greater than 1).

Peptide linkers can be of a suitable length. The peptide linker sequence may be at least 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, or more amino acid residues in length. For example, a peptide linker can be from about 5 to about 50 amino acids in length; from about 10 to about 40 amino acids in length; from about 15 to about 30 amino acids in length; or from about 15 to about 20 amino acids in length. Variation in peptide linker length may retain or enhance activity, giving rise to superior efficacy in activity studies. The peptide linker sequence may be comprised of naturally or non-naturally occurring amino acids, or a mixture of both naturally and non-naturally occurring amino acids.

In some aspects, the amino acids glycine and serine comprise the amino acids within the linker sequence. In certain aspects, the linker region comprises sets of glycine repeats (GSG3)ₙ (SEQ ID NO: 18), where n is a positive integer equal to or greater than 1 (for example 1 to about 20). More specifically, the linker sequence may be GSGGG (SEQ ID NO: 19). The linker sequence may be GSGG (SEQ ID NO: 20). In certain other aspects, the linker region orientation comprises sets of glycine repeats (SerGly₃)ₙ, where n is a positive integer equal to or greater than 1 (for example 1 to about 20) (SEQ ID NO: 21).

In other embodiments, a linker may contain glycine (G) and serine (S) in a random or a repeated pattern. For example, the linker can be (GGGGS)ₙ (SEQ ID NO: 22), wherein n is an integer ranging from 1 to 20, for example 1 to 4. In a particular example, n is 4 and the linker is GGGGSGGGGSGGGGSGGGGS (SEQ ID NO: 23). In another particular example, n is 3 and the linker is GGGGSGGGGSGGGGS (SEQ ID NO: 24).

In other embodiments, a linker may contain glycine (G), serine (S) and proline (P) in a random or repeated pattern. For example, the linker can be (GPPGS)ₙ, wherein n is an integer ranging from 1 to 20, for example 1-4. In a particular example, n is 1 and the linker is GPPGS (SEQ ID NO: 25).

In general, the linker is not immunogenic when administered in a patient, such as a human. Thus, linkers may be chosen such that they have low immunogenicity or are thought to have low immunogenicity.

The linkers described herein are exemplary, and the linker can include other amino acids, such as Glu and Lys, if desired. The peptide linkers may include multiple repeats of, for example, (G₃S) (SEQ ID NO: 26), (G₄S) (SEQ ID NO: 27), (GYS) (SEQ ID NO: 28), and/or (GlySer) (SEQ ID NO: 29), if desired. In certain aspects, the peptide linkers may include multiple repeats of, for example, (SG₄) (SEQ ID NO: 30), (SG₃) (SEQ ID NO: 31), (SG₂) (SEQ ID NO: 32), (SG)₂ (SEQ ID NO: 33) or (SerGly) (SEQ ID NO: 34).

In other aspects, the peptide linkers may include combinations and multiples of repeating amino acid sequence units, such as (G₃S)+(G₄S)+(GlySer) (SEQ ID NO: 26 +SEQ ID NO: 27 +SEQ ID NO: 29). In other aspects, Ser can be replaced with Ala e.g., (G₄A) (SEQ ID NO: 35) or (G₃A) (SEQ ID NO: 36). In yet other aspects, the linker comprises the motif (EAAAK)ₙ, where n is a positive integer equal to or greater than 1, for example 1 to about 20 (SEQ ID NO: 37). In certain aspects, peptide linkers may also include cleavable linkers.

The linkers may comprise further domains and/or features, such as a furin cleavage site (RRKR)(SEQ ID NO: 38), a P2A ribosomal skip peptide (ATNFSLLKQAGDVEENPGP)(SEQ ID NO: 39) and/or a T2A ribosomal skip peptide (EGRGSLLTCGDVEENPGP)(SEQ ID NO: 40). Examples of linkers comprising these domains include SGSG + a P2A ribosomal skip peptide (SGSGATNFSLLKQAGDVEENPGP)(SEQ ID NO: 41), SGSG + a T2A ribosomal skip peptide (SGSGEGRGSLLTCGDVEENPGP)(SEQ ID NO: 42), and versions also including a furin cleavage site, i.e. furin cleavage site + SGSG + a P2A ribosomal skip peptide (RRKRSGSGATNFSLLKQAGDVEENPGP) (SEQ ID NO: 43) and furin cleavage site + SGSG + a T2A ribosomal skip peptide (RRKRSGSGEGRGSLLTCGDVEENPGP) (SEQ ID NO: 44). Alternative ribosomal skip peptides that may be used in the invention include F2A (VKQTLNFDLLKLAGDVESNPGP) (SEQ ID NO: 45) and E2A (QCTNYALLKLAGDVESNPGP) (SEQ ID NO: 46).

### 4.2.5. N-terminal sequences and C-terminal sequences

Various sequences may be attached to the N- or C-terminus of the fusion polypeptides of the disclosure, or to the NKG2D polypeptides disclosed herein. These may be functional, such as signal peptides, purification tags/sequences, or half-life extension moieties, or may simply comprise spacer sequences. Alternatively, they may comprise a function, such as a T-cell stimulatory function.

### 4.2.5.1. Purification tags and markers

A variety of tags or markers may be attached to the N- or C-terminus of the fusion polypeptides of the disclosure to assist with purification. Any affinity tag may be combined with the fusion polypeptides of the disclosure to assist with purification. Examples of such affinity tags are a His-tag, a FLAG-tag, Arg-tag, T7-tag, Strep-tag, S-tag, aptamer-tag, V5 tag, AviTagTM, myc epitope tag or any combination of these tags. In one embodiment the affinity tag is a His-tag (usually comprising 5-10 histidine residues), for example a 6His tag (i.e. HHHHHH) (SEQ ID NO: 47). In another embodiment the affinity tag is a FLAG tag (i.e. DYKDDDDK) (SEQ ID NO: 48). In another embodiment, the affinity tag is an AviTagTM (i.e. GLNDIFEAQKIEWHE) (SEQ ID NO: 49). In another embodiment, the affinity tag is a V5 tag (GKPIPNPLLGLDST) (SEQ ID NO: 50) or (IPNPLLGLD) (SEQ ID NO: 51). In another embodiment, the affinity tag is a myc epitope tag recognised by the 9e10 antibody (EQKLISEEDL) (SEQ ID NO: 52). Various other tags for use in the disclosure are well known in the art.

Combinations of such affinity tags may also be used, either comprising one or more tags at the N-terminus, one or more tags at the C-terminus, or one or more tags at each of the N-terminus and the C-terminus. Examples of such combinations include a His tag (H) combined with an AviTag (A), or a His tag (H) combined with both an AviTag (A) and a FLAG tag (F). The tags may be in either orientation, thus the AviTag/His tag may have the orientation N-AH-C or N-HA-C, while the Avi/His/FLAG tag may have the orientation N-AHF-C, N-FHA-C, etc.

In one embodiment, a fusion polypeptide according to the disclosure comprises an "AHF" tag having the sequence "GLNDIFEAQKIEWHEGGHHHHHHDYKDDDDK" (SEQ ID NO: 53). In another embodiment, a fusion polypeptide according to the disclosure comprises an "FHA" tag having the sequence "DYKDDDDKHHHHHHGGGLNDIFEAQKIEWHE" (SEQ ID NO: 54).

The CD8α leader sequence (amino acids 1-21 of UniProt: P01732 or a shortened derivative comprising amino acids 1-18), is a commonly used T-cell sequence, and is referred to as SEQ ID NO: 55 herein.

### 4.2.5.2. Co-stimulatory sequences

Various T-cell co-stimulatory activation sequences are known from previous work to engineer CAR-T cells. These may also be added to fusion polypeptides of the disclosure.

The 4-1BB endodomain (amino acids 214-255 of UniProt: Q07011) may also be used as an N- or C-terminal sequence. The 4-1BB endodomain is referred to as SEQ ID NO: 56 herein. The 4-1BB endodomain may act as a co-stimulatory domain.

The CD27 endodomain (amino acids 213-260 of UniProt: P26842) may also be used as an N- or C-terminal sequence. The CD27 endodomain is referred to as SEQ ID NO: 57 herein. The CD27 endodomain may act as a co-stimulatory domain.

The human IgG1 hinge (amino acids 218-229 or 218-232 of UniProt: P0DOX5) may also be used as an N- or C-terminal sequence. The human IgG1 hinge is referred to as SEQ ID NO: 58 or SEQ ID NO: 104.

A truncated CD8α hinge (amino acids 138-182 of Uniprot: P01732) may also be used as an N- or C-terminal sequence. The truncated CD8a hinge is referred to as SEQ ID NO: 59.

### 4.2.6. Exemplary constructs

The present disclosure provides the following exemplary fusion polypeptide constructs in Table 1:

**Table 1: Exemplary DAP10/DAP12 fusion polypeptide constructs**

| **Name** | **Sequence** |
|---|---|
| DAP10 (full sequence - aa1-93)-DAP12 endodomain (aa62-113) (SEQ ID NO: 60) | MIHLGHILFLLLLPVAAAQTTPGERSSLPAFYPGTSGSCSGCGS LSLPLLAGLVAADAVASLLIVGAVFLCARPRRSPAQEDGKVYI NMPGRGYFLGRLVPRGRGAAEAATRKQRITETESPYQELQGQ RSDVYSDLNTQRPYYK |
| CD8α leader-FLAG-DAP10 (extracellular and TM - aa19-69)-DAP12 endodomain (aa62-113) (SEQ ID NO: 61) | MALPVTALLLPLALLLHAARPDYKDDDDKQTTPGERSSLPAF YPGTSGSCSGCGSLSLPLLAGLVAADAVASLLIVGAVFYFLGR LVPRGRGAAEAATRKQRITETESPYQELQGQRSDVYSDLNTQ RPYYK |
| CD8α leader-FLAG-human IgG1 hinge (aa 218-229 Uniprot ref P0DOX5)-DAP10 (TM and endodomain aa 49-93)-DAP12 endodomain (aa 62-113) (SEQ ID NO: 62) | MALPVTALLLPLALLLHAARPDYKDDDDKEPKSCDKTHTCPL LAGLVAADAVASLLIVGAVFLCARPRRSPAQEDGKVYINMPG RGYFLGRLVPRGRGAAEAATRKQRITETESPYQELQGQRSDV YSDLNTQRPYYK |
| CD8α leader-truncated CD8a hinge (aa 138-182 Uniprot reference P01732)-DAP10 (TM and endodomain-aa 49-93)-DAP12 endodomain (aa 62-113) (SEQ ID NO: 63) | MALPVTALLLPLALLLHAARPDYKDDDDKTTTPAPRPPTPAP TIASQPLSLRPEACRPAAGGAVHTRGLDFACDLLAGLVAADA VASLLIVGAVFLCARPRRSPAQEDGKVYINMPGRGYFLGRLV PRGRGAAEAATRKQRITETESPYQELQGQRSDVYSDLNTQRP YYK |

Furthermore, as mentioned above, fusion polypeptides of the disclosure may be expressed as a single chimeric construct with a NKG2D polypeptide, for translation-associated or post-translational cleavage. In such constructs, following expression, the translated polypeptides are cleaved to create the separate polypeptides which then self-associate to form a CAR. In one embodiment, a fusion polypeptide of the disclosure is cleaved from a NKG2D polypeptide. Examples of such constructs are shown in Table 2:

**Table 2: Exemplary chimeric constructs**

| **Name** | **Sequence** |
|---|---|
| DAP10 (aa1-93)-DAP12 endodomain (aa62-113)-Furin cleavage-linker-P2A-NKG2D (aa1-216) (Construct 1) (SEQ ID NO: 64) (N1012) | MIHLGHILFLLLLPVAAAQTTPGERSSLPAFYPGTSGSCSGCGS LSLPLLAGLVAADAVASLLIVGAVFLCARPRRSPAQEDGKVYI NMPGRGYFLGRLVPRGRGAAEAATRKQRITETESPYQELQGQ RSDVYSDLNTQRPYYKRRKRSGSGATNFSLLKQAGDVEENPG PMGWIRGRRSRHSWEMSEFHNYNLDLKKSDFSTRWQKQRCP VVKSKCRENASPFFFCCFIAVAMGIRFIIMVAIWSAVFLNSLFN QEVQIPLTESYCGPCPKNWICYKNNCYQFFDESKNWYESQAS CMSQNASLLKVYSKEDQDLLKLVKSYHWMGLVHIPTNGSW QWEDGSILSPNLLTIIEMQKGDCALYASSFKGYIENCSTPNTYI CMQRTV |
| CD8α leader-FLAG-DAP10 (aa19-69)-DAP12 (aa62-113) - Furin cleavage-linker-T2A-DAP10 (aa1-93)-Furin cleavage-linker-P2A- NKG2D (aa1-216) (Construct 3) (SEQ ID NO: 65) | MALPVTALLLPLALLLHAARPDYKDDDDKQTTPGERSSLPAF YPGTSGSCSGCGSLSLPLLAGLVAADAVASLLIVGAVFYFLGR LVPRGRGAAEAATRKQRITETESPYQELQGQRSDVYSDLNTQ RPYYKRRKRSGSGEGRGSLLTCGDVEENPGPMIHLGHILFLLL LPVAAAQTTPGERSSLPAFYPGTSGSCSGCGSLSLPLLAGLVA ADAVASLLIVGAVFLCARPRRSPAQEDGKVYINMPGRGRRKR SGSGATNFSLLKQAGDVEENPGPMGWIRGRRSRHSWEMSEF HNYNLDLKKSDFSTRWQKQRCPVVKSKCRENASPFFFCCFIA VAMGIRFIIMVAIWSAVFLNSLFNQEVQIPLTESYCGPCPKNWI CYKNNCYQFFDESKNWYESQASCMSQNASLLKVYSKEDQDL LKLVKSYHWMGLVHIPTNGSWQWEDGSILSPNLLTIIEMQKG DCALYASSFKGYIENCSTPNTYICMQRTV |
| DAP10 (aa1-93)-DAP12 endodomain | MIHLGHILFLLLLPVAAAQTTPGERSSLPAFYPGTSGSCSGCGS LSLPLLAGLVAADAVASLLIVGAVFLCARPRRSPAQEDGKVYI NMPGRGYFLGRLVPRGRGAAEAATRKQRITETESPYQELQGQ |
| (aa62-113)-Furin cleavage-linker-P2A-NKG2D (aa1-216)-Furin cleavage-linker-T2A-DAP10 (aa1-71)-4-1BB endodomain (aa214-255) (Construct 8) (SEQ ID NO: 66) | RSDVYSDLNTQRPYYKRRKRSGSGATNFSLLKQAGDVEENPG PMGWIRGRRSRHSWEMSEFHNYNLDLKKSDFSTRWQKQRCP VVKSKCRENASPFFFCCFIAVAMGIRFIIMVAIWSAVFLNSLFN QEVQIPLTESYCGPCPKNWICYKNNCYQFFDESKNWYESQAS CMSQNASLLKVYSKEDQDLLKLVKSYHWMGLVHIPTNGSW QWEDGSILSPNLLTIIEMQKGDCALYASSFKGYIENCSTPNTYI CMQRTVRRKRSGSGEGRGSLLTCGDVEENPGPMIHLGHILFLL LLPVAAAQTTPGERSSLPAFYPGTSGSCSGCGSLSLPLLAGLV AADAVASLLIVGAVFLCKRGRKKLLYIFKQPFMRPVQTTQEE DGCSCRFPEEEEGGCEL |
| DAP10 (aa1-93)-DAP12 endodomain (aa62-113)-Furin cleavage-linker-P2A-NKG2D (aa1-216)-Furin cleavage-linker-T2A-CD8α leader (aa1-21)-FLAG-DAP10 (aa19-71)-4-1BB endodomain (aa214-255) (Construct 9) (SEQ ID NO: 67) | MIHLGHILFLLLLPVAAAQTTPGERSSLPAFYPGTSGSCSGCGS LSLPLLAGLVAADAVASLLIVGAVFLCARPRRSPAQEDGKVYI NMPGRGYFLGRLVPRGRGAAEAATRKQRITETESPYQELQGQ RSDVYSDLNTQRPYYKRRKRSGSGATNFSLLKQAGDVEENPG PMGWIRGRRSRHSWEMSEFHNYNLDLKKSDFSTRWQKQRCP VVKSKCRENASPFFFCCFIAVAMGIRFIIMVAIWSAVFLNSLFN QEVQIPLTESYCGPCPKNWICYKNNCYQFFDESKNWYESQAS CMSQNASLLKVYSKEDQDLLKLVKSYHWMGLVHIPTNGSW QWEDGSILSPNLLTIIEMQKGDCALYASSFKGYIENCSTPNTYI CMQRTVRRKRSGSGEGRGSLLTCGDVEENPGPMALPVTALLL PLALLLHAARPDYKDDDDKQTTPGERSSLPAFYPGTSGSCSGC GSLSLPLLAGLVAADAVASLLIVGAVFLCKRGRKKLLYIFKQP FMRPVQTTQEEDGCSCRFPEEEEGGCEL |
| DAP10 (aa1-93)-DAP12 endodomain (aa62-113)-Furin cleavage-linker-P2A-NKG2D (aa1-216)-Furin cleavage-linker-T2A-DAP10 (aa1-71)-CD27 endodomain (aa213-260) (Construct 10) (SEQ ID NO: 68) | MIHLGHILFLLLLPVAAAQTTPGERSSLPAFYPGTSGSCSGCGS LSLPLLAGLVAADAVASLLIVGAVFLCARPRRSPAQEDGKVYI NMPGRGYFLGRLVPRGRGAAEAATRKQRITETESPYQELQGQ RSDVYSDLNTQRPYYKRRKRSGSGATNFSLLKQAGDVEENPG PMGWIRGRRSRHSWEMSEFHNYNLDLKKSDFSTRWQKQRCP VVKSKCRENASPFFFCCFIAVAMGIRFIIMVAIWSAVFLNSLFN QEVQIPLTESYCGPCPKNWICYKNNCYQFFDESKNWYESQAS CMSQNASLLKVYSKEDQDLLKLVKSYHWMGLVHIPTNGSW QWEDGSILSPNLLTIIEMQKGDCALYASSFKGYIENCSTPNTYI CMQRTVRRKRSGSGEGRGSLLTCGDVEENPGPMIHLGHILFLL LLPVAAAQTTPGERSSLPAFYPGTSGSCSGCGSLSLPLLAGLV AADAVASLLIVGAVFLCQRRKYRSNKGESPVEPAEPCHYSCP REEEGSTIPIQEDYRKPEPACSP |
| DAP10 (aa1-93)-DAP12 endodomain (aa62-113)-Furin cleavage-linker-P2A-NKG2D (aa1-216)-Furin cleavage-linker-T2A-CD8α leader (aa1-21)-FLAG-DAP10 (aa19-71)-CD27 endodomain (aa213-260) (Construct 11) (SEQ ID NO: 69) | MIHLGHILFLLLLPVAAAQTTPGERSSLPAFYPGTSGSCSGCGS LSLPLLAGLVAADAVASLLIVGAVFLCARPRRSPAQEDGKVYI NMPGRGYFLGRLVPRGRGAAEAATRKQRITETESPYQELQGQ RSDVYSDLNTQRPYYKRRKRSGSGATNFSLLKQAGDVEENPG PMGWIRGRRSRHSWEMSEFHNYNLDLKKSDFSTRWQKQRCP VVKSKCRENASPFFFCCFIAVAMGIRFIIMVAIWSAVFLNSLFN QEVQIPLTESYCGPCPKNWICYKNNCYQFFDESKNWYESQAS CMSQNASLLKVYSKEDQDLLKLVKSYHWMGLVHIPTNGSW QWEDGSILSPNLLTIIEMQKGDCALYASSFKGYIENCSTPNTYI CMQRTVRRKRSGSGEGRGSLLTCGDVEENPGPMALPVTALLL PLALLLHAARPDYKDDDDKQTTPGERSSLPAFYPGTSGSCSGC GSLSLPLLAGLVAADAVASLLIVGAVFLCQRRKYRSNKGESP VEPAEPCHYSCPREEEGSTIPIQEDYRKPEPACSP |

### 4.2.7. Nucleic acid molecules encoding NKG2D, CXCR2, DAP10, DAP12, and DAP10/DAP12 fusion polypeptides of the disclosure

Another aspect of the disclosure pertains to nucleic acid molecules that encode one or more polypeptides of the disclosure. This may be as DNA or RNA. Unless specifically limited herein, the term encompasses nucleic acids containing known analogues of natural nucleotides that have similar properties as the reference nucleic acid and are metabolized in a manner similar to naturally occurring nucleotides. Examples of such analogs include, without limitation, phosphorothioates, phosphoramidates, methyl phosphonates, chiral-methyl phosphorates, 2-O-methyl ribonucleotides, peptide-nucleic acids (PNAs). Unless otherwise indicated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be achieved by generating sequences in which the third position of one or more selected (or all) codons is substituted with mixed-base and/or deoxyinosine residues (Batzer et al., 1991, Nucleic Acid Res. 19:5081; Ohtsuka et al., 1985, J. Biol. Chem. 260:2605-2608; and Rossolini et al., 1994, Mol. Cell. Probes 8:91-98).

Thus, the disclosure also provides a nucleic acid comprising a nucleotide sequence encoding the polypeptide sequence of any one or more of SEQ ID NOs: 60-69, 87, 90, and 102.

The disclosure further provides a nucleic acid comprising a nucleotide sequence having at least about 80%, at least about 85%, at least about 90%, at least about 95%, at least about 97% or at least about 99% sequence identity with a nucleic acid encoding any of SEQ ID NOS: 60-69, 87, 90, and 102. Sequence identity is typically measured along the full length of the reference sequence.

The disclosure further provides a nucleic acid comprising a nucleotide sequence having at least about 80%, at least about 85%, at least about 90%, at least about 95%, at least about 97% or at least about 99% sequence identity with any one of SEQ ID NOs: 70-79, 88, 91, and 103.

The disclosure also provides a nucleic acid comprising the nucleotide sequence of any one of SEQ ID NOs: 70-79, 88, 91, and 103. The disclosure also provides a nucleic acid consisting of the nucleotide sequence of any one of SEQ ID NOs: 70-79, 91, and 103.

The polynucleotide sequences can be produced by de novo solid-phase DNA synthesis or by PCR mutagenesis of an existing sequence (e.g., sequences as described in the Examples below). Direct chemical synthesis of nucleic acids can be accomplished by methods known in the art, such as the phosphotriester method of Narang et al., 1979, Meth. Enzymol. 68:90; the phosphodiester method of Brown et al., 1979, Meth. Enzymol. 68:109; the diethylphosphoramidite method of Beaucage et al., 1981, Tetra. Lett., 22:1859; and the solid support method of U.S. Pat. No. 4,458,066. Introducing mutations to a polynucleotide sequence by PCR can be performed as described in, e.g., PCR Technology: Principles and Applications for DNA Amplification, H. A. Erlich (Ed.), Freeman Press, NY, N.Y., 1992; PCR Protocols: A Guide to Methods and Applications, Innis et al. (Ed.), Academic Press, San Diego, Calif, 1990; Mattila et al., 1991, Nucleic Acids Res. 19:967; and Eckert et al., 1991, PCR Methods and Applications 1:17.

### 4.2.8. Vectors

The present disclosure also provides vectors comprising one or more nucleic acid molecules of the disclosure.

For expression in host cells, the nucleic acid encoding one or more polypeptides of the disclosure can be present in a suitable vector and after introduction into a suitable host, the sequence can be expressed to produce the encoded polypeptide(s) according to standard cloning and expression techniques, which are known in the art (e.g., as described in Sambrook, J., Fritsh, E. F., and Maniatis, T. Molecular Cloning: A Laboratory Manual 2nd, ed., Cold Spring Harbor Laboratory, Cold Spring Harbor Laboratory Press, Cold Spring Harbor, N.Y., 1989). The disclosure also relates to such vectors comprising a nucleic acid sequence according to the disclosure.

Various expression vectors can be employed to express the polynucleotides encoding the polypeptide(s) of the disclosure. Both viral-based and non-viral expression vectors can be used to produce the polypeptide(s) in a host cell, such as a mammalian host cell. Non-viral vectors and systems include plasmids, episomal vectors, typically with an expression cassette for expressing a protein or RNA, and human artificial chromosomes (see, e.g., Harrington et al., 1997, Nat Genet. 15:345). For example, non-viral vectors useful for expression of the polynucleotides and polypeptides of the disclosure in mammalian (e.g., human) cells include pThioHis A, B and C, pcDNA3.1/His, pEBVHis A, B and C, (Invitrogen, San Diego, Calif.), MPS V vectors, and numerous other vectors known in the art for expressing other proteins. Useful viral vectors include vectors based on retroviruses, adenoviruses, adeno-associated viruses, herpes viruses, vectors based on SV40, papilloma virus, HBP Epstein Barr virus, vaccinia virus vectors and Semliki Forest virus (SFV). See, Brent et al., supra; Smith, 1995, Annu. Rev. Microbiol. 49:807; and Rosenfeld et al., 1992, Cell 68: 143. In particular, retroviral, lentiviral, adenoviral or adeno-associated viral vectors are commonly used for expression in T-cells. Examples of such vectors include the SFG retroviral expression vector (see Riviere et al., 1995, Proc. Natl. Acad. Sci. (USA) 92:6733-6737). In one embodiment a lentiviral vector is used, these include self-inactivating lentiviral vectors (so-called SIN vectors).

The choice of expression vector depends on the intended host cells in which the vector is to be expressed. Expression vectors for mammalian host cells can include expression control sequences, such as an origin of replication, a promoter, and an enhancer (see, e.g., Queen, et al., 1986, Immunol. Rev. 89:49-68), and necessary processing information sites, such as ribosome binding sites, RNA splice sites, polyadenylation sites, and transcriptional terminator sequences. These expression vectors usually contain promoters derived from mammalian genes or from mammalian viruses. Suitable promoters may be constitutive, cell type-specific, stage-specific, and/or modulatable or regulatable. Useful promoters include, but are not limited to, the metallothionein promoter, the constitutive adenovirus major late promoter, the dexamethasone-inducible MMTV promoter, the SV40 promoter, the MRP polIII promoter, the constitutive MPS V promoter, the tetracycline-inducible CMV promoter (such as the human immediate-early CMV promoter), the constitutive CMV promoter, the EF1 alpha promoter, the phosphoglycerate kinase (PGK) promoter and promoter-enhancer combinations known in the art.

Cultures of transformed organisms can be expanded under non-inducing conditions without biasing the population for coding sequences whose expression products are better tolerated by the host cells. In addition to promoters, other regulatory elements may also be required or desired for efficient expression of the polypeptide(s) of the disclosure. These elements typically include an ATG initiation codon and adjacent ribosome binding site or other sequences. In addition, the efficiency of expression may be enhanced by the inclusion of enhancers appropriate to the cell system in use (see, e.g., Scharf et al., 1994, Results Probl. Cell Differ. 20:125; and Bittner et al., 1987, Meth. Enzymol., 153:516). For example, the SV40 enhancer or CMV enhancer may be used to increase expression in mammalian host cells.

The disclosure provides a cloning or expression vector comprising a nucleic acid comprising a nucleotide sequence having at least about 80%, at least about 85%, at least about 90%, at least about 95%, at least about 97% or at least about 99% sequence identity with a nucleic acid encoding any of SEQ ID NOS: 60-69, 87, 90, and 102. Furthermore, the disclosure provides a cloning or expression vector comprising a nucleic acid encoding one or more of SEQ ID NOs: 60-69, 87, 90, and 102. The disclosure provides a cloning or expression vector comprising the nucleic acid sequence of any one of SEQ ID NOs: 70-79, 88, 91, and 103.

### 4.2.9. Host cells

Host cells comprising a polypeptide of the disclosure, nucleic acid of the disclosure, vector of the disclosure, or combinations of either or both thereof are provided. Such cells are generally utilized for the expression of the polypeptide(s) according to the disclosure.

The nucleic acid or vector may be transfected into a host cell by standard techniques.

The various forms of the term "transfection" are intended to encompass a wide variety of techniques commonly used for the introduction of exogenous DNA into a prokaryotic or eukaryotic host cell, e.g., electroporation, calcium-phosphate precipitation, DEAE-dextran transfection and the like.

Alternatively, the nucleic acid or vector may be delivered into the host cell by transduction. For example, a viral vector, as disclosed above, may be used for delivery of the nucleic acid or vector.

It is possible to express the polypeptide(s) of the disclosure in either prokaryotic or eukaryotic host cells. Representative host cells include many E. coli strains, mammalian cell lines, such as CHO, CHO-K1, and HEK293; insect cells, such as Sf9 cells; and yeast cells, such as S. cerevisiae and P. pastoris. In one embodiment the host cell is an immunoresponsive cell, such as a NK cell (either a primary NK cell, or a NK cell line) or a T cell (either a primary T-cell, or a T-cell line). Other types of host cells include macrophages, induced pluripotent stem cells (iPSCs), neutrophils and invariant NKT (iNKT) cells. The T-cell may be a CD4+ or CD8+ T-cell. In one embodiment the host cell is a human cell. In one embodiment, the host cell is a human T-cell. In another embodiment, the host cell is a primary human T-cell. Cell lines which may be used include the NK cell line NK-92.

Mammalian host cells for expressing the polypeptide(s) of the disclosure include Chinese Hamster Ovary (CHO cells) (including dhfr- CHO cells, described Urlaub and Chasin, 1980, Proc. Natl. Acad. Sci. USA 77:4216-4220 used with a DH FR selectable marker, e.g., as described in R.J. Kaufman and P.A. Sharp, 1982, Mol. Biol. 159:601-621), NSO myeloma cells, COS cells and SP2 cells. In one embodiment the host cells are CHO K1PD cells. In another embodiment the host cells are NSO1 cells. In particular, for use with NSO myeloma cells, another expression system is the GS gene expression system shown in WO 87/04462, WO 89/01036 and EP 338,841. When recombinant expression vectors encoding polypeptides are introduced into mammalian host cells, the polypeptides may be produced by culturing the host cells for a period of time sufficient to allow for expression of the polypeptides in the host cells or secretion of the polypeptides into the culture medium in which the host cells are grown. Polypeptides can be recovered from the culture medium using standard protein purification methods.

### 4.3. Methods of producing immunoresponsive cells of the disclosure

The present disclosure also provides methods of producing immunoresponsive cells comprising polypeptides of the disclosure. Such a method may comprise transducing a cell with a nucleic acid or vector encoding polypeptide(s) of the disclosure. The method may further comprise culturing the cell, such that the polypeptides are expressed and associates with a NKG2D polypeptide to form a CAR.

In one embodiment the present disclosure provides a method for preparing an immunoresponsive cell comprising the steps of (i) transducing a nucleic acid molecule or vector of the disclosure into the immunoresponsive cell, and (ii) culturing the immunoresponsive cell such that the transduced cell expresses a NKG2D polypeptide, a DAP10/DAP12 fusion polypeptide, and a CXCR2 polypeptide, wherein the NKG2D polypeptide and the DAP10/DAP12 fusion polypeptide associate in the cell membrane.

In a further embodiment, the present disclosure provides a method comprising, (i) obtaining T-cells and/or NK cells from a patient, (ii) transducing a nucleic acid or vector of the disclosure into the T-cells and/or NK cells, and (iii) culturing the T-cells and/or NK cells such that the transduced cell expresses a NKG2D polypeptide, a DAP10/DAP12 fusion polypeptide, and a CXCR2 polypeptide, wherein the NKG2D polypeptide and the DAP10/DAP12 fusion polypeptide associate in the cell membrane.

Various methods for the culture of immunoresponsive cells are well known in the art. See, for example, Parente-Pereira AC et al. 2014, J. Biol. Methods 1(2):e7, Ghassemi S et al. 2018, Cancer Immunol Res 6(9):1100-1109, and Denman CJ et al. 2012, PLoS One 7(1): e30264.

### 4.4. Pharmaceutical compositions

The disclosure also provides pharmaceutical compositions comprising polypeptides, nucleic acid, vector or host cell as described herein. Such pharmaceutical compositions can comprise a pharmaceutically or physiologically acceptable diluent and/or carrier. The carrier is generally selected to be suitable for the intended mode of administration and can include agents for modifying, maintaining, or preserving, for example, the pH, osmolarity, viscosity, clarity, colour, isotonicity, odour, sterility, stability, rate of dissolution or release, adsorption, or penetration of the composition. Typically, these carriers include aqueous or alcoholic/aqueous solutions, emulsions or suspensions, including saline and/or buffered media.

Suitable agents for inclusion in the pharmaceutical compositions include, but are not limited to, amino acids (such as glycine, glutamine, asparagine, arginine, or lysine), antimicrobials, antioxidants (such as ascorbic acid, sodium sulphite, or sodium hydrogensulphite), buffers (such as borate, bicarbonate, Tris-HCl, citrates, phosphates, or other organic acids), bulking agents (such as mannitol or glycine), chelating agents (such as ethylenediamine tetraacetic acid (EDTA)), complexing agents (such as caffeine, polyvinylpyrrolidone, beta-cyclodextrin, or hydroxypropyl-beta-cyclodextrin), fillers, monosaccharides, disaccharides, and other carbohydrates (such as glucose, mannose, or dextrins), proteins (such as free serum albumin, gelatin, or immunoglobulins), colouring, flavouring and diluting agents, emulsifying agents, hydrophilic polymers (such as polyvinylpyrrolidone), low molecular weight polypeptides, salt-forming counterions (such as sodium), preservatives (such as benzalkonium chloride, benzoic acid, salicylic acid, thimerosal, phenethyl alcohol, methylparaben, propylparaben, chlorhexidine, sorbic acid, or hydrogen peroxide), solvents (such as glycerin, propylene glycol, or polyethylene glycol), sugar alcohols (such as mannitol or sorbitol), suspending agents, surfactants or wetting agents (such as pluronics; PEG; sorbitan esters; polysorbates such as Polysorbate 20 or Polysorbate 80; Triton; tromethamine; lecithin; cholesterol or tyloxapal), stability enhancing agents (such as sucrose or sorbitol), tonicity enhancing agents (such as alkali metal halides, such as sodium or potassium chloride, or mannitol sorbitol), delivery vehicles, diluents, excipients and/or pharmaceutical adjuvants.

Parenteral vehicles include sodium chloride solution, Ringer's dextrose, dextrose and sodium chloride and lactated Ringer's. Suitable physiologically-acceptable thickeners such as carboxymethylcellulose, polyvinylpyrrolidone, gelatin and alginates may be included. Intravenous vehicles include fluid and nutrient replenishers and electrolyte replenishers, such as those based on Ringer's dextrose. In some cases one might include agents to adjust tonicity of the composition, for example, sugars, polyalcohols such as mannitol, sorbitol, or sodium chloride in a pharmaceutical composition. For example, in many cases it is desirable that the composition is substantially isotonic. Preservatives and other additives, such as antimicrobials, antioxidants, chelating agents and inert gases, may also be present. The precise formulation will depend on the route of administration. Additional relevant principle, methods and components for pharmaceutical formulations are well known (see, e.g., Allen, Loyd V. Ed, (2012) Remington's Pharmaceutical Sciences, 22nd Edition).

A pharmaceutical composition of the present disclosure can be administered by one or more routes of administration using one or more of a variety of methods known in the art. As will be appreciated by the skilled artisan, the route and/or mode of administration will vary depending upon the desired results. Routes of administration for pharmaceutical compositions of the disclosure include intravenous, intramuscular, intradermal, intraperitoneal, subcutaneous, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intra-sternal injection and infusion. In one embodiment, the pharmaceutical composition is administered intratumorally. When parenteral administration is contemplated, the pharmaceutical compositions are usually in the form of a sterile, pyrogen-free, parenterally acceptable composition. A particularly suitable vehicle for parenteral injection is a sterile, isotonic solution, properly preserved. The pharmaceutical composition can be in the form of a lyophilizate, such as a lyophilized cake.

Alternatively, the pharmaceutical composition described herein can be administered by a non-parenteral route, such as a topical, epidermal or mucosal route of administration, for example, intranasally, orally, vaginally, rectally, sublingually or topically.

In certain embodiments, the pharmaceutical composition is for subcutaneous administration. Suitable formulation components and methods for subcutaneous administration of polypeptide therapeutics (e.g., antibodies, fusion polypeptides and the like) are known in the art, see, for example, US2011/0044977, US8465739 and US8476239. Typically, the pharmaceutical compositions for subcutaneous administration contain suitable stabilizers (e.g, amino acids, such as methionine, and or saccharides such as sucrose), buffering agents and tonicifying agents.

Typically, in cell therapy, the composition comprising the host cell is administered to the patient by intravenous infusion.

Administration of the pharmaceutically useful composition of the present disclosure is preferably in a "therapeutically effective amount" or "prophylactically effective amount" (as the case can be, although prophylaxis can be considered therapy), this being sufficient to show benefit to the individual. The actual amount administered, and rate and time-course of administration, will depend on the nature and severity of protein aggregation disease being treated. Prescription of treatment, e.g. decisions on dosage etc, is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Remington's Pharmaceutical Sciences, 16th edition, Osol, A. (ed), 1980.

A composition can be administered alone or in combination with other treatments, either simultaneously or sequentially dependent upon the condition to be treated.

### 4.5. Methods of Treatment

In some embodiments, the polypeptides, nucleic acids, vectors, cells and/or pharmaceutical compositions of the disclosure are used for treating a disease or disorder, prophylaxis, and/or for delaying the onset of disease symptoms. The methods include administering a therapeutically effective amount of the polypeptides, nucleic acids, vectors, cells, and/or pharmaceutical compositions of the disclosure.

The disclosure provides a polypeptide, nucleic acid, vector, host cell or pharmaceutical composition of the disclosure for use in therapy or as a medicament. The disclosure further provides a polypeptide, nucleic acid, vector, host cell or pharmaceutical composition of the disclosure for use in the treatment or prophylaxis of a pathological disorder. The disclosure provides a polypeptide, nucleic acid, vector, host cell or pharmaceutical composition of the disclosure for use in the treatment or prophylaxis of cancer. Also provided is the immunoresponsive cell of the disclosure for use in the treatment or prophylaxis of cancer. The disclosure also provides the use of a polypeptide, nucleic acid, vector, host cell or pharmaceutical composition of the disclosure in the manufacture of a medicament for the treatment of a pathological disorder. Also provided is use of a polypeptide, nucleic acid, vector, host cell or pharmaceutical composition of the disclosure in the treatment or prophylaxis of a pathological disorder. The disclosure further provides a method of treating a patient suffering from a pathological disorder comprising administering a therapeutically effective amount of a polypeptide, nucleic acid, vector, host cell or pharmaceutical composition of the disclosure to said patient.

As used herein, the terms "disease" and "pathological disorder" include cancer, including but not limited to, a solid tumor cancer, a soft tissue tumor, a metastatic lesion and a hematological cancer. For example, the cancer can be liver cancer, lung cancer, breast cancer, prostate cancer, lymphoid cancer, colon cancer, renal cancer, bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemias including acute myeloid leukemia, chronic myeloid leukemia, acute lymphoblastic leukemia, chronic lymphocytic leukemia, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, myelodysplastic syndrome (MDS), chronic myelogenous leukaemia-chronic phase (CMLCP), diffuse large B-cell lymphoma (DLBCL), cutaneous T-cell lymphoma (CTCL), peripheral T-cell lymphoma (PTCL), hepatocellular carcinoma (HCC), gastrointestinal stromal tumors (GIST), non-small cell lung carcinoma (NSCLC), squamous cell carcinoma of the head and neck (SCCHN), environmentally induced cancers including those induced by asbestos, and combinations of said cancers. In particular, the cancer can be breast cancer, such as an estrogen receptor-positive (ER pos) breast cancer and/or a metastatic form of breast cancer.

In one embodiment the cancer is a solid tumor cancer. In one embodiment, treatment of the pathological disorder involves targeting of non-tumor cells, such as tumor-associated stromal cells. Example types of such tumor-associated stromal cells include pancreatic stromal cells. Other types of non-tumor cells that may be targeted include macrophages, regulatory T-cells and myeloid-derived suppressor cells.

In one embodiment, the patient has been pre-treated with a chemotherapeutic agent.

In one embodiment, the administration of host cells to the patient results in a decrease in tumor size of 10%, 20%, 30%, 40%, 50%, 60%, 70%, 80%, 90%, or even 100%, when compared to an untreated tumor.

The amount of host cells administered to the patent should take into account the route of administration, the cancer being treated, the weight of the patient and/or the age of the patient. In general, about 1 × 10⁶ to about 1 × 10¹¹ cells are administered to the patient. In one embodiment, about 1 × 10⁷ to about 1 × 10¹⁰ cells, or about 1 × 10⁸ to about 1 × 10⁹ cells are administered to the patient.

### 4.6. Kits

In another aspect, components or embodiments described herein for the system are provided in a kit. For example, any of the plasmids, as well as the mammalian cells, related buffers, media, triggering agents, or other components related to cell culture and virion production can be provided, with optional components frozen and packaged as a kit, alone or along with separate containers of any of the other agents and optional instructions for use. In some embodiments, the kit may comprise culture vessels, vials, tubes, or the like.

### 4.7. Examples

Below are examples of specific embodiments for carrying out the present invention. The examples are offered for illustrative purposes only and are not intended to limit the scope of the present invention in any way. Efforts have been made to ensure accuracy with respect to numbers used (e.g., amounts, temperatures, etc.), but some experimental error and deviation should, of course, be allowed for.

The practice of the present invention will employ, unless otherwise indicated, conventional methods of protein chemistry, biochemistry, recombinant DNA techniques and pharmacology, within the skill of the art. Such techniques are explained fully in the literature.

### 4.7.1. Methods

### T cell Isolation and Retroviral Transduction

Peripheral blood mononuclear cells (PBMCs) were isolated from blood samples from healthy volunteers through density-mediated centrifugation. T-cells were activated with phytohaemagglutinin for 72 hours, with 100IU/mL IL-2 added for the final 24 hours. 1×10⁶ activated PBMC were plated onto a RetroNectin-coated plate that had been pre-treated with 3mL retroviral supernatant. Each well was subsequently treated with 3mL fresh viral supernatant and 100IU/mL IL-2. Retroviral transduction was performed with viral particles produced by stable gibbon ape leukemia virus (GALV)-pseudotyped 293TVec stable packaging cells. Thereafter, T-cells were fed with 100IU/mL in RPMI1640 media + 5% normal human AB serum, with fresh media and IL-2 (100IU/mL) provided thrice weekly.

### Flow Cytometry

T-cell transduction and transfection of 293T cells was assessed by flow cytometry, making comparison, where indicated, with an appropriate isotype control. To assess the expression of the NKG2D-based constructs, cells were stained with mouse anti-human CD4-FITC, mouse anti-human NKG2D-PE and mouse anti-human CD8-APC and compensated appropriately. Due to high levels of endogenous NKG2D expression in CD8⁺ T-cells, transduction efficiency was compared against NKG2D expression in untransduced CD4⁺ T-cells. Expression of the panErbB-specific T4 and TMY CARs was assessed using biotinylated goat anti-human EGF, followed by PE-conjugated streptavidin. Transduction efficiency was calculated by comparison with N1012⁺ T-cells stained using the same reagents. Prior to use, the transduction efficiency was normalized between constructs by spiking in the requisite proportion of untransduced T-cells. This ensured that all conditions were identical for the total number of CAR⁺ T-cells and overall T-cell concentration.

To perform intracellular staining, transfected 293T cells were fixed in 4% formaldehyde for 10 minutes at room temperature before washing twice in permeabilization solution (PBS + 0.5% BSA + 0.1% saponin). The cells were subsequently stained with 500ng PE-conjugated anti-human NKG2D, or an appropriate isotype control, in the presence of 100uL permeabilization solution. Cells were further washed twice in permeabilization solution prior to analysis by flow cytometry.

To assess T-cell differentiation after multiple rounds of stimulation, T-cells were removed from tumor cell co-cultures and stained with FITC-conjugated anti-human CD45RO, allophycocyanin (APC)-conjugated anti-human CD62L, PE-conjugated anti-human CCR7, FITC-conjugated anti-human CD4, APCCy7-conjugated anti-human CD8α and phycoerythrin cyanine7 (PECy7)-conjugated anti-human CD27 antibodies. After washing in 2 mL ice-cold PBS the cells were re-suspended in 0.5 mL ice-cold PBS and assessed by flow cytometry. Staining efficiency was assessed using T-cells stained with the appropriate isotype and fluorescence minus one (FMO) controls.

### Dose response assays

1×10⁴ tumor cells were plated per well (100µL) of a 96-well plate and incubated at 37°C and 5% CO₂ overnight. Twenty-four hours later T-cells were added at log² CAR T-cell: tumor cell ratios ranging from 1: 1 to 1:64. After 72 hours, the T-cells were removed and 100µL MTT solution (500µg/mL) added, before the plates were incubated at 37°C and 5% CO₂ for approximately 1 hour. Following removal of the MTT solution, the resulting formazan crystals were solubilized in DMSO (100µL/well) and the absorbance measured at 560nm. Tumor cell viability was calculated as follows: (Absorbance of monolayer with T-cells/absorbance of monolayer without T-cells)* 100.

### Re-stimulation assays

1×10⁵ tumor cells were plated in triplicate wells of a 24-well plate and incubated for 24 hours at 37°C and 5% CO₂. Twenty-four hours later, 1mL containing 1×10⁵ CAR⁺ T-cells were added per well. After 72 hours, the T-cells were gently removed and the well was washed with 1mL PBS. Following removal of the PBS, 1mL of MTT (at a final concentration of 500/µg/mL) was added to each well and the plate incubated at 37°C and 5% CO₂ for approximately 1 hour. Absorbance was measured in the appropriate wells at 560nm and tumor cell viability calculated as detailed in the `dose response' section. A re-stimulation was considered successful if the tumor cell viability was measured as less than 50%.

The T-cells that had been removed from the plate were centrifuged at 400xg for 5 minutes and the supernatant removed. The pellet was re-suspended in 3.2mL R5 media and 1mL added to each well of fresh tumor monolayer (1×10⁵ tumor cells per well of a 24-well plate) in triplicate. Total T-cell number was assessed by trypan blue exclusion of a small aliquot of the remaining 200µL.

### Binding avidity

Binding avidity of CAR T-cells for target cells was assessed by measuring the percentage of T-cells bound to target cells at increasing levels of acoustic force. CD19-engineered LO68 or SKOV-3 tumor cells were seeded in a z-Movi microfluidic chip (Lumicks, Amsterdam, Netherlands) coated with poly-L-lysine and cultured for 16 hours. The next day, flow sorted CAR-T cells were serially flowed in the chips and incubated with the target cells for 5 minutes prior to initializing a 3-minute linear force ramp. During the force ramp, the z-Movi device (Lumicks) captured a time series of images using a bright field microscope integrated into the platform. Detached cells were levitated towards the acoustic nodes, allowing the tracking of cells based on their XY positions. Changes in the Z-position resulted in a change in the diffraction pattern, which permitted distinction between cells adhered to the substrate and cells suspended to the acoustic nodes. This information was used to correlate cell detachment events with a specific rupture force. Cell detachment was acquired using (z-Movi Tracking_v1.6.0) and post experiment image analysis performed using Cell Tracking offline analysis_v2.1.

### ELISA

Secretion of IFN-γ and IL-2 by T-cells were assessed in supernatant aliquots removed 24 hours after the initiation of co-culture using Duo-set and Ready-Steady-Go ELISA kits respectively.

### Tumor spheroid generation

To generate tumor spheroids, 1×10³ tumor cells and 1×10³ PS1 stellate cells were added per well of an ultra-low affinity 96-well plate and incubated for 72 hours at 37°C and 5% CO2. Spheroid generation was confirmed by standard light microscope visualization.

### In vivo studies

### Bioluminescence imaging

1×10⁵ firefly luciferase (ffLUC)-tagged BxPC3 cells were injected into the intraperitoneal cavity of NSG mice. Twelve days after tumor inoculation, mice (n = 5 per group) were treated intraperitoneally with either PBS, 4×10⁶ (N1012⁺ (N1012 (lo)), T4⁺ or TMY⁺ CAR T-cells) or 1×10⁷ (N1012 (hi) or NKG2D) CAR⁺ T-cells. Alternatively, NSG mice were inoculated i.p. with 1×10⁶ ffLUC-tagged H226 malignant mesothelioma cells. Eight days after tumor inoculation, mice were treated with either PBS, or 4×10⁶ N1012⁺ T-cells. As a control, one group of mice were treated with 4×10⁶ T-cells expressing NKG2D alone.

In other studies, NSG mice were inoculated i.p. with 1×10⁵ ffLuc-expressing Ovsaho or 5×10⁵ ffLuc-expressing SKOV-3 cells. Mice inoculated with Ovasho cells were treated with either PBS or 5×10⁶ CAR T-cells (N1012, N1012_CXCR2, CYAD-01 replica, or untransduced) via intraperitoneal injection (6 days after tumor inoculation) or intravenous injection (7 days after inoculation). Mice inoculated with SKOV-3 cells were treated intravenously with either PBS or 1×10⁷ CAR T-cells (N1012, N1012_CXCR2, or CYAD-01_10) 14 days after tumor inoculation.

In order to evaluate T-cell trafficking, T-cells were double transduced to express a CAR construct (N1012 or N1012_CXCR2) and a reporter construct, either renilla luciferase (rLuc) alone or a dual reporter which encodes both rLuc and GFP. Mice inoculated with Ovasho cells were treated with 5×10⁶ CAR T-cells (N1012_Renilla Luciferase or N1012_CXCR2_Renilla Luciferase) by intravenous injection 7 days after tumor inoculation. Mice inoculated with SKOV-3 cells were treated with 1×10⁷ CAR T-cells (N1012_CXCR2_rLuc or N1012_rLuc) by intravenous injection 14 days after tumor inoculation. Mice inoculated with Kuramochi cells were treated with 2×10⁶ CAR T-cells cells (N1012_CXCR2_rLuc or N1012_rLuc) by intravenous injection 18 days after tumor inoculation. Coelenterazine, a substrate for renilla luciferase, was administered intravenously to the mice in order to monitor T-cell tracking.

Tumor growth was monitored by BLI, with all data presented as total flux (photons/second) or average total flux (photons/second) per treatment. Mice were monitored closely and weighed three times per week for signs of ill health.

### Tumor volume

1×10⁵ CFPac-1 or BxPC3 cells were injected subcutaneously in 50 µl of Matrigel (1:1 PBS) into the left flank of NSG mice. Twenty-nine days after inoculation with CFPac-1 cells or fourteen days after inoculation with BxPC3 cells, mice were treated intravenously with either PBS or 1×10⁷ CAR T-cells (N1012_CXCR2, N1012, NKG2D, CYAD-01 replica, untransduced (UT)).

Tumor growth was measured weekly by caliper measurements and presented as tumor volume (mm³).

### Immunohistochemistry

1×10⁵ CFPac-1 cells were injected subcutaneously in 50 µl of Matrigel (1:1 PBS) into the left flank of NSG mice. Twenty-nine days after inoculation, mice were treated intravenously with either PBS or 1×10⁷ CAR T-cells (N1012_CXCR2, N1012, NKG2D, CYAD-01 replica or untransduced (UT) T-cells). Three days following T-cell injections, three mice from each of the N1012, N1012_CXCR2, and NKG2D T-cell treatment groups were sacrificed and tumors were harvested for immunohistochemistry (IHC) analysis. Briefly, tissues were fixed in formalin, embedded in paraffin, sectioned, mounted on slides, and stained with anti-human CD3 antibody (Abcam) using standard methods.

### 4.7.2. Example 1: Expression of NKG2D and DAP10/12 fusion protein in 293T cells

293T cells were transfected with the SFG retroviral plasmid backbone containing either the DAP10/12 fusion protein N1012 or NKG2D expression cassette. N1012 (SEQ ID NO: 64) comprises a complex comprising an exogenous human NKG2D protein and fusion exogenous DAP10/12 homodimers according to the invention. The N1012 plasmid comprises SEQ ID NO: 74, which encodes SEQ ID NO: 64. The surface expression of NKG2D was assessed 72 hours later by flow cytometry. Whereas NKG2D expression was readily detected at the surface of 293T cells transfected with the N1012 plasmid, no NKG2D expression was detected at the surface of those transfected with the plasmid encoding the control NKG2D (Figure 2, top panel). Given that NKG2D surface expression is dependent upon the expression of DAP 10, the lack of NKG2D surface expression could be explained by the absence of DAP10 co-expression within the NKG2D plasmid. To confirm the lack of surface NKG2D expression in the NKG2D-transfected 293T cells was not due to poor transfection, intracellular staining for the presence of NKG2D was undertaken. Critically, intracellular expression of NKG2D was observed in 293T cells transfected with either the N1012 or the NKG2D plasmid (Figure 2, bottom panel). This demonstrates the successful expression of NKG2D from both constructs and also confirms the requirement of DAP10 co-expression to achieve surface expression of NKG2D.

### 4.7.3. Example 2: Expression of N1012 and NKG2D in primary human T-cells

Primary human T-cells were activated with paramagnetic beads coated with anti-human CD3 and anti-human CD28 antibodies. Forty-eight hours after activation, T-cells were engineered by retroviral transduction to express N1012 or NKG2D. Surface expression of NKG2D was assessed by flow cytometry, co-staining for CD4 and CD8 expression. The percentage expression **(****Figure 3A****)** and median fluorescence intensity (MFI, **Figure 3B****)** of NKG2D was compared against untransduced T-cells. Due to endogenous expression of NKG2D in CD8⁺ T-cells, data are gated on CD4⁺ T-cells. As shown, both NKG2D and N1012 constructs are reproducibly expressed at high levels at the surface of primary human T-cells, in contrast to either UT T-cells, or T-cells expressing a control CAR **(****Figure 4****).**

### 4.7.4. Example 3: Assessment of target cell destruction and recognition by N1012 T-cells

To assess cytolytic capacity, N1012⁺ T-cells were co-cultured with eleven different human tumor cell lines, representing 5 different tumor types (mesothelioma, ovarian cancer, head and neck squamous cell carcinoma, pancreatic cancer and breast cancer), or with tumor-associated stromal cells (PS1) at varying E:T ratios. After 72 hours, the T-cells were removed and MTT assay performed to assess tumor cell viability. Whereas a minimal reduction in tumor viability was observed when target cells were co-cultured with either UT T-cells, or those expressing NKG2D, N1012⁺ T-cells demonstrated potent lysis of all target cell lines, even at low E:T ratios **(****Figures 5** **and** **7A** and 7B). Analysis of co-culture supernatants by ELISA demonstrated substantial secretion of both Interferon-γ (IFN-γ) and Interleukin-2 (IL-2) by N1012⁺ T-cells, but not by either UT T-cells or those expressing the control NKG2D construct **(****Figures 6A** **and** **6B****).** These data demonstrate the ability of N1012⁺ T-cells to recognize and lyse a broad variety of tumor types, including tumor-associated stromal cells.

### 4.7.5. Example 5: Exemplification of tumor spheroid destruction by N1012 T-cells

To assess the ability of N1012⁺ T-cells to mediate target cell lysis within a 3D system, they were co-cultured with tumor spheroids. Following spheroid generation, 6×10³ CellTracker Violet-labelled T-cells were added per well. Tumor cell and stellate cell viability was assessed after 3 and 8 days using fluorescent microscopy, by measuring GFP and RFP, respectively. Quantification of the GFP and RFP signals was undertaken using Image J software and expressed as a percentage of the fluorescence readings from spheroids grown in the absence of T-cells. Potent lysis of the spheroids was observed with N1012⁺ T-cells, but not with either NKG2D or UT T-cells **(****Figures 7I-7J****).** Furthermore, only N1012⁺ T-cells demonstrated secretion of IFN-γ **(****Figure 7K****).**

Spheroid viability and T-cell proliferation were alternatively assessed using flow cytometry. To achieve this, the spheroids were removed from the plate, either 3 or 8 days after T-cell addition and placed into a flow cytometry tube, with up to five spheroids treated with the same CAR T-cells added to the same tube. Spheroid disaggregation was achieved using Accutase solution and vigorous re-suspension through a pipette tip. The resulting single cell suspension was washed in RPMI1640 media + 5% normal human AB serum and subsequently re-suspended in PBS containing counting beads. An equal number of counting beads were acquired per tube and the resulting number of tumor cells (as assessed by GFP and RFP fluorescence) and T-cells (as assessed by CellTracker Violet fluorescence) determined. The data are shown as a percentage of the sum of GFP and RFP cells present in spheroids grown in the absence of T-cells **(****Figure 15****).**

### 4.7.6. Example 6: Exemplification of serial target recognition by N1012 T-cells

To assess the ability of N1012⁺ T-cells to undertake serial lysis of target cells (`re-stimulation'), they were co-cultured with fresh monolayer twice weekly until monolayer destruction was not observed. Whereas UT or NKG2D⁺ T-cells mediated minimal target cell destruction and displayed no evidence of proliferation, N1012⁺ T-cells mediated potent lysis through multiple rounds of re-stimulation **(****Figures 8A-8B****).** Target cell destruction was also associated with substantial proliferation and maximum fold expansion of N1012⁺ T-cells **(****Figures 8C-8D****).**

When compared to the CYAD-01 replica NKG2D CAR and control T-cells, N1012⁺ T-cells underwent significantly more rounds of re-stimulation upon BxPC3_LT-cells (Figure 14A). Furthermore, N1012⁺ T-cells also demonstrated substantially greater proliferation than CYAD-01 T-cells or controls **(****Figure 14B****).**

### 4.7.7. Example 7: Efficacy of N1012 T-cells in in vivo models of pancreatic cancer

To determine the ability of N1012⁺ T-cells to target tumor cells *in vivo,* T-cells expressing N1012, NKG2D or two different iterations of a pan-ErbB targeting CAR (T4 and TMY) were generated. Expression of the various constructs within primary human T-cells **(****Figure 9A****)** and efficacy of the T-cells cells was demonstrated *in vitro* against three tumor cell lines after a 72 hour co-culture at a 1:1 ratio **(****Figure 9B****).** To assess function *in vivo,* intraperitoneal firefly luciferase (ffLUC)-tagged BxPC3 tumor xenografts were established for 12 days in NSG mice. Tumor-bearing mice were treated intraperitoneally with either PBS, 4×10⁶ (N1012 (lo), T4 or TMY) T-cells, or 1×10⁷ (N1012 (hi) or NKG2D) transduced T-cells. Tumor growth was measured weekly by bioluminescence imaging, with mice weighed thrice weekly. The data are presented as both average total flux (photons/second) per treatment group **(****Figure 10A****),** or total flux (photons/second) per individual mouse **(****Figure 10B****).** Tumor burden in mice receiving NKG2D⁺, T4⁺ or TMY⁺ T-cells was identical to those receiving PBS, suggesting a lack of efficacy. In contrast, tumor was completely eradicated in 2/5 mice and 4/5 mice treated with either N1012 (lo) or N1012 (hi) respectively. These mice remained tumor free 76 days after T-cell administration. No evidence of toxicity was observed when assessed by measurement of percentage change in body weight **(****Figure 10C****).**

To determine whether N1012⁺ T-cells could engraft within NSG mice and provide immunological memory, those mice that had completely rejected tumor received a second inoculation of 1×10⁵ ffLUC-tagged BxPC3 cells into the peritoneal cavity 88 days after initial tumor inoculation (76 days post T-cell infusion). While an increase in luminescence was observed by BLI 24 hours after tumor re-challenge, 4/5 mice subsequently demonstrated a substantial reduction in tumor size, thus indicating re-activation of the N1012⁺ T-cells **(****Figure 10D****).** The experiment was ended after 145 days and a survival curve generated, which demonstrated the potent anti-tumor efficacy of the N1012 T-cells **(****Figure 10E****).**

To further confirm the efficacy of N1012⁺ T-cells against a pancreatic cancer model *in vivo,* a repeat experiment was undertaken. 1×10⁷ CAR⁺ or control untransduced T-cells were injected i.p. into NSG mice twelve days after inoculation with 1×10⁵ ffLUC-tagged BxPC3 cells. Tumor growth was monitored weekly by bioluminescence imaging and the data are presented as both average total flux (photons/second) per treatment group **(****Figure 11A****),** and total flux (photons/second) per individual mouse **(****Figure 11B****).** Significant and sustained tumor regression was once again observed in mice treated with N1012⁺ T-cells. Indeed, tumor was completely eradicated in 5/6 mice treated with N1012⁺ T-cells. In contrast, the kinetics of tumor growth in mice treated with UT T-cells were identical to those receiving PBS. These data confirm that tumor eradication was N1012⁺-specific.

To investigate the potential formation of memory T-cells, mice that were tumor free at day 41 (29 days after T-cell infusion) were re-challenged i.p. with a fresh bolus of 1×10⁵ ffLUC-tagged BxPC3 cells. Mice were imaged on day 42 to confirm tumor take. Subsequent imaging demonstrated that 5/5 re-challenged mice reduced tumor burden below detection, with 3/5 mice demonstrating long term tumor control **(****Figure 11B****).** These data suggest that N1012 CAR T-cells can form memory populations that can re-activate in response to re-emergence of the target.

### 4.7.8. Example 8: Efficacy of N1012 T-cells in an in vivo model of malignant mesothelioma

To confirm the efficacy of N1012 in another *in vivo* model, NSG mice were inoculated via intraperitoneal injection with 1×10⁶ ffLUC-tagged H226 malignant mesothelioma cells. Eight days after tumor cell inoculation, mice were treated with either PBS, or 4×10⁶ N1012⁺ T-cells. As a control, one group of mice were treated with 4×10⁶ T-cells expressing NKG2D alone. Tumor growth was monitored weekly by bioluminescence imaging and the data are presented as average total flux (photons/second) per treatment **(****Figure 12A****)** and as total flux (photons/second) per individual mouse **(****Figure 12B****).** Whereas consistent tumor growth was observed in the mice that received PBS, 100% tumor eradication was observed in mice receiving N1012⁺ T-cells.

To confirm T-cell persistence and maintenance of function, all tumor-free mice were inoculated i.p. with an additional 1×10⁶ ffLUC-tagged H226 cells, 91 days after initial tumor inoculation. Tumor take was confirmed in all mice after 24 hours by bioluminescence imaging. All re-challenged mice rejected the tumor, confirming persistence of the N1012⁺ T-cells and the ability of these T-cells to mediate long-term tumor control.

### 4.7.9. Example 9: Comparison of N1012 T-cells to CYAD-01 replica T-cells

The restimulation and proliferation potential of N1012 T-cells was compared to CYAD-01 replica T-cells. As noted previously, the CYAD-01 CAR consists of a fusion of NKG2D to CD3ζ and represents a human version of the mouse CAR originally described by Sentman et al (Zhang et al, 2005, Blood 106:1544-1551). Although nominally a first-generation CAR, it associates with endogenous DAP10 in T-cells, meaning that both signals 1 and 2 are provided. The CYAD-01 CAR is currently undergoing clinical development by Celyad Oncology, and a replicate of this CAR is provided in these examples for the purposes of comparison only.

Surface expression of CYAD-01 replicate was confirmed in primary human T-cells when assessed by flow cytometry **(****Figure 13****).**

Briefly, N1012 or CYAD-01 replicate T-cells were co-cultured with fresh monolayer twice weekly until monolayer destruction was not observed. To achieve this, 1×10⁵ tumor cells were plated in triplicate wells of a 24-well plate and incubated for 24 hours at 37°C and 5% CO2. Twenty-four hours later, 1×10⁵ CAR⁺ T-cells were added per well at a final concentration of 1×10⁵ CAR⁺/mL. After 72 hours, the T-cells were gently removed and the well was washed with 1mL PBS. Following removal of the PBS, 1mL of MTT (at a final concentration of 500 µg/mL) was added to each well and the plate incubated at 37°C and 5% CO₂ for approximately 1 hour. The plate was read and tumor cell viability calculated as detailed above. A re-stimulation was considered successful if the tumor cell viability was measured as less than 50%.

To investigate T-cell proliferation in response to target cell recognition, the T-cells that had been removed from the plate were centrifuged at 400xg for 5 minutes and the supernatant removed. The pellet was re-suspended in 3.2mL R5 media and 1mL added to each well of fresh tumor monolayer in triplicate. Total T-cell number was assessed by trypan blue exclusion of a small aliquot of the remaining 200µL.

When compared to the CYAD-01 replicate, NKG2D CAR and control T-cells, N1012⁺ T-cells underwent significantly more rounds of re-stimulation upon BxPC3_LT-cells **(****Figure 14A****).** Furthermore, N1012 T-cells also demonstrated substantially greater mean fold expansion than CYAD-01 replicate T-cells or controls **(****Figure 14B****).**

When co-cultured with tumor spheroids, a significant reduction in spheroid viability was observed with N1012⁺ T-cells, but not with UT control T-cells, nor with those expressing the functional CYAD-01 replicate CAR **(****Figure 15A****).** N1012 T-cells also demonstrated significant proliferation compared to either UT or CYAD-01 replicate T-cells **(****Figure 15B****).**

### 4.7.10. Example 10: Generating N1012 T-cells and N1012_CXCR2 T-cells

T-cells expressing a CAR construct (e.g., N1012, N1012_CXCR2, CYAD-01_10) were generated by isolating peripheral blood mononuclear cells (PBMCs), activating the T-cells, and transducing the T-cells with virus containing nucleic acid encoding the CAR **(****Figures 16A-16B****).**

Briefly, to generate virus, 1.65×10⁶ HEK293T cells were seeded in a 10cm² tissue culture dish in 10mL of IMDM media containing 10% FBS and 2mM L-glutamine (I10 media) and incubated for 24 hours at 37°C at 5% CO₂. The following day, transfection mix was generated for each CAR construct according to the protocol in Table 3. The HEK293T cells were separately transfected with the following plasmids: **N1012** (encoding DAP10/12 fusion protein and human NKG2D receptor (SEQ ID NO: 74)), **N1012_CXCR2** (encoding DAP10/12 fusion protein, human NKG2D receptor, and CXCR2 (SEQ ID NO: 91)), **CYAD-01** replica (encoding NKG2D receptor fused to CD3ζ (SEQ ID NO: 93)), and **CYAD-01_10** (encoding CYAD-01 replica and DAP10 (SEQ ID NO: 94).

**Table 3: Transfection protocol for CAR constructs (volumes per 10cm² dish)**

| **Reagent** | **Volume/Amount** | Incubation **Time** |
|---|---|---|
| Serum-free media | 470 µL | Incubate for 5 minutes at room temperature. This is Mix A |
| Genejuice | 30 µL | |
| PeqPam-3 | 4.6875 µg | Add all three plasmids to Mix A, gently mix and then incubate for 15 minutes at room temperature. This is now Mix B |
| RDF | 3.125 µg | |
| SFG vector containing CAR (e.g. N1012_CXCR2) | 4.6875 µg | |

Upon completion of the 15 minute incubation, Mix B was added dropwise to the HEK293T cells and gently swirled. The cells were then placed back in the incubator. Supernatant was harvested 48 hours after transfection, collected into pre-chilled 50 mL Falcon tubes, and stored at 4°C.

HEK293T cells were fed with 10 mL of fresh I10 media and returned to the incubator. After an additional 24 hours, the supernatant was harvested a second time from the HEK293T cells and combined with the supernatant harvested 48 hours after transfection. The combined supernatant was aliquoted into pre-labeled tubes, snap frozen, and stored at -80°C.

PBMCs were isolated using standard Ficoll Paque-mediated density centrifugation. Once re-suspended at a concentration of 3×10⁶ cells/mL in RPMI + 5% normal human AB serum and 2mM L-glutamine ('R5' media), the T-cells were activated using paramagnetic beads coated with anti-human CD3 and anti-human CD28 antibodies (1:2 cell Lead ratio), or phytohaemagglutinin (PHA) at a concentration of 5ug/mL. Forty-eight hours after activation, 1×10⁶ T-cells were plated onto RetroNectin-coated non-tissue culture treated plates and mixed with 3mL viral supernatant harvested from transiently transfected HEK 293T cells. T-cells were fed with 100IU/mL IL-2 in RPMI1640 media + 5% normal human AB serum, with fresh media and IL-2 (100IU/mL) provided thrice weekly.

Surface expression of the N1012, N1012_CXCR2, CYAD-01 and CYAD-01_10 CARs on T-cells was assessed by flow cytometry. Briefly, T-cells were stained with fluorescein isothiocyanate (FITC)-conjugated anti-human CD4, phycoerythrin (PE)-conjugated anti-human NKG2D, Alexafluor_647 (AF_647)-conjugated anti-human CXCR2 and allophycocyanin cyanine7 (APCCy7)-conjugated anti-human CD8α antibodies on ice for 30 minutes. As a control for background CXCR2 expression, one tube was stained with FITC-conjugated CD4, PE-conjugated NKG2D, APCCy7-conjugated CD8α and AF_647-conjugated isotype antibodies. After washing in 2 mL ice-cold PBS, the cells were re-suspended in 0.5 mL ice-cold PBS and assessed by flow cytometry. Due to endogenous expression of NKG2D in CD8⁺ T-cells, gene transfer efficiency was calculated within the CD4⁺T-cells and compared to that seen in untransduced (UT) T-cells. Results shown in **Figure 16C** demonstrate that high levels of gene transfer are achieved with all constructs (Figure 16C, left panel) but that N1012 has significantly higher surface expression compared to other CARs, including N1012_CXCR2 (Figure 16C, right panel).

### 4.7.11. Example 11: Evaluating efficacy of N1012_CXCR2 T-cells against human ovarian cancer cells

Cytotoxicity (MTT) assays were performed to assess anti-tumor efficacy of CAR T-cells *in vitro* on ovarian cancer cells. Briefly, two epithelial ovarian cancer cell lines representative of high grade serous ovarian cancer (HGSOC) - Kuramochi and Ovsaho - were separately co-cultured with CAR T-cells (N1012, N1012_CXCR2, CYAD-01 replica, or CYAD-01_10) at log2 effector:target ratios ranging from 1: 1 to 1:64. Tumor cell viability was assessed 48 hours after T-cell addition by performing MTT assays as described above. Tumor cell viability was expressed as a percentage of tumor cells grown in the absence of T-cells **(****Figure 17****).** Results show that N1012, N1012_CXCR2, CYAD-01 replica, and CYAD-01_10 T-cells demonstrated potent cytotoxicity in both tumor cell lines **(****Figure 17****).** By contrast, untransduced T-cells mediated negligible tumor cell death.

### 4.7.12. Example 12: N1012 and N1012_CXCR2 T-cells demonstrate superior proliferation

Re-stimulation assays were performed to assess the ability of CAR T-cells to undergo multiple rounds of target cell lysis. Briefly, CAR-T cells were co-cultured with Ren mesothelioma tumor cells at a 1:1 effector ratio. After 72 hours, the T-cells were gently removed and each well gently washed with 1mL PBS. Following removal of the PBS, 0.5mL MTT solution was added per well and the plates incubated at 37°C and 5% CO2 for approximately 1 hour. Following removal of the MTT solution, the resulting formazan crystals were solubilized in DMSO (0.5mL/well) and the absorbance measured at 560nm. Tumor cell viability was calculated as follows: (Absorbance of monolayer with T-cells/absorbance of monolayer without T-cells)* 100. The T-cells that had been removed from the monolayer were centrifuged at 400xg for 5 minutes and the supernatant removed. The pellet was re-suspended in 3.2 mL R5 media and 1 mL was added to each well of fresh tumor monolayer in triplicate. The assay was repeated twice weekly until the T-cells failed to mediate greater than 40% target cell lysis. To investigate T-cell proliferation in response to target cell recognition, T-cell number was assessed by trypan blue exclusion of a small aliquot of the remaining 200µL. Fold-expansion was calculated as follows (highest total T-cell number achieved during re-stimulation/initial T-cell number seeded).

Results shown in **Figure 18** demonstrate significantly improved proliferation and persistence of N1012 and N1012_CXCR2 T-cells during re-stimulation assays compared to CYAD-01 replica and CYAD-01_10 T-cells. Whereas no expansion of untransduced T-cells or T-cells expressing NKG2D was observed, all CAR⁺ T-cells proliferated from the number initially seeded. The level of expansion of N1012 and N1012_CXCR2 T-cells was substantially greater than that observed with either CYAD-01 replica or CYAD-01_10 T-cells. Furthermore, while the number of CYAD-01 replica and CYAD-01_10 T-cells rapidly diminished upon additional rounds of stimulation, both N1012 and N1012_CXCR2 T-cells demonstrated robust proliferation. Indeed, N1012 and N1012_CXCR2 T-cells demonstrated a 59.7-fold and 58.1-fold maximum expansion, respectively, in T-cell number, when compared to the number of cells initially seeded. It was also apparent that both N1012 and N1012_CXCR2 T-cells demonstrated bursts of proliferation, followed by a contraction in T-cell number, which were then followed by additional rounds of proliferation **(****Figure 18****).** This pattern of proliferation and contraction mirrors that of an endogenous immune response.

### 4.7.13. Example 13: Assessing T-cell differentiation, CD4:CD8 ratio, and expression of co-stimulatory molecules following T-cell stimulation

A small aliquot of T-cells was removed from ongoing re-stimulation assays following stimulation 16 and stimulation 23 and the T-cells were stained with FITC-conjugated anti-human CD4 and APCCy7-conjugated CD8α antibodies. T-cells were stained separately for CD27 using a PECy7-conjugated antibody. T-cells were assessed by flow cytometry. At both timepoints, N1012 and N1012_CXCR2 T-cells demonstrated an almost complete shift towards the CD8⁺ subset, with very few CD4⁺ cells detected **(****Figures 19A-19B****).** The shift to CD8⁺ was more pronounced following stimulation 23 than stimulation 16 (comparing **Figure 19B** to **Figure 19A****).** Critically, the N1012 T-cells retained expression of a key co-stimulatory molecule, CD27, even after 28 rounds of re-stimulation **(****Figure 20****,** right panel). Results also demonstrate that N1012 T-cells maintain some memory T-cells (CD45RO⁺ CD62L⁺) following 28 rounds of stimulation **(****Figure 20****,** left panel).

N1012 T-cells further appeared to cycle between central memory T-cells (CD45RO⁺ CD62L⁺) and effector memory T-cells (CD45RO⁺ CD62L⁻). Minimal evidence of terminal exhaustion (CD45RO⁻ CD62L⁻) was observed, even at advanced rounds of re-stimulation **(****Figure 21****).**

### 4.7.14. Example 14: Assessing avidity of CAR T-cells

In order to assess binding avidity of NKG2D-targeted CAR T-cells for target cells, the percentage of T-cells bound to target cells at increasing levels of acoustic force was measured. Briefly, CD19-engineered LO68 or SKOV-3 tumor cells (both of which express NKG2D ligands) were seeded in a z-Movi microfluidic chip (Lumicks, Amsterdam, Netherlands) coated with poly-L-lysine and cultured for 16 hours. The next day, flow sorted CAR-T cells were serially flowed in the chips and incubated with the target cells for 5 minutes prior to initializing a 3-minute linear force ramp. During the force ramp, the z-Movi device (Lumicks) captured a time series of images using a bright field microscope integrated into the platform. Detached cells were levitated towards the acoustic nodes, allowing the tracking of cells based on their XY positions. Changes in the Z-position resulted in a change in the diffraction pattern, which permitted distinction between cells adhered to the substrate and cells suspended to the acoustic nodes. This information was used to correlate cell detachment events with a specific rupture force. Cell detachment was acquired using (z-Movi Tracking_v1.6.0) and post experiment image analysis performed using Cell Tracking offline analysis _v2.1.

Results shown in **Figure 22** demonstrate that avidity of the CAR T-cells appeared to mirror the level of cell surface expression of the CAR **(****Figure 16C****).** N1012 CAR T-cells showed higher avidity than N1012_CXCR2, CYAD-01 replica, and CYAD-01_10 CAR T-cells for both Lo68-19 and SKOV3 cells **(****Figure 22****).** All NKG2D CAR T-cells demonstrated lower binding avidity than the CD19-specific T-cells (FMC63) for Lo68_19 cells, reflecting the lower affinity of NKG2D compared to FMC63 for its ligand(s).

### 4.7.15. Example 15: Assessing cytokine secretion from N1012 and N1012_CXCR2 T-cells

Secretion of Interferon-gamma (IFN-γ) and Interleukin-2 (IL-2) by CAR T-cells was assessed by ELISA following co-culture with various tumor cell lines. CAR T-cells were separately co-cultured with cells derived from ovarian cancer (Kuramochi, Ovsaho) and pancreatic cancer (CFPac-1) for 24 hours. Supernatant was subsequently removed and assessed by ELISA as previously described.

Results demonstrate thatN1012_CXCR2 T-cells secrete substantially less IFN-γ **(****Figure 23A****)** and IL-2 **(****Figure 23B****)** than N1012 T-cells across all cell lines tested.

### 4.7.16. Example 16: Unstimulated N1012 and N1012_CXCR2 T-cells demonstrate improved expansion ex vivo compared to CYAD-01 replica or CYAD-01_10 T-cells

Experiments were performed to evaluate expansion and, consequentially, scale-up potential of N1012, N1012_CXCR2, CYAD-01 replica, and CYAD-01_10 T-cells. Briefly, T-cells were transduced as previously described, and fed every 48-72 hours with 100% volume R5 media supplemented with 100 IU/mL recombinant human IL-2. Cell expansion was calculated as total number of T-cells at the end of the 12 day culture period/initial number of T-cells transduced.

Results show both N1012 and N1012_CXCR2 T-cells demonstrate levels of expansion that slightly exceed those of untransduced T-cells **(****Figure 24****).** In contrast, CYAD-01 replica and CYAD-01_10 T-cells demonstrate substantially poorer expansion compared to control T-cells **(****Figure 24****).**

### 4.7.17. Example 17: Evaluating anti-tumor efficacy of N1012_CXCR2 T-cells in vivo in mouse models of ovarian cancer

In order to evaluate efficacy of CAR T-cells on ovarian tumors *in vivo,* CAR T-cells were injected into mice bearing firefly luciferase (ffLuc)-expressing Ovsaho ovarian tumor xenografts, ffLuc-expressing SKOV-3 ovarian tumor xenografts, or ffLuc-expressing Kuramochi tumor xenografts. Ovsaho tumor cells express low levels of IL-8; SKOV-3 tumor cells express moderate levels of IL-8. Tumor growth was subsequently monitored by bioluminescence imaging.

Briefly, 1x10⁵ ffLuc-expressing Ovsaho or Kuramochi or 5×10⁵ ffLuc-expressing SKOV-3 cells were injected into the intraperitoneal cavity of NSG mice. Mice inoculated with Ovasho cells were treated with either PBS [n=5] or 5×10⁶ CAR T-cells (N1012 [n=7], N1012_CXCR2 [n=7], CYAD-01 replica [n=4], or untransduced T-cells) administered via intraperitoneal injection (6 days after tumor inoculation) or intravenous injection (7 days after inoculation). Mice inoculated with SKOV-3 cells were treated intravenously with either PBS [n=5] or 1x10⁷ CAR T-cells (N1012 [n=11], N1012_CXCR2 [n=11], or CYAD-01_10 [n=8]) 14 days after tumor inoculation. On day 17 following inoculation, mice inoculated with Kuramochi cells were treated via intraperitoneal injection with PBS or 2×10⁶ CAR T-cells (N1012 or N1012_CXCR2) or untransduced T-cells. On day 18, mice inoculated with Kuramochi cells were treated via intravenous injection with PBS or 2×10⁶ CAR T-cells (N1012 or N1012_CXCR2) or untransduced T-cells.

Results show an initial anti-tumor effect on Ovsaho cell tumors in mice treated with N1012, N1012_CXCR2, and CYAD_01 replicate T-cells **(****Figure 25** and **Figure 38****).** Ovasho xenograft mice were re-challenged with tumor on Day 56 following initial tumor inoculation by intraperitoneal injection of 1x10⁵ ffLuc-expressing Ovsaho cells. Following tumor re-challenge, tumor growth was not observed in mice that had been treated with N1012 or N1012_CXCR2 T-cells **(****Figure 38****).** By comparison, mice that had been treated with CYAD-01 replica T-cells showed a marked increase in tumor growth following tumor re-challenge **(****Figure 38****).** The results demonstrate the superior anti-ovarian tumor activity of N1012_CXCR2 and N1012 T-cells compared to CYAD-01 replica T-cells.

SKOV-3 xenograft mice treated with N1012_CXCR2 T-cells showed superior anti-tumor activity **(****Figure 27****)** and survival **(****Figure 37****)** compared to mice treated with N1012 or CYAD-01_10 T-cells.

Treatment with N1012 or N1012_CXCR2 T-cells also demonstrated anti-tumor activity in Kuramochi xenograft mice **(****Figure 39** and **Figure 41****).** N1012 and N1012_CXCR2-treated mice that had rejected tumors were re-challenged with tumor on day 86 by intraperitoneal injection of 1×10⁵ ffLuc-expressing Kuramochi cells. Tumor growth was inhibited in some of the re-challenged mice, demonstrating retained anti-tumor activity in the mice **(****Figure 39** and **Figure 41****).** **Figures 40** **and** **43** are Kaplan-Meier survival curves of the Kuramochi xenograft-bearing mice treated in the experiments detailed in **Figure 39** and **Figure 41****,** respectively. The T-cells used in a single experiment were from the same donor. T-cells used in the experiment detailed in **Figure 41** were from a different donor than the T-cells used in the experiment detailed in **Figure 39****.**

In order to evaluate T-cell trafficking, T-cells were double transduced to express a CAR construct (N1012 or N1012_CXCR2) and a reporter construct (rLuc alone or rLuc co-expressed with GFP)).

Mice inoculated with Ovasho cells as previously described were treated with 5×10⁶ CAR T-cells (N1012_SW [n=3] or N1012_CXCR2_rLuc/GFP [n=3]) by intravenous injection 7 days after tumor inoculation. Results of bioluminescence imaging **(****Figure 26****,** right panel) show that between days 1-3 following T-cell injection, N1012_CXCR2_rLuc/GFP T-cells trafficked to the peritoneum more efficiently than N1012_SW T-cells while equivalent anti-tumor activity was observed in the two treatment groups **(****Figure 26****,** left and middle panels). Given that Ovsaho cells express IL-8 at moderate to low levels, the increased trafficking of N1012_CXCR2_rLuc/GFP T-cells may be due to CXCR2 ligands other than IL-8.

Mice inoculated with SKOV-3 cells as previously described were treated with 1×10⁷ CAR T-cells (N1012_CXCR2_rLuc/GFP or N1012_rLuc/GFP ) by intravenous injection 14 days after tumor inoculation. Results shown in **Figure 28** and **Figure 29** demonstrate that N1012_CXCR2 T-cells trafficked more efficiently than N1012 T-cells in the first four days following T-cell administration **(****Figure 28****,** right panel and **Figure 29****).** Results shown in **Figure 28** (left panel) further demonstrate that mice treated with N1012_rLuc/GFP and N1012_CXCR2_rLuc/GFP T-cells maintain anti-tumor activity more than 60 days following T-cell administration.

Mice inoculated with Kuramochi cells as previously described were treated on Day 18 post tumor inoculation with 2×10⁶ CAR T-cells co-transduced with either N1012 or N1012_CXCR2 and the dual reporter, rLuc/GFP. Results shown in **Figure 42** demonstrate that N1012_CXCR2 T-cells trafficked more efficiently into the peritoneal cavity than N1012 T-cells in the first four days following T-cell administration.

### 4.7.18. Example 18: Evaluating anti-tumor efficacy of N1012_CXCR2 T cells in vivo in mouse models of pancreatic cancer

In order to evaluate efficacy of CAR T-cells on pancreatic tumors *in vivo,* CAR T-cells were injected into mice bearing either CFPac-1 or BxPC3 tumor cells.

Briefly, 1x10⁵ CFPac-1 or BxPC3 cells were injected subcutaneously in 50 µl of Matrigel (1:1 PBS) into the left flank of NSG mice. Twenty-nine days after inoculation with CFPac-1 cells or fourteen days after inoculation with BxPC3 cells, mice were treated intravenously with either PBS or 1×10⁷ CAR T-cells (N1012_CXCR2, N1012, NKG2D, CYAD-01 replica or untransduced (UT) T-cells). Three days following T-cell injections, three mice from each of the N1012, N1012_CXCR2, and UT T-cell treatment groups were sacrificed and tumors were harvested for immunohistochemistry (IHC) analysis. Tumor growth was measured weekly by caliper measurements and presented as tumor volume (mm³).

Results show that tumors in CFPac-1 xenograft mice treated with N1012_CXCR2 T-cells were completely eradicated following T-cell administration and no tumor relapse was observed **(****Figure 30****).** In contrast, tumor growth in CFPac-1 xenograft mice treated with N1012 and CYAD-01 replica T-cells was initially inhibited following T-cell administration prior to loss of anti-tumor activity and tumor relapse **(****Figure 30****).** IHC analysis showed improved infiltration of N1012_CXCR2 T-cells into CFPac-1 tumors compared to N1012 or NKG2D T-cells **(****Figure 31****).**

BxPC3 xenograft mice treated with N1012_CXCR2 T-cells showed improved tumor inhibition **(****Figure 33****)** and survival **(****Figure 34****)** compared to mice treated with N1012 or CYAD-01 replica T-cells, suggesting that the presence of CXCR2 improves homing of the CAR T-cells to IL-8-producing BxPC3 tumors.

Additional studies were performed to compare treatment of high and low doses of CAR T-cells. Briefly, 1x10⁵ CFPac-1 cells were injected into the intraperitoneal cavity of NSG mice. Twenty eight days after tumor inoculation, mice were treated with either PBS [n=5], a high dose of 10×10⁶ CAR T-cells (N1012 [n=7], N1012_CXCR2 [n=7], CYAD-01 replica [n=5]) or a low dose of 4×10⁶ CAR T-cells (N1012 [n=6], N1012_CXCR2 [n=7]). Results shown in **Figure 32** demonstrate that efficacy of treatment with a low dose of N1012_CXCR2 T-cells tracks treatment with a high dose of N1012 T-cells.

### 4.7.19. Example 19: Evaluating anti-tumor efficacy of N1012_CXCR2 T-cells in a mesothelioma patient derived xenograft (PDX) tumor model

A mesothelioma patient derived xenograft (PDX) mouse tumor model was generated to evaluate efficacy of N1012 and N1012_CXCR2 CAR T-cell treatment of mesothelioma *in vivo.* In order to establish the mesothelioma PDX model, primary patient mesothelioma tumor fragments (2 x 2 mm) were engrafted subcutaneously into the left flank of NSG mice using 15G trocar implant needles. The tumors were grown over a period of six months and were passaged three times into new cohorts. At passage 4, an experimental cohort of male NSG mice were implanted with PDX material into the left flank. On Day 111 following tumor engraftment, mice were intravenously administered either PBS [n=8] or 1×10⁷ CAR T-cells (untransduced (UT), N1012_CXCR2 [n=8], N1012 [n=9] or CYAD-01_10 [n=5]). A subgroup of mice was treated with T-cells transduced with luciferase and TD tomato (LT) with or without a CAR in order to track T-cells by bioluminescence imaging (N1012_CXCR2_LT [n=3], N1012_LT or LT alone [n=3]). Tumor growth was measured weekly by caliper measurements and presented as tumor volume (mm³).

Results in **Figure 35** and **Figure 44** show that anti-tumor activity was observed in mice treated with N1012 and N1012_CXCR2 T-cells. In contrast, mice treated with CYAD-01_10 T-cells did not respond well to treatment. **Figure 36** shows bioluminescence imaging of T-cells, demonstrating that N1012 and N1012_CXCR2 T-cells localized to the tumor site in the mesothelioma PDX mice whereas T-cells transduced with only the LT reporter (no CAR) did not localize to tumor. A Kaplan-Meier survival curve of the mice in the study is shown in **Figure 45****.**

### 6. SEQUENCES

SEQ ID NO: 1 (human DAP10 full sequence) 
MIHLGHILFL LLLPVAAAQT TPGERSSLPA FYPGTSGSCS GCGSLSLPLL AGLVAADAVA
SLLIVGAVFL CARPRRSPAQ EDGKVYINMP GRG
SEQ ID NO: 2 (DAP10 aa19-93 - lacking leader sequence) 
QTTPGERSSL PAFYPGTSGS CSGCGSLSLP LLAGLVAADA VASLLIVGAV FLCARPRRSP
AQEDGKVYIN MPGRG
SEQ ID NO: 3 (DAP10 aa19-69 - extracellular/transmembrane domain)
   QTTPGERSSL PAFYPGTSGS CSGCGSLSLP LLAGLVAADA VASLLIVGAV F
SEQ ID NO: 4 (DAP10 aa1-71) 
MIHLGHILFL LLLPVAAAQT TPGERSSLPA FYPGTSGSCS GCGSLSLPLL AGLVAADAVA
SLLIVGAVFL C
SEQ ID NO: 5 (DAP10 aa19-71)
   QTTPGERSSL PAFYPGTSGS CSGCGSLSLP LLAGLVAADA VASLLIVGAV FLC
**SEQ ID NO: 6 (DAP10 aa70-93 - intracellular domain)**
   LCARPRRSPA QEDGKVYINM PGRG
**SEQ ID NO: 7 (DAP10 aa49-93 - transmembrane and intracellular domain)**
   LLAGLVAADA VASLLIVGAV FLCARPRRSP AQEDGKVYIN MPGRG
**SEQ ID NO: 8 (DAP10 aa49-69 - transmembrane domain)**
   LLAGLVAADA VASLLIVGAV F
**SEQ ID NO: 9 (human DAP12 full sequence)** 
MGGLEPCSRL LLLPLLLAVS GLRPVQAQAQ SDCSCSTVSP GVLAGIVMGD LVLTVLIALA
VYFLGRLVPR GRGAAEAATR KQRITETESP YQELQGQRSD VYSDLNTQRP YYK
**SEQ ID NO: 10 (DAP12 aa22-113 - lacking leader sequence)** 
LRPVQAQAQS DCSCSTVSPG VLAGIVMGDL VLTVLIALAV YFLGRLVPRG RGAAEAATRK
QRITETESPY QELQGQRSDV YSDLNTQRPY YK
**SEQ ID NO: 11 (DAP12 aa62-113 - cytoplasmic/intracellular domain)**
   YFLGRLVPRG RGAAEAATRK QRITETESPY QELQGQRSDV YSDLNTQRPY YK
**SEQ ID NO: 12 (DAP12 aa41-61 - transmembrane domain)**
   GVLAGIVMGD LVLTVLIALA V
**SEQ ID NO: 13 (DAP12 aa22-61 - extracellular and transmembrane domains)**
   LRPVQAQAQS DCSCSTVSPG VLAGIVMGDL VLTVLIALAV
**SEQ ID NO: 14 (human NKG2D full sequence)** 
MGWIRGRRSR HSWEMSEFHN YNLDLKKSDF STRWQKQRCP VVKSKCRENA SPFFFCCFIA
VAMGIRFIIM VAIWSAVFLN SLFNQEVQIP LTESYCGPCP KNWICYKNNC YQFFDESKNW
YESQASCMSQ NASLLKVYSK EDQDLLKLVK SYHWMGLVHI PTNGSWQWED GSILSPNLLT
IIEMQKGDCA LYASSFKGYI ENCSTPNTYI CMQRTV
**SEQ ID NO: 15 (human NKG2D aa73-216 - extracellular domain)** 
IWSAVFLNSL FNQEVQIPLT ESYCGPCPKN WICYKNNCYQ FFDESKNWYE SQASCMSQNA
SLLKVYSKED QDLLKLVKSY HWMGLVHIPT NGSWQWEDGS ILSPNLLTII EMQKGDCALY
ASSFKGYIEN CSTPNTYICM QRTV
**SEQ ID NO: 16 (human NKG2D aa82-216 - extracellular domain)** 
LFNQEVQIPL TESYCGPCPK NWICYKNNCY QFFDESKNWY ESQASCMSQN ASLLKVYSKE
DQDLLKLVKS YHWMGLVHIP TNGSWQWEDG SILSPNLLTI IEMQKGDCAL YASSFKGYIE
NCSTPNTYIC MQRTV
**SEQ ID NO: 17 (human NKG2D aa52-216 - transmembrane and extracellular domain)** 
PFFFCCFIAV AMGIRFIIMV AIWSAVFLNS LFNQEVQIPL TESYCGPCPK NWICYKNNCY
QFFDESKNWY ESQASCMSQN ASLLKVYSKE DQDLLKLVKS YHWMGLVHIP TNGSWQWEDG
SILSPNLLTI IEMQKGDCAL YASSFKGYIE NCSTPNTYIC MQRTV
**SEQ ID NO: 18 (linker)**
   GSG
**SEQ ID NO: 19 (linker)**
   GSGGG
SEQ ID NO: 20 (linker)
   GSGG
**SEQ ID NO: 21 (linker)**
   SGGG
**SEQ ID NO: 22 (linker)**
   GGGGS
**SEQ ID NO: 23 (linker)**
   GGGGSGGGGSGGGGSGGGGS
**SEQ ID NO: 24 (linker)**
   GGGGSGGGGSGGGGS
**SEQ ID NO: 25 (linker)**
   GPPGS
**SEQ ID NO: 26 (linker)**
   GGGS
**SEQ ID NO: 27 (linker)**
   GGGGS
**SEQ ID NO: 28 (linker)**
   GYS
**SEQ ID NO: 29 (linker)**
   GS
**SEQ ID NO: 30 (linker)**
   SGGGG
**SEQ ID NO: 31 (linker)**
   SGGG
**SEQ ID NO: 32 (linker)**
   SGG
**SEQ ID NO: 33 (linker)**
   SGSG
**SEQ ID NO: 34 (linker)**
   SG
**SEQ ID NO: 35 (linker)**
   GGGGA
**SEQ ID NO: 36 (linker)**
   GGGA
**SEQ ID NO: 37 (linker)**
   EAAAK
**SEQ ID NO: 38 (furin cleavage site)**
   RRKR
**SEQ ID NO:39 (P2A skip peptide)**
   ATNFSLLKQAGDVEENPGP
**SEQ ID NO: 40 (T2A skip peptide)**
   EGRGSLLTCGDVEENPGP
**SEQ ID NO: 41 (SGSG + P2A)**
   SGSGATNFSLLKQAGDVEENPGP
**SEQ ID NO: 42 (SGSG + T2A)**
   SGSGEGRGSLLTCGDVEENPGP
**SEQ ID NO: 43 (furin + SGSG + P2A)**
   RRKRSGSGATNFSLLKQAGDVEENPGP
**SEQ ID NO: 44 (furin + SGSG + T2A)**
   RRKRSGSGEGRGSLLTCGDVEENPGP
**SEQ ID NO: 45 (F2A skip peptide)**
   VKQTLNFDLLKLAGDVESNPGP
**SEQ ID NO: 46 (E2A skip peptide)**
   QCTNYALLKLAGDVESNPGP
**SEQ ID NO: 47 (His tag)**
   HHHHHH
**SEQ ID NO: 48 (FLAG tag)**
   DYKDDDDK
**SEQ ID NO: 49 (Avi tag)**
   GLNDIFEAQKIEWHE
**SEQ ID NO: 50 (V5 tag)**
   GKPIPNPLLGLDST
**SEQ ID NO: 51 (V5 tag)**
   IPNPLLGLD
**SEQ ID NO: 52 (Myc tag)**
   EQKLISEEDL
**SEQ ID NO: 53 (AHF tag)**
   GLNDIFEAQKIEWHEGGHHHHHHDYKDDDDK
**SEQ ID NO: 54 (FHA tag)**
   DYKDDDDKHHHHHHGGGLNDIFEAQKIEWHE
**SEQ ID NO: 55 (CD8α leader sequence)**
   MALPVTALLL PLALLLHAAR P
**SEQ ID NO: 56 (4-1BB endodomain)**
   KRGRKKLLYI FKQPFMRPVQ TTQEEDGCSC RFPEEEEGGC EL
**SEQ ID NO: 57 (CD27 endodomain)**
   QRRKYRSNKG ESPVEPAEPC HYSCPREEEG STIPIQEDYR KPEPACSP
**SEQ ID NO: 58 (human IgG1 hinge - aa 218-229 of UniProt: P0DOX5)**
   EPKSCDKTHT CP
**SEQ ID NO: 59 (truncated CD8α hinge)**
   TTTPAPRPPT PAPTIASQPL SLRPEACRPA AGGAVHTRGL DFACD
**SEQ ID NO: 60** 
MIHLGHILFLLLLPVAAAQTTPGERSSLPAFYPGTSGSCSGCGSLSLPLLAGLVAADAVASLLIVGAVFLCARPR
RSPAQEDGKVYINMPGRGYFLGRLVPRGRGAAEAATRKQRITETESPYQELQGQRSDVYSDLNTQRPYYK
**SEQ ID NO: 61** 
MALPVTALLLPLALLLHAARPDYKDDDDKQTTPGERSSLPAFYPGTSGSCSGCGSLSLPLLAGLVAADAVASLLI
VGAVFYFLGRLVPRGRGAAEAATRKQRITETESPYQELQGQRSDVYSDLNTQRPYYK
**SEQ ID NO: 62** 
MALPVTALLLPLALLLHAARPDYKDDDDKEPKSCDKTHTCPLLAGLVAADAVASLLIVGAVFLCARPRRSPAQED
GKVYINMPGRGYFLGRLVPRGRGAAEAATRKQRITETESPYQELQGQRSDVYSDLNTQRPYYK
**SEQ ID NO: 63** 
MALPVTALLLPLALLLHAARPDYKDDDDKTTTPAPRPPTPAPTIASQPLSLRPEACRPAAGGAVHTRGLDFACDL
LAGLVAADAVASLLIVGAVFLCARPRRSPAQEDGKVYINMPGRGYFLGRLVPRGRGAAEAATRKQRITETESPYQ
ELQGQRSDVYSDLNTQRPYYK
**SEQ ID NO: 64 (Construct 1/N1012)** 
MIHLGHILFLLLLPVAAAQTTPGERSSLPAFYPGTSGSCSGCGSLSLPLLAGLVAADAVASLLIVGAVFLCARPR
RSPAQEDGKVYINMPGRGYFLGRLVPRGRGAAEAATRKQRITETESPYQELQGQRSDVYSDLNTQRPYYKRRKRS
GSGATNFSLLKQAGDVEENPGPMGWIRGRRSRHSWEMSEFHNYNLDLKKSDFSTRWQKQRCPVVKSKCRENASPF
FFCCFIAVAMGIRFIIMVAIWSAVFLNSLFNQEVQIPLTESYCGPCPKNWICYKNNCYQFFDESKNWYESQASCM
SQNASLLKVYSKEDQDLLKLVKSYHWMGLVHIPTNGSWQWEDGSILSPNLLTIIEMQKGDCALYASSFKGYIENC
STPNTYICMQRTV
**SEQ ID NO: 65 (Construct 3)** 
MALPVTALLLPLALLLHAARPDYKDDDDKQTTPGERSSLPAFYPGTSGSCSGCGSLSLPLLAGLVAADAVASLLI
VGAVFYFLGRLVPRGRGAAEAATRKQRITETESPYQELQGQRSDVYSDLNTQRPYYKRRKRSGSGEGRGSLLTCG
DVEENPGPMIHLGHILFLLLLPVAAAQTTPGERSSLPAFYPGTSGSCSGCGSLSLPLLAGLVAADAVASLLIVGA
VFLCARPRRSPAQEDGKVYINMPGRGRRKRSGSGATNFSLLKQAGDVEENPGPMGWIRGRRSRHSWEMSEFHNYN
LDLKKSDFSTRWQKQRCPVVKSKCRENASPFFFCCFIAVAMGIRFIIMVAIWSAVFLNSLFNQEVQIPLTESYCG
PCPKNWICYKNNCYQFFDESKNWYESQASCMSQNASLLKVYSKEDQDLLKLVKSYHWMGLVHIPTNGSWQWEDGS
ILSPNLLTIIEMQKGDCALYASSFKGYIENCSTPNTYICMQRTV
**SEQ ID NO: 66 (Construct 8)** 
MIHLGHILFLLLLPVAAAQTTPGERSSLPAFYPGTSGSCSGCGSLSLPLLAGLVAADAVASLLIVGAVFLCARPR
RSPAQEDGKVYINMPGRGYFLGRLVPRGRGAAEAATRKQRITETESPYQELQGQRSDVYSDLNTQRPYYKRRKRS
GSGATNFSLLKQAGDVEENPGPMGWIRGRRSRHSWEMSEFHNYNLDLKKSDFSTRWQKQRCPVVKSKCRENASPF
FFCCFIAVAMGIRFIIMVAIWSAVFLNSLFNQEVQIPLTESYCGPCPKNWICYKNNCYQFFDESKNWYESQASCM
SQNASLLKVYSKEDQDLLKLVKSYHWMGLVHIPTNGSWQWEDGSILSPNLLTIIEMQKGDCALYASSFKGYIENC
STPNTYICMQRTVRRKRSGSGEGRGSLLTCGDVEENPGPMIHLGHILFLLLLPVAAAQTTPGERSSLPAFYPGTS
GSCSGCGSLSLPLLAGLVAADAVASLLIVGAVFLCKRGRKKLLYIFKQPFMRPVQTTQEEDGCSCRFPEEEEGGC
EL
**SEQ ID NO: 67 (Construct 9)** 
MIHLGHILFLLLLPVAAAQTTPGERSSLPAFYPGTSGSCSGCGSLSLPLLAGLVAADAVASLLIVGAVFLCARPR
RSPAQEDGKVYINMPGRGYFLGRLVPRGRGAAEAATRKQRITETESPYQELQGQRSDVYSDLNTQRPYYKRRKRS
GSGATNFSLLKQAGDVEENPGPMGWIRGRRSRHSWEMSEFHNYNLDLKKSDFSTRWQKQRCPVVKSKCRENASPF
FFCCFIAVAMGIRFIIMVAIWSAVFLNSLFNQEVQIPLTESYCGPCPKNWICYKNNCYQFFDESKNWYESQASCM
SQNASLLKVYSKEDQDLLKLVKSYHWMGLVHIPTNGSWQWEDGSILSPNLLTIIEMQKGDCALYASSFKGYIENC
STPNTYICMQRTVRRKRSGSGEGRGSLLTCGDVEENPGPMALPVTALLLPLALLLHAARPDYKDDDDKQTTPGER
SSLPAFYPGTSGSCSGCGSLSLPLLAGLVAADAVASLLIVGAVFLCKRGRKKLLYIFKQPFMRPVQTTQEEDGCS
CRFPEEEEGGCEL
**SEQ ID NO: 68 (Construct 10)** 
MIHLGHILFLLLLPVAAAQTTPGERSSLPAFYPGTSGSCSGCGSLSLPLLAGLVAADAVASLLIVGAVFLCARPR
RSPAQEDGKVYINMPGRGYFLGRLVPRGRGAAEAATRKQRITETESPYQELQGQRSDVYSDLNTQRPYYKRRKRS
GSGATNFSLLKQAGDVEENPGPMGWIRGRRSRHSWEMSEFHNYNLDLKKSDFSTRWQKQRCPVVKSKCRENASPF
FFCCFIAVAMGIRFIIMVAIWSAVFLNSLFNQEVQIPLTESYCGPCPKNWICYKNNCYQFFDESKNWYESQASCM
SQNASLLKVYSKEDQDLLKLVKSYHWMGLVHIPTNGSWQWEDGSILSPNLLTIIEMQKGDCALYASSFKGYIENC
STPNTYICMQRTVRRKRSGSGEGRGSLLTCGDVEENPGPMIHLGHILFLLLLPVAAAQTTPGERSSLPAFYPGTS
GSCSGCGSLSLPLLAGLVAADAVASLLIVGAVFLCQRRKYRSNKGESPVEPAEPCHYSCPREEEGSTIPIQEDYR
KPEPACSP
**SEQ ID NO: 69 (Construct 11)** 
MIHLGHILFLLLLPVAAAQTTPGERSSLPAFYPGTSGSCSGCGSLSLPLLAGLVAADAVASLLIVGAVFLCARPR
RSPAQEDGKVYINMPGRGYFLGRLVPRGRGAAEAATRKQRITETESPYQELQGQRSDVYSDLNTQRPYYKRRKRS
GSGATNFSLLKQAGDVEENPGPMGWIRGRRSRHSWEMSEFHNYNLDLKKSDFSTRWQKQRCPVVKSKCRENASPF
FFCCFIAVAMGIRFIIMVAIWSAVFLNSLFNQEVQIPLTESYCGPCPKNWICYKNNCYQFFDESKNWYESQASCM
SQNASLLKVYSKEDQDLLKLVKSYHWMGLVHIPTNGSWQWEDGSILSPNLLTIIEMQKGDCALYASSFKGYIENC
STPNTYICMQRTVRRKRSGSGEGRGSLLTCGDVEENPGPMALPVTALLLPLALLLHAARPDYKDDDDKQTTPGER
SSLPAFYPGTSGSCSGCGSLSLPLLAGLVAADAVASLLIVGAVFLCQRRKYRSNKGESPVEPAEPCHYSCPREEE
GSTIPIQEDYRKPEPACSP
**SEQ ID NO: 70 (encoding polypeptide of SEQ ID NO: 60)** 
ATGATCCACCTGGGCCACATCCTGTTCCTGCTGCTGCTGCCCGTGGCCGCTGCCCAGACCACCCCTGGCGAGCGG
AGCAGCCTGCCTGCCTTCTACCCTGGCACCAGCGGCAGCTGCAGCGGCTGCGGCAGCCTGAGCCTGCCCCTGCTG
GCCGGCCTGGTGGCCGCCGACGCCGTGGCCAGCCTGCTGATCGTGGGCGCCGTGTTCCTGTGCGCCAGGCCCAGG
CGGAGCCCtGCCCAGGAGGACGGCAAGGTGTACATCAACATGCCCGGCCGGGGCTACTTCCTGGGCAGGCTGGTG
CCCAGGGGCAGGGGCGCTGCCGAGGCTGCCACCCGGAAGCAGCGGATCACCGAGACCGAGAGCCCCTACCAGGAG
CTGCAGGGCCAGCGGAGCGACGTGTACAGCGACCTGAACACCCAGAGGCCCTACTACAAG
**SEQ ID NO: 71 (encoding polypeptide of SEQ ID NO: 61)** 
ATGGCTCTGCCTGTGACAGCTCTGCTGCTGCCTCTGGCTCTGCTGCTGCACGCCGCTAGACCCGATTATAAGGAC
GACGACGACAAGCAGACCACCCCTGGCGAGCGGAGCAGCCTGCCTGCCTTCTACCCTGGCACCAGCGGCAGCTGC
AGCGGCTGCGGCAGCCTGAGCCTGCCCCTGCTGGCtGGCCTGGTGGCCGCCGACGCCGTGGCCAGCCTGCTGATC
GTGGGCGCCGTGTTCTACTTCCTGGGCAGGCTGGTGCCCAGGGGCAGGGGCGCTGCCGAGGCTGCCACCCGGAAG
CAGCGGATCACCGAGACCGAGAGCCCCTACCAGGAGCTGCAGGGCCAGCGGAGCGACGTGTACAGCGACCTGAAC
ACCCAGAGGCCCTACTACAAG
**SEQ ID NO: 72 (encoding polypeptide of SEQ ID NO: 62)** 
ATGGCTCTGCCTGTGACAGCTCTGCTGCTGCCTCTGGCTCTGCTGCTGCACGCCGCTAGACCCGATTATAAGGAC
GACGACGACAAGGAGCCCAAGAGCTGCGACAAGACACACACATGCCCTCTTctggccggCCTGGTGGCCGCCGAC
GCCGTGGCCAGCCTGCTGATCGTGGGCGCCGTGTTCCTGTGCGCCAGGCCCAGGCGGAGCCCtGCCCAGGAGGAC
GGCAAGGTGTACATCAACATGCCCGGCCGGGGCTACTTCCTGGGCAGGCTGGTGCCCAGGGGCAGGGGCGCTGCC
GAGGCTGCCACCCGGAAGCAGCGGATCACCGAGACCGAGAGCCCCTACCAGGAGCTGCAGGGCCAGCGGAGCGAC
GTGTACAGCGACCTGAACACCCAGAGGCCCTACTACAAG
**SEQ ID NO: 73 (encoding polypeptide of SEQ ID NO: 63)** 
ATGGCTCTGCCTGTGACAGCTCTGCTGCTGCCTCTGGCTCTGCTGCTGCACGCCGCTAGACCCGATTATAAGGAC
GACGACGACAAGACCACAACACCTGCTCCTAGACCTCCCACCCCTGCTCCCACCATCGCCAGCCAGCCCCTGAGC
 
CTGAGACCCGAGGCCTGCAGACCCGCTGCTGGCGGCGCTGTGCATACCAGAGGCCTGGATTTCGCCTGCGACCTT
ctggccggCCTGGTGGCCGCCGACGCCGTGGCCAGCCTGCTGATCGTGGGCGCCGTGTTCCTGTGCGCCAGGCCC
AGGCGGAGCCCtGCCCAGGAGGACGGCAAGGTGTACATCAACATGCCCGGCCGGGGCTACTTCCTGGGCAGGCTG
GTGCCCAGGGGCAGGGGCGCTGCCGAGGCTGCCACCCGGAAGCAGCGGATCACCGAGACCGAGAGCCCCTACCAG
GAGCTGCAGGGCCAGCGGAGCGACGTGTACAGCGACCTGAACACCCAGAGGCCCTACTACAAG
SEQ ID NO: 74 (encoding polypeptide of SEQ ID NO: 64/construct 1/N1012) 
ATGATCCACCTGGGCCACATCCTGTTCCTGCTGCTGCTGCCCGTGGCCGCTGCCCAGACCACCCCTGGCGAGCGG
AGCAGCCTGCCTGCCTTCTACCCTGGCACCAGCGGCAGCTGCAGCGGCTGCGGCAGCCTGAGCCTGCCCCTGCTG
GCCGGCCTGGTGGCCGCCGACGCCGTGGCCAGCCTGCTGATCGTGGGCGCCGTGTTCCTGTGCGCCAGGCCCAGG
CGGAGCCCtGCCCAGGAGGACGGCAAGGTGTACATCAACATGCCCGGCCGGGGCTACTTCCTGGGCAGGCTGGTG
CCCAGGGGCAGGGGCGCTGCCGAGGCTGCCACCCGGAAGCAGCGGATCACCGAGACCGAGAGCCCCTACCAGGAG
CTGCAGGGCCAGCGGAGCGACGTGTACAGCGACCTGAACACCCAGAGGCCCTACTACAAGAGGCGGAAAAGGTCT
GGGAGTGGGGCTACCAATTTCTCTCTCCTCAAGCAAGCCGGAGACGTTGAGGAAAACCCTGGaCCcATGGGCTGG
ATCCGGGGACGGAGGAGCCGGCACAGCTGGGAGATGAGCGAGTTCCACAACTACAACCTGGACCTGAAGAAGAGC
GACTTCAGCACCCGGTGGCAGAAGCAGCGGTGCCCCGTGGTGAAGAGCAAGTGCCGGGAGAACGCCAGCCCCTTC
TTCTTCTGCTGCTTCATCGCCGTGGCtATGGGCATCCGGTTCATCATCATGGTGGCCATCTGGAGCGCCGTGTTC
CTGAACAGCCTGTTCAACCAGGAGGTGCAGATCCCCCTGACCGAGAGCTACTGCGGCCCCTGCCCCAAGAACTGG
ATCTGCTACAAGAACAACTGCTACCAGTTCTTCGACGAGAGCAAGAACTGGTACGAGAGCCAGGCCAGCTGCATG
AGCCAGAACGCCAGCCTGCTGAAGGTGTACAGCAAGGAGGACCAGGACCTGCTGAAGCTGGTGAAGAGCTACCAC
TGGATGGGCCTGGTGCACATCCCCACCAACGGCAGCTGGCAGTGGGAGGACGGCAGCATCCTGAGCCCCAACCTG
CTGACCATCATCGAGATGCAGAAGGGCGACTGCGCCCTGTACGCCAGCAGCTTCAAGGGCTACATCGAGAACTGC
AGCACCCCCAACACCTACATCTGCATGCAGCGGACCGTG
SEQ ID NO: 75 (encoding polypeptide of SEQ ID NO: 65/construct 3) 
ATGGCTCTGCCTGTGACAGCTCTGCTGCTGCCTCTGGCTCTGCTGCTGCACGCCGCTAGACCCGATTATAAGGAC
GACGACGACAAGCAGACCACCCCTGGCGAGCGGAGCAGCCTGCCTGCCTTCTACCCTGGCACCAGCGGCAGCTGC
AGCGGCTGCGGCAGCCTGAGCCTGCCCCTGCTGGCtGGCCTGGTGGCCGCCGACGCCGTGGCCAGCCTGCTGATC
GTGGGCGCCGTGTTCTACTTCCTGGGCAGGCTGGTGCCCAGGGGCAGGGGCGCTGCCGAGGCTGCCACCCGGAAG
CAGCGGATCACCGAGACCGAGAGCCCCTACCAGGAGCTGCAGGGCCAGCGGAGCGACGTGTACAGCGACCTGAAC
ACCCAGAGGCCCTACTACAAGCGGAGAAAGCGCtccGGCTCCGGCGAGGGCcgcGGCAGCCTGCTGACCTGCGGC
GACGTGGAAGAGAACCCCGGACCCATGATCCACCTGGGCCACATCCTGTTCCTGCTGCTGCTGCCCGTGGCCGCT
GCCCAAACAACACCCGGCGAGAGATCCTCCTTGCCCGCTTTCTATCCCGGAACATCCGGAAGCTGTtccggaTGT
GGATCCCTTTCTTTGcctttgCTTGCTGGATTGGTCGCAGCTGACGCTGTCGCTTCCCTCCTTATTGTCGGAGCT
GTCTTCCTGTGCGCCAGGCCCAGGCGGAGCCCtGCCCAGGAGGACGGCAAGGTGTACATCAACATGCCCGGCCGG
GGCAGGCGGaagcgctccGGGAGTGGGGCTACCAATTTCTCTCTCCTCAAGCAAGCCGGAGACGTTGAGGAAAAC
CCTGGaCCcATGGCTGGATCCGGGGACGGAGGAGCCGGCACAGCTGGGAGATGAGCGAGTTCCACAACTACAAC
CTGGACCTGAAGAAGAGCGACTTCAGCACCCGGTGGCAGAAGCAGCGGTGCCCCGTGGTGAAGAGCAAGTGCCGG
GAGAACGCCAGCCCCTTCTTCTTCTGCTGCTTCATCGCCGTGGCtATGGGCATCCGGTTCATCATCATGGTGGCC
ATCTGGAGCGCCGTGTTCCTGAACAGCCTGTTCAACCAGGAGGTGCAGATCCCCCTGACCGAGAGCTACTGCGGC
 
CCCTGCCCCAAGAACTGGATCTGCTACAAGAACAACTGCTACCAGTTCTTCGACGAGAGCAAGAACTGGTACGAG
AGCCAGGCCAGCTGCATGAGCCAGAACGCCAGCCTGCTGAAGGTGTACAGCAAGGAGGACCAGGACCTGCTGAAG
CTGGTGAAGAGCTACCACTGGATGGGCCTGGTGCACATCCCCACCAACGGCAGCTGGCAGTGGGAGGACGGCAGC
ATCCTGAGCCCCAACCTGCTGACCATCATCGAGATGCAGAAGGGCGACTGCGCCCTGTACGCCAGCAGCTTCAAG
GGCTACATCGAGAACTGCAGCACCCCCAACACCTACATCTGCATGCAGCGGACCGTG
SEQ ID NO:76 (encoding polypeptide of SEQ ID NO: 66/Construct 8) 
ATGATCCACCTGGGCCACATCCTGTTCCTGCTGCTGCCCGTGGCCGCTGCCCAGACCACCCCTGGCGAGCGG
AGCAGCCTGCCTGCCTTCTACCCTGGCACCAGCGGCAGCTGCAGCGGCTGCGGCAGCCTGAGCCTGCCCCTGCTG
GCCGGCCTGGTGGCCGCCGACGCCGTGGCCAGCCTGCTGATCGTGGGCGCCGTGTTCCTGTGCGCCAGGCCCAGG
CGGAGCCCtGCCCAGGAGGACGGCAAGGTGTACATCAACATGCCCGGCCGGGGCTACTTCCTGGGCAGGCTGGTG
CCCAGGGGCAGGGGCGCTGCCGAGGCTGCCACCCGGAAGCAGCGGATCACCGAGACCGAGAGCCCCTACCAGGAG
CTGCAGGGCCAGCGGAGCGACGTGTACAGCGACCTGAACACCCAGAGGCCCTACTACAAGAGGCGGAAAAGGTCT
GGGAGTGGGGCTACCAATTTCTCTCTCCTCAAGCAAGCCGGAGACGTTGAGGAAAACCCTGGaCCcATGGGCTGG
ATCCGGGGACGGAGGAGCCGGCACAGCTGGGAGATGAGCGAGTTCCACAACTACAACCTGGACCTGAAGAAGAGC
GACTTCAGCACCCGGTGGCAGAAGCAGCGGTGCCCCGTGGTGAAGAGCAAGTGCCGGGAGAACGCCAGCCCCTTC
TTCTTCTGCTGCTTCATCGCCGTGGCtATGGGCATCCGGTTCATCATCATGGTGGCCATCTGGAGCGCCGTGTTC
CTGAACAGCCTGTTCAACCAGGAGGTGCAGATCCCCCTGACCGAGAGCTACTGCGGCCCCTGCCCCAAGAACTGG
ATCTGCTACAAGAACAACTGCTACCAGTTCTTCGACGAGAGCAAGAACTGGTACGAGAGCCAGGCCAGCTGCATG
AGCCAGAACGCCAGCCTGCTGAAGGTGTACAGCAAGGAGGACCAGGACCTGCTGAAGCTGGTGAAGAGCTACCAC
TGGATGGGCCTGGTGCACATCCCCACCAACGGCAGCTGGCAGTGGGAGGACGGCAGCATCCTGAGCCCCAACCTG
CTGACCATCATCGAGATGCAGAAGGGCGACTGCGCCCTGTACGCCAGCAGCTTCAAGGGCTACATCGAGAACTGC
AGCACCCCCAACACCTACATCTGCATGCAGCGGACCGTGAGAAGAAAGAGAAGCGGCAGCGGCGAGGGCAGAGGC
AGCCTGCTGACCTGCGGCGACGTGGAGGAGAACCCCGGACccATGATTCATCTCGGACATATTCTCTTTCTCTTG
CTCTTGCCTGTCGCTGCCGCTCAAACAACTCCCGGAGAAAGATCTTCTCTCCCCGCTTTTTATCCCGGAACATCT
GGATCTTGTTCTGGATGTGGATCTTTGTCTCTCCCTCTCCTCGCTGGACTCGTCGCAGCTGATGCTGTCGCTTCT
CTCTTGATTGTCGGAGCTGTCTTTTTGTGTAAGAGAGGCAGAAAGAAGCTGCTGTACATCTTCAAGCAGCCCTTC
ATGAGACCCGTGCAGACCACCCAGGAGGAGGACGGCTGCAGCTGCAGATTCCCCGAGGAGGAGGAGGGCGGCTGC
GAGCTG
SEQ ID NO:77 (encoding polypeptide of SEQ ID NO: 67/construct 9) 
ATGATCCACCTGGGCCACATCCTGTTCCTGCTGCTGCTGCCCGTGGCCGCTGCCCAGACCACCCCTGGCGAGCGG
AGCAGCCTGCCTGCCTTCTACCCTGGCACCAGCGGCAGCTGCAGCGGCTGCGGCAGCCTGAGCCTGCCCCTGCTG
GCCGGCCTGGTGGCCGCCGACGCCGTGGCCAGCCTGCTGATCGTGGGCGCCGTGTTCCTGTGCGCCAGGCCCAGG
CGGAGCCCtGCCCAGGAGGACGGCAAGGTGTACATCAACATGCCCGGCCGGGGCTACTTCCTGGGCAGGCTGGTG
CCCAGGGGCAGGGGCGCTGCCGAGGCTGCCACCCGGAAGCAGCGGATCACCGAGACCGAGAGCCCCTACCAGGAG
CTGCAGGGCCAGCGGAGCGACGTGTACAGCGACCTGAACACCCAGAGGCCCTACTACAAGAGGCGGAAAAGGTCT
GGGAGTGGGGCTACCAATTTCTCTCTCCTCAAGCAAGCCGGAGACGTTGAGGAAAACCCTGGaCCcATGGGCTGG
ATCCGGGGACGGAGGAGCCGGCACAGCTGGGAGATGAGCGAGTTCCACAACTACAACCTGGACCTGAAGAAGAGC
GACTTCAGCACCCGGTGGCAGAAGCAGCGGTGCCCCGTGGTGAAGAGCAAGTGCCGGGAGAACGCCAGCCCCTTC
TTCTTCTGCTGCTTCATCGCCGTGGCtATGGGCATCCGGTTCATCATCATGGTGGCCATCTGGAGCGCCGTGTTC
 
CTGAACAGCCTGTTCAACCAGGAGGTGCAGATCCCCCTGACCGAGAGCTACTGCGGCCCCTGCCCCAAGAACTGG
ATCTGCTACAAGAACAACTGCTACCAGTTCTTCGACGAGAGCAAGAACTGGTACGAGAGCCAGGCCAGCTGCATG
AGCCAGAACGCCAGCCTGCTGAAGGTGTACAGCAAGGAGGACCAGGACCTGCTGAAGCTGGTGAAGAGCTACCAC
TGGATGGGCCTGGTGCACATCCCCACCAACGGCAGCTGGCAGTGGGAGGACGGCAGCATCCTGAGCCCCAACCTG
CTGACCATCATCGAGATGCAGAAGGGCGACTGCGCCCTGTACGCCAGCAGCTTCAAGGGCTACATCGAGAACTGC
AGCACCCCCAACACCTACATCTGCATGCAGCGGACCGTGAGAAGAAAGAGAAGCGGCAGCGGCGAGGGCAGAGGC
AGCCTGCTGACCTGCGGCGACGTGGAGGAGAACCCCGGACcTatggctctgcctgtgacagctctgctgctgcct
ctggctctgctgctgcacgccgctagacccgattataaggacgacgacgacaagCAAACAACTCCCGGAGAAAGA
TCTTCTCTCCCCGCTTTTTATCCCGGAACATCTGGATCTTGTTCTGGATGTGGATCTTTGTCTCTCCCTCTCCTC
GCTGGACTCGTCGCAGCTGATGCTGTCGCTTCTCTCTTGATTGTCGGAGCTGTCTTTTTGTGTAAGAGAGGCAGA
AAGAAGCTGCTGTACATCTTCAAGCAGCCCTTCATGAGACCCGTGCAGACCACCCAGGAGGAGGACGGCTGCAGC
TGCAGATTCCCCGAGGAGGAGGAGGGCGGCTGCGAGCTG
SEQ ID NO:78 (encoding polypeptide of SEQ ID NO: 68/construct 10) 
ATGATCCACCTGGGCCACATCCTGTTCCTGCTGCTGCCCGTGGCCGCTGCCCAGACCACCCCTGGCGAGCGG
AGCAGCCTGCCTGCCTTCTACCCTGGCACCAGCGGCAGCTGCAGCGGCTGCGGCAGCCTGAGCCTGCCCCTGCTG
GCCGGCCTGGTGGCCGCCGACGCCGTGGCCAGCCTGCTGATCGTGGGCGCCGTGTTCCTGTGCGCCAGGCCCAGG
CGGAGCCCtGCCCAGGAGGACGGCAAGGTGTACATCAACATGCCCGGCCGGGGCTACTTCCTGGGCAGGCTGGTG
CCCAGGGGCAGGGGCGCTGCCGAGGCTGCCACCCGGAAGCAGCGGATCACCGAGACCGAGAGCCCCTACCAGGAG
CTGCAGGGCCAGCGGAGCGACGTGTACAGCGACCTGAACACCCAGAGGCCCTACTACAAGAGGCGGAAAAGGTCT
GGGAGTGGGGCTACCAATTTCTCTCTCCTCAAGCAAGCCGGAGACGTTGAGGAAAACCCTGGaCCcATGGGCTGG
ATCCGGGGACGGAGGAGCCGGCACAGCTGGGAGATGAGCGAGTTCCACAACTACAACCTGGACCTGAAGAAGAGC
GACTTCAGCACCCGGTGGCAGAAGCAGCGGTGCCCCGTGGTGAAGAGCAAGTGCCGGGAGAACGCCAGCCCCTTC
TTCTTCTGCTGCTTCATCGCCGTGGCtATGGGCATCCGGTTCATCATCATGGTGGCCATCTGGAGCGCCGTGTTC
CTGAACAGCCTGTTCAACCAGGAGGTGCAGATCCCCCTGACCGAGAGCTACTGCGGCCCCTGCCCCAAGAACTGG
ATCTGCTACAAGAACAACTGCTACCAGTTCTTCGACGAGAGCAAGAACTGGTACGAGAGCCAGGCCAGCTGCATG
AGCCAGAACGCCAGCCTGCTGAAGGTGTACAGCAAGGAGGACCAGGACCTGCTGAAGCTGGTGAAGAGCTACCAC
TGGATGGGCCTGGTGCACATCCCCACCAACGGCAGCTGGCAGTGGGAGGACGGCAGCATCCTGAGCCCCAACCTG
CTGACCATCATCGAGATGCAGAAGGGCGACTGCGCCCTGTACGCCAGCAGCTTCAAGGGCTACATCGAGAACTGC
AGCACCCCCAACACCTACATCTGCATGCAGCGGACCGTGAGAAGAAAGAAGCGGCAGCGGCGAGGGCAGAGGC
AGCCTGCTGACCTGCGGCGACGTGGAGGAGAACCCCGGACccATGATTCATCTCGGACATATTCTCTTTCTCTTG
CTCTTGCCTGTCGCTGCCGCTCAAACAACTCCCGGAGAAAGATCTTCTCTCCCCGCTTTTTATCCCGGAACATCT
GGATCTTGTTCTGGATGTGGATCTTTGTCTCTCCCTCTCCTCGCTGGACTCGTCGCAGCTGATGCTGTCGCTTCT
CTCTTGATTGTCGGAGCTGTCTTTTTGTGTCAGAGGCGGAAGTACCGGAGCAACAAGGGCGAGAGCCCCGTGGAG
CCTGCCGAGCCCTGCCACTACAGCTGTCCCCGGGAGGAGGAGGGCAGCACCATCCCCATCCAGGAGGACTACCGG
AAGCCCGAGCCTGCCTGCAGCCCC
SEQ ID NO:79 (encoding polypeptide of SEQ ID NO: 69/Construct 11) 
ATGATCCACCTGGGCCACATCCTGTTCCTGCTGCTGCTGCCCGTGGCCGCTGCCCAGACCACCCCTGGCGAGCGG
AGCAGCCTGCCTGCCTTCTACCCTGGCACCAGCGGCAGCTGCAGCGGCTGCGGCAGCCTGAGCCTGCCCCTGCTG
 
GCCGGCCTGGTGGCCGCCGACGCCGTGGCCAGCCTGCTGATCGTGGGCGCCGTGTTCCTGTGCGCCAGGCCCAGG
CGGAGCCCtGCCCAGGAGGACGGCAAGGTGTACATCAACATGCCCGGCCGGGGCTACTTCCTGGGCAGGCTGGTG
CCCAGGGGCAGGGGCGCTGCCGAGGCTGCCACCCGGAAGCAGCGGATCACCGAGACCGAGAGCCCCTACCAGGAG
CTGCAGGGCCAGCGGAGCGACGTGTACAGCGACCTGAACACCCAGAGGCCCTACTACAAGAGGCGGAAAAGGTCT
GGGAGTGGGGCTACCAATTTCTCTCTCCTCAAGCAAGCCGGAGACGTTGAGGAAAACCCTGGaCCcATGGGCTGG
ATCCGGGGACGGAGGAGCCGGCACAGCTGGGAGATGAGCGAGTTCCACAACTACAACCTGGACCTGAAGAAGAGC
GACTTCAGCACCCGGTGGCAGAAGCAGCGGTGCCCCGTGGTGAAGAGCAAGTGCCGGGAGAACGCCAGCCCCTTC
TTCTTCTGCTGCTTCATCGCCGTGGCtATGGGCATCCGGTTCATCATCATGGTGGCCATCTGGAGCGCCGTGTTC
CTGAACAGCCTGTTCAACCAGGAGGTGCAGATCCCCCTGACCGAGAGCTACTGCGGCCCCTGCCCCAAGAACTGG
ATCTGCTACAAGAACAACTGCTACCAGTTCTTCGACGAGAGCAAGAACTGGTACGAGAGCCAGGCCAGCTGCATG
AGCCAGAACGCCAGCCTGCTGAAGGTGTACAGCAAGGAGGACCAGGACCTGCTGAAGCTGGTGAAGAGCTACCAC
TGGATGGGCCTGGTGCACATCCCCACCAACGGCAGCTGGCAGTGGGAGGACGGCAGCATCCTGAGCCCCAACCTG
CTGACCATCATCGAGATGCAGAAGGGCGACTGCGCCCTGTACGCCAGCAGCTTCAAGGGCTACATCGAGAACTGC
AGCACCCCCAACACCTACATCTGCATGCAGCGGACCGTGAGAAGAAAGAAGCGGCAGCGGCGAGGGCAGAGGC
AGCCTGCTGACCTGCGGCGACGTGGAGGAGAACCCCGGACcTatggctctgcctgtgacagctctgctgctgcct
ctggctctgctgctgcacgccgctagacccgattataaggacgacgacgacaagCAAACAACTCCCGGAGAAAGA
TCTTCTCTCCCCGCTTTTTATCCGGAACATCTGGATCTTGTTCTGGATGTGGATCTTTGTCTCTCCCTCTCCTC
GCTGGACTCGTCGCAGCTGATGCTGTCGCTTCTCTCTTGATTGTCGGAGCTGTCTTTTTGTGTCAGAGGCGGAAG
TACCGGAGCAACAAGGGCGAGAGCCCCGTGGAGCCTGCCGAGCCTGCCACTACAGCTGTCCCCGGAGGAGGAG
GGCAGCACCATCCCCATCCAGGAGGACTACCGGAAGCCCGAGCCTGCCTGCAGCCCC
SEQ ID NO: 80 (A20FMDV2 peptide)
   NAVPNLRGDLQVLAQKVART
SEQ ID NO: 81 (CD124 signal peptide)
   MGWLCSGLLFPVSCLVLLQVASSGN
SEQ ID NO: 82 (CD28 aa114-220) 
IEVMYPPPYLDNEKSNGTIIHVKGKHLCPSPLFPGPSKPFWVLVVVGGVLACYSLLVTVAFIIFWVRSKRSRLLH
SDYMNMTPRRPGPTRKHYQPYAPPRDFAAYRS
SEQ ID NO: 83 (CD247 aa52-164) 
RVKFSRSADAPAYQQGQNQLYNELNLGRREEYDVLDKRRGRDPEMGGKPRRKNPQEGLYNELQKDKMAEAYSEIG
MKGERRRGKGHDGLYQGLSTATKDTYDALHMQALPPR
SEQ ID NO: 84 (SEQ ID NO: 1 of WO 2019/182425) 
MGWSCIILFLVATATGVHSQIQLVQSGPELKKPGETVKISCKTSGYTFTDYSMHWVNQAP
GKGLKWMGWINTETGEPTYTDDFKGRFAFSLETSASTAYLQINNLKNEDTATYFCARTAV
 
YWGQGTTLTVSSGSTSGSGKPGSGEGSDIQMTQSPSSLSASLGERVSLTCRASQEISGSL
SWLQQKPDGTIKRLIYAASTLNSGVPKRFSGRRSGSDYSLTISSLESEDFVDYYCLQYSS
YPWSFGGGTKLEIKEPKSPDKTHTCPPCPSHTQPLGVFLFPPKPKDQLMISRTPEVTCVV
VDVSQEDPEVQFNWYVDGVEVHNAKTKPREEQFNSTYRVVSVLTVLHQDWLNGKEYKCKV
SNKGLPSSIEKTISKAKGQPREPQVYTLPPSQEEMTKNQVSLTCLVKGFYPSDIAVEWES
NGQPENNYKTTPPVLDSDGSFFLYSRLTVDKSRWQEGNVFSCSVLHEALHNHYTQKSLSL
SLGKFWVLVVVGGVLACYSLLVTVAFIIFWVARPRRSPAQEDGKVYINMPGRGGRLVPRG
RGAAEAATRKQRITETESPYQELQGQRSDVYSDLNTQRPYYKRVKFSRSADAPAYQQGQN
QLYNELNLGRREEYDVLDKRRGRDPEMGGKPQRRKNPQEGLYNELQKDKMAEAYSEIGMK
GERRRGKGHDGLYQGLSTATKDTYDALHMQALPPR
SEQ ID NO: 85 (SEQ ID NO: 9 of WO 2019/182425)
   ARPRRSPAQEDGKVYINMPGRG
SEQ ID NO: 86 (SEQ ID NO: 11 of WO 2019/182425)
   GRLVPRGRGAAEAATRKQRITETESPYQELQGQRSDVYSDLNTQRPYYK
SEQ ID NO: 87 (sequence of human CXCR2 polypeptide expressed in N1012_CXCR2)
SEQ ID NO: 88 (nucleic acid sequence encoding polypeptide of SEQ ID NO: 87) 
atggaggatttcaatatggagagcgactccttcgaggattttggaagggcgaggacctgtctaactacagctat
agctccacactgccccctttctgctggatgccgccccttgtgagccagagtccctggagatcaacaagtacttc
gtggtcatcatctatgccctggtgtttctgctgtctctgctgggcaatagcctggtcatgctggtcatcctgtac
tccagggtgggccgctctgtgaccgacgtgtatctgctgaatctggccctggccgatctgctgttcgcactgaca
ctgccaatctgggcagcaagcaaggtgaacggctggatcttcggcacctttctgtgcaaggtggtgtctctgctg
aaggaggtgaacttctacagcggcatcctgctgctggcctgtatctccgtggaccggtatctggccatcgtgcac
gccaccaggacactgacccagaagcggtacctggtgaagttcatctgcctgagcatctggggactgtccctgctg
ctggccctgcctgtgctgctgtttaggcgcacagtgtactctagcaacgtgtctccagcctgttatgaggatatg
ggcaacaataccgccaattggaggatgctgctgcgcatcctgccacagagcttcggctttatcgtgcccctgctg
atcatgctgttctgctacggctttacactgcggaccctgttcaaggcccacatgggccagaagcaccgggccatg
agagtgatcttcgccgtggtgctgatctttctgctgtgctggctgccctataacctggtgctgctggccgacaca
ctgatgcggacccaggtcatccaggagacatgcgagcggagaaaccacatcgacagagccctggatgccaccgag
atcctgggcatcctgcactcctgtctgaatcctctgatctatgccttcatcggccagaagtttaggcacggcctg
ctgaagatcctggccatccacggcctgatctccaaggactctctgcccaaggatagccgccccttccttcgtgggc
tcctctagcggccacacctctaccacactg
SEQ ID NO: 89 (nucleic acid sequence of SFG N1012_CXCR2) 
gatccggattagtccaatttgttaaagacaggatatcagtggtccaggctctagttttgact
caacaatatcaccagctgaagcctatagagtacgagccatagataaaataaaagatttatt
tagtctccagaaaaagggggaatgaaagaccccacctgtaggtttggcaagctagcttaag
taacgccattttgcaaggcatggaaaaatacataactgagaatagagaagttcagatcaagg
 
tcaggaacagatggaacagctgaatatgggccaaacaggatatctgtggtaagcagttcctg
ccccggctcagggccaagaacagatggaacagctgaatatgggccaaacaggatatctgtgg
taagcagttcctgccccggctcagggccaagaacagatggtccccagatgcggtccagccct
cagcagtttctagagaaccatcagatgtttccagggtgccccaaggacctgaaatgaccctg
tgccttatttgaactaaccaatcagttcgcttctcgcttctgttcgcgcgcttctgctcccc
gagctcaataaaagagcccacaacccctcactcggggcgccagtcctccgattgactgagtc
gcccgggtacccgtgtatccaataaaccctcttgcagttgcatccgacttgtggtctcgctg
ttccttgggagggtctcctctgagtgattgactacccgtcagcgggggtctttcacatgcag
catgtatcaaaattaatttggtttttttcttaagtatttacattaaatggccatagtactt
aaagttacattggcttccttgaaataaacatggagtattcagaatgtgtcataaatatttct
aattttaagatagtatctccattggctttctacttttctttatttttttttgtcctctgt
cttccatttgttgttgttgttgtttgtttgtttgtttgttggttggttggttaattttttt
taaagatcctacactatagttcaagctagactattagctactctgtaacccagggtgacctt
gaagtcatgggtagcctgctgttttagccttcccacatctaagattacaggtatgagctatc
atttttggtatattgattgattgattgattgatgtgtgtgtgtgtgattgtgtttgtgtgtg
tgattgtgtatatgtgtgtatggttgtgtgtgattgtgtgtatgtatgtttgtgtgtgattg
tgtgtgtgtgattgtgcatgtgtgtgtgtgtgattgtgtttatgtgtatgattgtgtgtgtg
tgtgtgtgtgtgtgtgtgtgtgtgtgtgtgtgtgtgtgttgtgtatatatatttatggtagtga
gaggcaacgctccggctcaggtgtcaggttggttttgagacagagtctttcacttagcttg
gaattcactggccgtcgttttacaacgtcgtgactgggaaaaccctggcgttacccaactta
atcgccttgcagcacatccccctttcgccagctggcgtaatagcgaagaggcccgcaccgat
cgcccttcccaacagttgcgcagcctgaatggcgaatggcgcctgatgcggtattttctcct
tacgcatctgtgcggtatttcacaccgcatatggtgcactctcagtacaatctgctctgatg
ccgcatagttaagccagccccgacacccgccaacacccgctgacgcgccctgacgggcttgt
ctgctcccggcatccgcttacagacaagctgtgaccgtctccgggagctgcatgtgtcagag
gttttcaccgtcatcaccgaaacgcgcgagacgaaagggcctcgtgatacgcctatttttat
aggttaatgtcatgataataatggtttcttagacgtcaggtggcacttttcggggaaatgtg
cgcggaacccctatttgtttatttttctaaatacattcaaatatgtatccgctcatgagaca
ataaccctgataaatgcttcaataatattgaaaaaggaagagtatgagtattcaacatttcc
gtgtcgcccttattcccttttttgcggcattttgccttcctgtttttgctcacccagaaacg
ctggtgaaagtaaaagatgctgaagatcagttgggtgcacgagtgggttacatcgaactgga
tctcaacagcggtaagatccttgagagttttcgccccgaagaacgttttccaatgatgagca
cttttaaagttctgctatgtggcgcggtattatcccgtattgacgccgggcaagagcaactc
ggtcgccgcatacactattctcagaatgacttggttgagtactcaccagtcacagaaaagca
tcttacggatggcatgacagtaagagaattatgcagtgctgccataaccatgagtgataaca
ctgcggccaacttacttctgacaacgatcggaggaccgaaggagctaaccgcttttttgcac
aacatgggggatcatgtaactcgccttgatcgttgggaaccggagctgaatgaagccatacc
aaacgacgagcgtgacaccacgatgcctgtagcaatggcaacaacgttgcgcaaactattaa
ctggcgaactacttactctagcttcccggcaacaattaatagactggatggaggcggataaa
gttgcaggaccacttctgcgctcggcccttccggctggctggtttattgctgataaatctgg
agccggtgagcgtgggtctcgcggtatcattgcagcactggggccagatggtaagccctccc
gtatcgtagttatctacacgacggggagtcaggcaactatggatgaacgaaatagacagatc
gctgagataggtgcctcactgattaagcattggtaactgtcagaccaagtttactcatatat
actttagattgatttaaaacttcatttttaatttaaaaggatctaggtgaagatcctttttg
ataatctcatgaccaaaatcccttaacgtgagttttcgttccactgagcgtcagaccccgta
gaaaagatcaaaggatcttcttgagatcctttttttctgcgcgtaatctgctgcttgcaaac
aaaaaaaccaccgctaccagcggtggtttgtttgccggatcaagagctaccaactctttttc
cgaaggtaactggcttcagcagagcgcagataccaaatactgttcttctagtgtagccgtag
ttaggccaccacttcaagaactctgtagcaccgcctacatacctcgctctgctaatcctgtt
accagtggctgctgccagtggcgataagtcgtgtcttaccgggttggactcaagacgatagt
taccggataaggcgcagcggtcgggctgaacggggggttcgtgcacacagcccagcttggag
 
cgaacgacctacaccgaactgagatacctacagcgtgagctatgagaaagcgccacgcttcc
cgaagggagaaaggcggacaggtatccggtaagcggcagggtcggaacaggagagcgcacga
gggagcttccagggggaaacgcctggtatctttatagtcctgtcgggtttcgccacctctga
cttgagcgtcgatttttgtgatgctcgtcaggggggcggagcctatggaaaaacgccagcaa
cgcggcctttttacggttcctggccttttgctggccttttgctcacatgttctttcctgcgt
tatcccctgattctgtggataaccgtattaccgcctttgagtgagctgataccgctcgccgc
agccgaacgaccgagcgcagcgagtcagtgagcgaggaagcggaagagcgcccaatacgcaa
accgcctctccccgcgcgttggccgattcattaatgcagctggcacgacaggtttcccgact
ggaaagcgggcagtgagcgcaacgcaattaatgtgagttagctcactcattaggcaccccag
gctttacactttatgcttccggctcgtatgttgtgtggaattgtgagcggataacaatttca
cacaggaaacagctatgaccatgattacgccaagctttgctcttaggagtttcctaatacat
cccaaactcaaatatataaagcatttgacttgttctatgccctagggggcgggggaagcta
agccagctttttttaacatttaaaatgttaattccattttaaatgcacagatgtttttattt
cataagggtttcaatgtgcatgaatgctgcaatattcctgttaccaaagctagtataaataa
aaatagataaacgtggaaattacttagagtttctgtcattaacgtttccttcctcagttgac
aacataaatgcgctgctgagaagccagtttgcatctgtcaggatcaatttcccattatgcca
gtcatattaattactagtcaattagttgatttttatttttgacatatacatgtgaaagaccc
cacctgtaggtttggcaagctagcttaagtaacgccattttgcaaggcatggaaaaatacat
aactgagaatagaaaagttcagatcaaggtcaggaacagatggaacagctgaatatgggcca
aacaggatatctgtggtaagcagttcctgccccggctcagggccaagaacagatggaacagc
tgaatatgggccaaacaggatatctgtggtaagcagttcctgccccggctcagggccaagaa
cagatggtccccagatgcggtccagccctcagcagtttctagagaaccatcagatgtttcca
gggtgccccaaggacctgaaatgaccctgtgccttatttgaactaaccaatcagttcgcttc
tcgcttctgttcgcgcgcttctgctccccgagctcaataaaagagcccacaacccctcactc
ggcgcgccagtcctccgattgactgagtcgcccgggtacccgtgtatccaataaaccctctt
gcagttgcatccgacttgtggtctcgctgttccttgggagggtctcctctgagtgattgact
acccgtcagcgggggtctttcatttgggggctcgtccgggatcgggagacccctgcccaggg
accaccgacccaccaccgggaggtaagctggccagcaacttatctgtgtctgtccgattgtc
tagtgtctatgactgattttatgcgcctgcgtcggtactagttagctaactagctctgtatc
tggcggacccgtggtggaactgacgagttcggaacacccggccgcaaccctgggagacgtcc
cagggacttcgggggccgtttttgtggcccgacctgagtcctaaaatcccgatcgtttagga
ctctttggtgcacccccttagaggagggatatgtggttctggtaggagacgagaacctaaa
acagttcccgcctccgtctgaatttttgctttcggtttgggaccgaagccgcgccgcgcgtc
ttgtctgctgcagcatcgttctgtgttgtctctgtctgactgtgtttctgtatttgtctgaa
aatatgggcccgggctagactgttaccactcccttaagtttgaccttaggtcactggaaaga
tgtcgagcggatcgctcacaaccagtcggtagatgtcaagaagagacgttgggttaccttct
gctctgcagaatggccaacctttaacgtcggatggccgcgagacggcacctttaaccgagac
ctcatcacccaggttaagatcaaggtcttttcacctggcccgcatggacacccagaccaggt
cccctacatcgtgacctgggaagccttggcttttgacccccctccctgggtcaagccctttg
tacaccctaagcctccgcctcctcttcctccatccgccccgtctctcccccttgaacctcct
cgttcgaccccgcctcgatcctccctttatccagccctcactccttctctaggcgcccccat
atggccatatgagatcttatatggggcacccccgccccttgtaaacttccctgaccctgaca
tgacaagagttactaacagcccctctctccaagctcacttacaggctctctacttagtccag
cacgaagtctggagacctctggcggcagcctaccaagaacaactggaccgaccggtggtacc
tcacccttaccgagtcggcgacacagtgtgggtccgccgacaccagactaagaacctagaac
ctcgctggaaaggaccttacacagtcctgctgaccacccccaccgccctcaaagtagacggc
atcgcagcttggatacacgccgcccacgtgaaggctgccgaccccgggggtggaccatcctc
tagactgccatgatccacctgggccacatcctgttcctgctgctgctgcccgtggccgctgc
ccagaccacccctggcgagcggagcagcctgcctgccttctaccctggcaccagcggcagct
gcagcggctgcggcagcctgagcctgcccctgctggccggcctggtggccgccgacgccgtg
gccagcctgctgatcgtgggcgccgtgttcctgtgcgccaggcccaggcggagccctgccca
 
ggaggacggcaaggtgtacatcaacatgcccggccggggctacttcctgggcaggctggtgc
ccagggcaggggcgctgccgaggctgccacccggaagcagcggatcaccgagaccgagagc
ccctaccaggagctgcagggccagcggagcgacgtgtacagcgacctgaacacccagaggcc
ctactacaagaggcggaaaaggtctggagtggggctaccaatttctctctcctcaagcaag
ccggagacgttgaggaaaaccctggacccatggctggatccggggacggaggagccggcac
agctggagatgagcgagttccacaactacaacctggacctgaagaagagcgacttcagcac
ccggtggcagaagcagcggtgccccgtggtgaagagcaagtgccgggagaacgccagcccct
tcttcttctgctgcttcatcgccgtggctatgggcatccggttcatcatcatggtggccatc
tggagcgccgtgttcctgaacagcctgttcaaccaggaggtgcagatcccccctgaccgagag
ctactgcggcccctgccccaagaactggatctgctacaagaacaactgctaccagttcttcg
acgagagcaagaactggtacgagagccaggccagctgcatgagccagaacgccagcctgctg
aaggtgtacagcaaggaggaccaggacctgctgaagctggtgaagagctaccactggatggg
cctggtgcacatccccaccaacggcagctggcagtgggaggacggcagcatcctgagcccca
acctgctgaccatcatcgagatgcagaagggcgactgcgccctgtacgccagcagcttcaag
ggctacatcgagaactgcagcacccccaacacctacatctgcatgcagcggacagtgcggag
aaagagatccggatctggagagggaagaggaagcctgctgacctgcggcgacgtggaggaga
acccaggacccatggaggatttcaatatggagagcgactccttcgaggattttggaaggc
gaggacctgtctaactacagctatagctccacactgccccctttctgctggatgccgcccc
ttgtgagccagagtccctggagatcaacaagtacttcgtggtcatcatctatgccctggtgt
ttctgctgtctctgctgggcaatagcctggtcatgctggtcatcctgtactccagggtgggc
cgctctgtgaccgacgtgtatctgctgaatctggccctggccgatctgctgttcgcactgac
actgccaatctggcagcaagcaaggtgaacggctggatcttcggcacctttctgtgcaagg
tggtgtctctgctgaaggaggtgaacttctacagcggcatcctgctgctggcctgtatctcc
gtggaccggtatctggccatcgtgcacgccaccaggacactgacccagaagcggtacctggt
gaagttcatctgcctgagcatctggggactgtccctgctgctggccctgcctgtgctgctgt
ttaggcgcacagtgtactctagcaacgtgtctccagcctgttatgaggatatgggcaacaat
accgccaattggaggatgctgctgcgcatcctgccacagagcttcggctttatcgtgcccct
gctgatcatgctgttctgctacggctttacactgcggaccctgttcaaggcccacatgggcc
agaagcaccgggccatgagagtgatcttcgccgtggtgctgatctttctgctgtgctggctg
ccctataacctggtgctgctggccgacacactgatgcggacccaggtcatccaggagacatg
cgagcggagaaaccacatcgacagagccctggatgccaccgagatcctgggcatcctgcact
cctgtctgaatcctctgatctatgccttcatcggccagaagtttaggcacggcctgctgaag
atcctggccatccacggcctgatctccaaggactctctgcccaaggatagccgccttcctt
cgtgggctcctctagcggccacacctctaccacactgtgacagccactcgag
SEQ ID NO: 90 (protein encoded by SEQ ID NO: 89 (includes: (i) fusion of full length DAP10/DAP12 intracellular domain; (ii) furin cleavage site (RRKR); (iii) SGSG linker; (iv) P2A skip sequence; (v) NKG2D; (vi) furin cleavage site (RRKR); (vii) SGSG linker; (viii) T2A skip sequence; (ix) CXCR2) 
MIHLGHILFLLLLPVAAAQTTPGERSSLPAFYPGTSGSCSGCGSLSLPLLAGLVAADAVASLLIVGAVFLCARPR
RSPAQEDGKVYINMPGRGYFLGRLVPRGRGAAEAATRKQRITETESPYQELQGQRSDVYSDLNTQRPYYKRRKRS
GSGATNFSLLKQAGDVEENPGPMGWIRGRRSRHSWEMSEFHNYNLDLKKSDFSTRWQKQRCPVVKSKCRENASPF
FFCCFIAVAMGIRFIIMVAIWSAVFLNSLFNQEVQIPLTESYCGPCPKNWICYKNNCYQFFDESKNWYESQASCM
SQNASLLKVYSKEDQDLLKLVKSYHWMGLVHIPTNGSWQWEDGSILSPNLLTIIEMQKGDCALYASSFKGYIENC
STPNTYICMQRTVRRKRSGSGEGRGSLLTCGDVEENPGPMEDFNMESDSFEDFWKGEDLSNYSYSSTLPPFLLDA
APCEPESLEINKYFVVIIYALVFLLSLLGNSLVMLVILYSRVGRSVTDVYLLNLALADLLFALTLPIWAASKVNG
WIFGTFLCKVVSLLKEVNFYSGILLLACISVDRYLAIVHATRTLTQKRYLVKFICLSIWGLSLLLALPVLLFRRT
VYSSNVSPACYEDMGNNTANWRMLLRILPQSFGFIVPLLIMLFCYGFTLRTLFKAHMGQKHRAMRVIFAVVLIFL
LCWLPYNLVLLADTLMRTQVIQETCERRNHIDRALDATEILGILHSCLNPLIYAFIGQKFRHGLLKILAIHGLIS
KDSLPKDSRPSFVGSSSGHTSTTL
SEQ ID NO: 91 (encoding the polypeptide of SEQ ID NO: 90) 
ATGATCCACCTGGGCCACATCCTGTTCCTGCTGCTGCTGCCCGTGGCCGCTGCCCAG
ACCACCCCTGGCGAGCGGAGCAGCCTGCCTGCCTTCTACCCTGGCACCAGCGGCAGCTGCAGCGGCTGCGGCAGCCTGAG
CCTGCCCCTGCTGGCCGGCCTGGTGGCCGCCGACGCCGTGGCCAGCCTGCTGATCGTGGGCGCCGTGTTCCTGTGCGCCA
GGCCCAGGCGGAGCCCtGCCCAGGAGGACGGCAAGGTGTACATCAACATGCCCGGCCGGGGCTACTTCCTGGGCAGGCTG
GTGCCCAGGGGCAGGGGCGCTGCCGAGGCTGCCACCCGGAAGCAGCGGATCACCGAGACCGAGAGCCCCTACCAGGAGCT
GCAGGGCCAGCGGAGCGACGTGTACAGCGACCTGAACACCCAGAGGCCCTACTACAAGAGGCGGAAAAGGTCTGGGAGTG
GGGCTACCAATTTCTCTCTCCTCAAGCAAGCCGGAGACGTTGAGGAAAACCCTGGaCCcATGGGCTGGATCCGGGGACGG
AGGAGCCGGCACAGCTGGGAGATGAGCGAGTTCCACAACTACAACCTGGACCTGAAGAAGAGCGACTTCAGCACCCGGTG
GCAGAAGCAGCGGTGCCCCGTGGTGAAGAGCAAGTGCCGGGAGAACGCCAGCCCCTTCTTCTTCTGCTGCTTCATCGCCG
TGGCtATGGGCATCCGGTTCATCATCATGGTGGCCATCTGGAGCGCCGTGTTCCTGAACAGCCTGTTCAACCAGGAGGTG
CAGATCCCCCTGACCGAGAGCTACTGCGGCCCCTGCCCCAAGAACTGGATCTGCTACAAGAACAACTGCTACCAGTTCTT
CGACGAGAGCAAGAACTGGTACGAGAGCCAGGCCAGCTGCATGAGCCAGAACGCCAGCCTGCTGAAGGTGTACAGCAAGG
AGGACCAGGACCTGCTGAAGCTGGTGAAGAGCTACCACTGGATGGGCCTGGTGCACATCCCCACCAACGGCAGCTGGCAG
TGGGAGGACGGCAGCATCCTGAGCCCCAACCTGCTGACCATCATCGAGATGCAGAAGGGCGACTGCGCCCTGTACGCCAG
CAGCTTCAAGGGCTACATCGAGAACTGCAGCACCCCCAACACCTACATCTGCATGCAGCGGACAGTGCGGAGAAAGAGAT
CCGGATCTGGAGAGGGAAGAGGAAGCCTGCTGACCTGCGGCGACGTGGAGGAGAACCCAGGACCCATGGAGGATTTCAAT
ATGGAGAGCGACTCCTTCGAGGATTTTTGGAAGGGCGAGGACCTGTCTAACTACAGCTATAGCTCCACACTGCCCCCTTT
TCTGCTGGATGCCGCCCCTTGTGAGCCAGAGTCCCTGGAGATCAACAAGTACTTCGTGGTCATCATCTATGCCCTGGTGT
TTCTGCTGTCTCTGCTGGGCAATAGCCTGGTCATGCTGGTCATCCTGTACTCCAGGGTGGGCCGCTCTGTGACCGACGTG
TATCTGCTGAATCTGGCCCTGGCCGATCTGCTGTTCGCACTGACACTGCCAATCTGGGCAGCAAGCAAGGTGAACGGCTG
GATCTTCGGCACCTTTCTGTGCAAGGTGGTGTCTCTGCTGAAGGAGGTGAACTTCTACAGCGGCATCCTGCTGCTGGCCT
GTATCTCCGTGGACCGGTATCTGGCCATCGTGCACGCCACCAGGACACTGACCCAGAAGCGGTACCTGGTGAAGTTCATC
TGCCTGAGCATCTGGGGACTGTCCCTGCTGCTGGCCCTGCCTGTGCTGCTGTTTAGGCGCACAGTGTACTCTAGCAACGT
GTCTCCAGCCTGTTATGAGGATATGGGCAACAATACCGCCAATTGGAGGATGCTGCTGCGCATCCTGCCACAGAGCTTCG
GCTTTATCGTGCCCCTGCTGATCATGCTGTTCTGCTACGGCTTTACACTGCGGACCCTGTTCAAGGCCCACATGGGCCAG
AAGCACCGGGCCATGAGAGTGATCTTCGCCGTGGTGCTGATCTTTCTGCTGTGCTGGCTGCCCTATAACCTGGTGCTGCT
GGCCGACACACTGATGCGGACCCAGGTCATCCAGGAGACATGCGAGCGGAGAAACCACATCGACAGAGCCCTGGATGCCA
CCGAGATCCTGGGCATCCTGCACTCCTGTCTGAATCCTCTGATCTATGCCTTCATCGGCCAGAAGTTTAGGCACGGCCTG
CTGAAGATCCTGGCCATCCACGGCCTGATCTCCAAGGACTCTCTGCCCAAGGATAGCCGCCCTTCCTTCGTGGGCTCCTC
TAGCGGCCACACCTCTACCACACTGTGACAGCCACTCGAG
SEQ ID NO: 92 (amino acid sequence of a replica of the Cyad-01 CAR (NKG2D fused to the intracellular domain of CD3ζ) 
MRVKFSRSADAPAYQQGQNQLYNELNLGRREEYDVLDKRRGRDPEMGGKPRRKNPQEGLYNELQKDKMAEAYSEI
GMKGERRRGKGHDGLYQGLSTATKDTYDALHMQALPPRMGWIRGRRSRHSWEMSEFHNYNLDLKKSDFSTRWQKQ
RCPVVKSKCRENASPFFFCCFIAVAMGIRFIIMVAIWSAVFLNSLFNQEVQIPLTESYCGPCPKNWICYKNNCYQ
FFDESKNWYESQASCMSQNASLLKVYSKEDQDLLKLVKSYHWMGLVHIPTNGSWQWEDGSILSPNLLTIIEMQKG
DCALYASSFKGYIENCSTPNTYICMQRTV
SEQ ID NO: 93 (nucleic acid sequence encoding polypeptide of SEQ ID NO: 92) 
ATGAGAGTGAAGTTCAGCAGGAGCGCAGACGCCCcGCGTACCAGCAGGGCCAGAACCAGCTCTATAACGAGCTC
AATCTAGGACGAAGAGAGGAGTACGATGTTTTGGACAAGAGACGTGGCCGGGACCCTGAGATGGGGGGAAAGCCG
AGAAGGAAGAACCCTCAGGAAGGCCTGTACAATGAACTGCAGAAAGATAAGATGGCGGAGGCCTACAGTGAGATT
GGGATGAAAGGCGAGCGCCGGAGGGGCAAGGGGCACGATGGCCTTTACCAGGGTCTCAGTACAGCCACCAAGGAC
ACCTACGATGCATTGCACATGCAGGCCCTGCCCCCTCGCATGGGCTGGATCCGCGGCCGCAGGAGCCGGCACAGC
TGGGAGATGAGCGAGTTCCACAACTACAACCTGGACCTGAAGAAGAGCGACTTCAGCACCCGGTGGCAGAAGCAG
CGGTGCCCCGTGGTGAAGAGCAAGTGCCGGGAGAACGCCAGCCCCTTCTTCTTCTGCTGCTTCATCGCCGTGGCt
ATGGGCATCCGGTTTATAATCATGGTGGCCATCTGGAGCGCCGTGTTCCTGAACAGCCTGTTCAACCAGGAGGTG
CAGATCCCCCTGACCGAGAGCTACTGCGGCCCCTGCCCCAAGAACTGGATCTGCTACAAGAACAACTGCTACCAG
TTCTTCGACGAGAGCAAGAACTGGTACGAGAGCCAGGCCAGCTGCATGAGCCAGAACGCCAGCCTGCTGAAGGTG
TACAGCAAGGAGGACCAGGACCTGCTGAAGCTGGTGAAGAGCTACCACTGGATGGGCCTGGTGCACATCCCCACC
AACGGCAGCTGGCAGTGGGAGGACGGCAGCATCCTGAGCCCCAACCTGCTGACCATCATCGAGATGCAGAAGGGC
GACTGCGCCCTGTACGCCAGCAGCTTCAAGGGCTACATCGAGAACTGCAGCACCCCCAACACCTACATCTGCATG
CAGCGGACCGTGtaa
SEQ ID NO: 94 (nucleic acid sequence encoding CYAD-01_10 - polypeptides of SEQ ID NO: 92 and SEQ ID NO: 1) 
ATGATCCACCTGGGCCACATCCTGTTCCTGCTGCTGCCCGTGGCCGCTGCCCAGACCACCCCTGGCGAGCGG
AGCAGCCTGCCTGCCTTCTACCCTGGCACCAGCGGCAGCTGCAGCGGCTGCGGCAGCCTGAGCCTGCCCCTGCTG
GCCGGCCTGGTGGCCGCCGACGCCGTGGCCAGCCTGCTGATCGTGGGCGCCGTGTTCCTGTGCGCCAGGCCCAGG
CGGAGCCCtGCCCAGGAGGACGGCAAGGTGTACATCAACATGCCCGGCCGGGGCAGGCGGAAAAGGTCTGGGAGT
GGGGCTACCAATTTCTCTCTCCTCAAGCAAGCCGGAGACGTTGAGGAAAACCCTGGaCCcATGAGAGTGAAGTTC
AGCAGGAGCGCAGACGCCCCcGCGTACCAGCAGGGCCAGAACCAGCTCTATAACGAGCTCAATCTAGGACGAAGA
GAGGAGTACGATGTTTTGGACAAGAGACGTGGCCGGGACCCTGAGATGGGGGGAAAGCCGAGAAGGAAGAACCCT
CAGGAAGGCCTGTACAATGAACTGCAGAAAGATAAGATGGCGGAGGCCTACAGTGAGATTGGGATGAAAGGCGAG
CGCCGGAGGGGCAAGGGGCACGATGGCCTTTACCAGGGTCTCAGTACAGCCACCAAGgacacctacgatgcaTTG
CACATGCAGGCCCTGCCCCCTCGCATGGGCTGGATCCGCGGCCGCAGGAGCCGGCACAGCTGGGAGATGAGCGAG
TTCCACAACTACAACCTGGACCTGAAGAAGAGCGACTTCAGCACCCGGTGGCAGAAGCAGCGGTGCCCCGTGGTG
AAGAGCAAGTGCCGGGAGAACGCCAGCCCCTTCTTCTTCTGCTGCTTCATCGCCGTGGCtATGGGCATCCGGTTT
ATAATCATGGTGGCCATCTGGAGCGCCGTGTTCCTGAACAGCCTGTTCAACCAGGAGGTGCAGATCCCCCTGACC
GAGAGCTACTGCGGCCCCTGCCCCAAGAACTGGATCTGCTACAAGAACAACTGCTACCAGTTCTTCGACGAGAGC
AAGAACTGGTACGAGAGCCAGGCCAGCTGCATGAGCCAGAACGCCAGCCTGCTGAAGGTGTACAGCAAGGAGGAC
CAGGACCTGCTGAAGCTGGTGAAGAGCTACCACTGGATGGGCCTGGTGCACATCCCCACCAACGGCAGCTGGCAG
TGGGAGGACGGCAGCATCCTGAGCCCCAACCTGCTGACCATCATCGAGATGCAGAAGGGCGACTGCGCCCTGTAC
GCCAGCAGCTTCAAGGGCTACATCGAGAACTGCAGCACCCCCAACACCTACATCTGCATGCAGCGGACCGTGtaa
SEQ ID NO: 95 (human extracellular NKG2D domain) 
LFNQEVQIPLTESYCGPCPKNWICYKNNCYQFFDESKNWYESQASCMSQNASLLKVYSKEDQDLLKLVKSYHWMG
LVHIPTNGSWQWEDGSILSPNLLTIIEMQKGDCALYASSFKGYIENCSTPNTYICMQRTV
SEQ ID NO: 96 (human extracellular NKG2D domain) 
IWSAVFLNSLFNQEVQIPLTESYCGPCPKNWICYKNNCYQFFDESKNWYESQASCMSQNASLLKVYSKEDQDLLK
LVKSYHWMGLVHIPTNGSWQWEDGSILSPNLLTIIEMQKGDCALYASSFKGYIENCSTPNTYICMQRTV
SEQ ID NO: 97 (SEQ ID NO: 95 minus 8 most N-terminal amino acids) 
PLTESYCGPCPKNWICYKNNCYQFFDESKNWYESQASCMSQNASLLKVYSKEDQDLLKLVKSYHWMGLVHIPTNG
SWQWEDGSILSPNLLTIIEMQKGDCALYASSFKGYIENCSTPNTYICMQRTV
SEQ ID NO: 98 (mouse NKG2D TM domain; UniProt accession no: 054709)
   WRVLAI ALAI RFTLNTLMW LAI
SEQ ID NO: 99 (mouse NKG2D TM domain)
   KISPMFWRVLAIALAIRFTLNTLMWLAIFKETFQPV
SEQ ID NO: 100 (rat NKG2D) 
MSKCHNYDLKPAKWDTSQEHQKQRSALPTSRPGENGIIRRRSSIEELKISPLFVVRVLVA

AMTIRFTVITLTWLAVFITLLCNKEVSVSSREGYCGPCPNDWICHRNNCYQFFNENKAWN

QSQASCLSQNSSLLKIYSKEEQDFLKLVKSYHWMGLVQSPANGSWQWEDGSSLSPNELTL

VKTPSGTCAVYGSSFKAYTEDCSNPNTYICMKRAV
SEQ ID NO: 101 (rat NKG2D TM domain; UniProt accession no: 070215 aa 52-74)
   LFWRVLVAAMTIRFTVITLTWL
SEQ ID NO: 102 (N5 polypeptide) 
MALPVTALLLPLALLLHAARPDYKDDDDKLRPVQAQAQSDCSCSTVSPGVLAGIVMGDLVLTVLIALAVYFLGRL
VPRGRGAAEAATRKQRITETESPYQELQGQRSDVYSDLNTQRPYYKRRKRSGSGEGRGSLLTCGDVEENPGPMIH
LGHILFLLLLPVAAAQTTPGERSSLPAFYPGTSGSCSGCGSLSLPLLAGLVAADAVASLLIVGAVFLCARPRRSP
AQEDGKVYINMPGRGRRKRSGSGATNFSLLKQAGDVEENPGPMKISPMFVVRVLAIALAIRFTLNTLMWLAIFKE
TFQPVLFNQEVQIPLTESYCGPCPKNWICYKNNCYQFFDESKNWYESQASCMSQNASLLKVYSKEDQDLLKLVKS
YHWMGLVHIPTNGSWQWEDGSILSPNLLTIIEMQKGDCALYASSFKGYIENCSTPNTYICMQRTV
SEQ ID NO: 103 (nucleic acid encoding polypeptide of SEQ ID NO: 102) 
ATGGCTCTGCCTGTGACAGCTCTGCTGCTGCCTCTGGCTCTGCTGCTGCACGCCGCTAGACCCGATTATAAGGAC
GACGACGACAAGCTGAGACCCGTGCAGGCCCAGGCCCAGAGCGACTGCAGCTGCAGCACCGTGAGCCCCGGCGTG
CTGGCCGGCATCGTGATGGGCGACCTGGTGCTGACCGTGCTCATCGCCCTTGCCGTGTACTTCCTGGGCAGACTG
GTCCCCAGGGGCAGAGGAGCTGCCGAGGCCGCTACCAGAAAGCAGAGGATCACCGAGACAGAGAGCCCCTACCAG
GAGCTGCAGGGCCAGAGATCCGACGTGTACAGCGACCTCAACACCCAGAGACCCTATTACAAGAGGCGGAAGCGC
TCCGGCTCCGGCGAGGGCCGCGGCAGCCTGCTGACCTGCGGCGACGTGGAAGAGAACCCCGGACCCATGATCCAC
CTGGGCCACATCCTGTTCCTGCTGCTGCTGCCCGTGGCCGCTGCCCAAACAACACCCGGCGAGAGATCCTCCTTG
CCCGCTTTCTATCCCGGAACATCCGGAAGCTGTTCCGGATGTGGATCCCTTTCTTTGCCTTTGCTTGCTGGATTG
GTCGCAGCTGACGCTGTCGCTTCCCTCCTTATTGTCGGAGCTGTCTTCCTGTGCGCCAGGCCCAGGCGGAGCCCT
GCCCAGGAGGACGGCAAGGTGTACATCAACATGCCCGGCCGGGGCAGGCGGAAGCGCTCCGGGAGTGGGGCTACC
AATTTCTCTCTCCTCAAGCAAGCCGGAGACGTTGAGGAAAACCCTGGACCCATGAAAATATCTCCAATGTTCGTT
GTTCGAGTCCTTGCTATAGCCTTGGCAATTCGATTCACCCTTAACACATTGATGTGGCTTGCCATTTTCAAAGAG
ACGTTTCAGCCAGTACTGTTCAACCAGGAGGTGCAGATCCCCCTGACCGAGAGCTACTGCGGCCCCTGCCCAAAA
AATTGGATCTGCTACAAGAACAACTGCTACCAGTTCTTCGACGAGAGCAAGAACTGGTACGAGAGCCAGGCCAGC
TGCATGAGCCAGAACGCCAGCCTGCTGAAGGTGTACAGCAAGGAGGACCAGGACCTGCTGAAGCTGGTGAAGAGC
TACCACTGGATGGGCCTGGTGCACATCCCCACCAACGGCAGCTGGCAGTGGGAGGACGGCAGCATCCTGAGCCCC
AACCTGCTGACCATCATCGAGATGCAGAAGGGCGACTGCGCCCTGTACGCCAGCAGCTTCAAGGGCTACATCGAG
AACTGCAGCACCCCCAACACCTACATCTGCATGCAGCGGACCGTG
SEQ ID NO: 104 (human IgG1 hinge - aa 218-232 of UniProt: P0DOX5)
   EPK SCDKTHTCPP CP
SEQ ID NO: 105 (amino acid sequence of CYAD-01_10 (NKG2D-CD3ζ + ribosomal skip peptide + DAP10))

MIHLGHILFLLLLPVAAAQTTPGERSSLPAFYPGTSGSCSGCGSLSLPLLAGLVAADAVASLLIVGAVFLCARPR
RSPAQEDGKVYINMPGRGRRKRSGSGATNFSLLKQAGDVEENPGPMRVKFSRSADAPAYQQGQNQLYNELNLGRR
EEYDVLDKRRGRDPEMGGKPRRKNPQEGLYNELQKDKMAEAYSEIGMKGERRRGKGHDGLYQGLSTATKDTYDAL
HMQALPPRMGWIRGRRSRHSWEMSEFHNYNLDLKKSDFSTRWQKQRCPVVKSKCRENASPFFFCCFIAVAMGIRF
IIMVAIWSAVFLNSLFNQEVQIPLTESYCGPCPKNWICYKNNCYQFFDESKNWYESQASCMSQNASLLKVYSKED
QDLLKLVKSYHWMGLVHIPTNGSWQWEDGSILSPNLLTIIEMQKGDCALYASSFKGYIENCSTPNTYICMQRTV

## Claims

1. An immunoresponsive cell comprising:
(a) an NKG2D polypeptide;
(b) a fusion polypeptide comprising (i) a DNAX-activating 10 (DAP10) polypeptide, or a functional variant thereof and (ii) a DNAX-activating protein 12 (DAP12) polypeptide, or a functional variant thereof; and
(c) a CXCR2 polypeptide.

2. The immunoresponsive cell of claim 1, wherein each of the NKG2D polypeptide, the DAP10 polypeptide, the DAP12 polypeptide, and the CXCR2 polypeptide is a mammalian polypeptide, optionally wherein each of the NKG2D polypeptide, the DAP 10 polypeptide, the DAP12 polypeptide, and the CXCR2 polypeptide is a human polypeptide.

3. The immunoresponsive cell of any one of the preceding claims, wherein
(i) the amino acid sequence of the NKG2D polypeptide has at least about 85% sequence identity to the sequence of SEQ ID NO: 14, and/or
(ii) the NKG2D polypeptide is a functional variant of the polypeptide having the amino acid sequence of SEQ ID NO: 14, optionally wherein the functional variant
(a) has one or more point mutations that add, delete, or substitute at least one of the amino acids of SEQ ID NO: 14,
(b) is a truncated version of the polypeptide having the amino acid sequence of SEQ ID NO: 14, or
(c) is a chimeric NKG2D polypeptide.

4. The immunoresponsive cell of any one of the preceding claims, wherein the fusion polypeptide has the formula, from N-terminus to C-terminus:
A-B-C-D-E,
wherein
A is an optional N-terminal sequence;
B is a DAP10 polypeptide or functional variant thereof;
C is an optional linker sequence;
D is a DAP12 polypeptide or functional variant thereof; and
E is an optional C-terminal sequence.

5. The immunoresponsive cell of any one of the preceding claims, comprising the functional variant of DAP10 polypeptide, wherein the functional variant of DAP10 polypeptide
(i) comprises an amino acid sequence having at least about 80%, at least about 85%, at least about 90%, at least about 95%, at least about 97%, at least about 98%, or at least about 99% sequence identity to the DAP10 polypeptide of SEQ ID NO: 1;
(ii) is a functional variant of SEQ ID NO: 1 having one or more (i.e. 1, 2, 3, 4, 5, 6, 7, 8, 9, 10 or more) point mutations that add, delete, or substitute any of the amino acids of the DAP 10 polypeptide of SEQ ID NO: 1; or
(iii) is a truncated version of the polypeptide having the amino acid sequence of SEQ ID NO: 1, optionally wherein the truncated version of DAP 10 comprises or consists of amino acids 19-93, 19-69, 1-71, 19-71, 19-48, 49-69, 49-93, or 70-93 of SEQ ID NO: 1.

6. The immunoresponsive cell of any one of claims 1-4, wherein the DAP10 polypeptide or the functional variant thereof comprises or consists of any one of SEQ ID NOs: 1-8.

7. The immunoresponsive cell of any one of the preceding claims, comprising the functional variant of DAP12 polypeptide, wherein the functional variant of DAP12 polypeptide
(i) comprises an amino acid sequence having at least about 80%, at least about 85%, at least about 90%, at least about 95%, at least about 97%, at least about 98%, or at least about 99% sequence identity to the DAP12 polypeptide of SEQ ID NO: 9;
(ii) is a functional variant of SEQ ID NO: 9 having one or more (i.e. 1, 2, 3, 4, 5, 6, 7, 8, 9, 10 or more) point mutations that add, delete, or substitute any of the amino acids of the DAP12 polypeptide of SEQ ID NO: 9; or
(iii) is a truncated version of the polypeptide having the amino acid sequence of SEQ ID NO: 9, optionally wherein the truncated version of DAP12 comprises or consists of amino acids 22-113, 62-113, 22-61, or 41-61 of SEQ ID NO: 9.

8. The immunoresponsive cell of any one of claims 1-6, wherein the DAP12 polypeptide or the functional variant thereof comprises or consists of any one of SEQ ID NOs: 9-13.

9. The immunoresponsive cell of any one of the preceding claims, wherein the DAP10 polypeptide or the functional variant thereof and the DAP12 polypeptide or the functional variant thereof are joined by a linker, optionally wherein the linker comprises or consists of the amino acid sequence recited in any of SEQ ID NOs: 18-46, preferably wherein the linker comprises or consists of the amino acid sequence recited in any of SEQ ID NOs: 33 or 38-44.

10. The immunoresponsive cell of any one of the preceding claims, wherein the fusion polypeptide comprises an N-terminal sequence and/or a C-terminal sequence, optionally wherein the N-terminal or C-terminal sequence comprises one or more of a His-tag, FLAG-tag, Arg-tag, T7-tag, Strep-tag, S-tag, an AviTag^{™}, an aptamer-tag, a myc tag, CD8α leader sequence, a 4-1BB endodomain, a V5 tag, or a CD27 endodomain, preferably wherein the N-terminal or C-terminal sequence comprises one or more of a CD8α leader sequence, a 4-1BB endodomain, or a CD27 endodomain.

11. The immunoresponsive cell of any one of the preceding claims, wherein the fusion polypeptide comprises or consists of the sequence of any one of SEQ ID NOs: 60 to 63.

12. The immunoresponsive cell of any one of claims 1-3, wherein the fusion polypeptide:
(a) does not comprise SEQ ID NO: 84; and/or
(b) does not comprise an anti-EpCAM peptide; and/or
(c) does not comprise SEQ ID NO: 85; and/or
(d) does not comprise SEQ ID NO: 86; and/or
(e) does not comprise both SEQ ID NO: 85 and SEQ ID NO: 86.

13. The immunoresponsive cell of any one of the preceding claims, wherein the fusion polypeptide comprises or consists of full length human DAP 10 fused at its C-terminus to the endodomain of human DAP12 polypeptide, wherein the endodomain is encoded by amino acids 62-113 of human DAP12, and/or, wherein the fusion polypeptide has the sequence of SEQ ID NO: 60.

14. The immunoresponsive cell of any one of the preceding claims, wherein the amino acid sequence of the CXCR2 polypeptide has at least about 85% sequence identity to the sequence of SEQ ID NO: 87, preferably wherein the CXCR2 polypeptide has the amino acid sequence of SEQ ID NO: 87.

15. The immunoresponsive cell of any one of the preceding claims, wherein the immunoresponsive cell is a T-cell or a Natural Killer (NK) cell, preferably wherein the immunoresponsive cell is an αβ T-cell, a γδ T-cell, a CD4⁺ T-cell, a CD8⁺ T-cell, or a Natural Killer T (NKT) cell.

16. A nucleic acid molecule encoding:
(a) an NKG2D polypeptide;
(b) a fusion polypeptide comprising (i) a DNAX-activating 10 (DAP10) polypeptide, or a functional variant thereof and (ii) a DNAX-activating protein 12 (DAP12) polypeptide, or a functional variant thereof; and
(c) a CXCR2 polypeptide.

17. The nucleic acid molecule of claim 16, wherein the nucleic acid molecule is a chimeric polynucleotide, optionally wherein the nucleic acid molecule encodes, from 5' to 3', (i) human DAP 10 in frame with the endodomain of human DAP 12; (ii) a first protease cleavage site; (iii) a first linker; (iv) a first ribosomal skip peptide; (v) human NKG2D; (vi) a second protease cleavage site; (viii) a second linker; (viii) a second ribosomal skip peptide; and (ix) human CXCR2.

18. The nucleic acid molecule of claim 16 or claim 17, wherein the nucleic acid molecule comprises the nucleotide sequence of SEQ ID NO: 88, or has the nucleotide sequence of SEQ ID NO: 91.

19. A vector comprising the nucleic acid sequence of any one of claims 16-18, optionally wherein the vector is a lentiviral vector or a retroviral vector, preferably wherein the vector is a SFG retroviral vector.

20. A method of making the immunoresponsive cell of any one of claims 1-15, comprising the steps of:
(a) transducing a T-cell or a Natural Killer cell with the nucleic acid molecule of any one of claims 16-18 or a vector of claim 19; and
(b) culturing the T-cell or Natural Killer cell such that the transduced cell expresses a NKG2D polypeptide, a DAP10/DAP 12 fusion polypeptide, and a CXCR2 polypeptide, wherein the NKG2D polypeptide and the DAP10/DAP12 fusion polypeptide associate in the cell membrane.

21. The immunoresponsive cell of any one of claims 1-15 for use in the treatment or prophylaxis of cancer, optionally wherein the cancer is a solid tumor cancer, preferably selected from liver cancer, lung cancer, breast cancer, prostate cancer, lymphoid cancer, colon cancer, renal cancer, bone cancer, pancreatic cancer, skin cancer, cancer of the head and neck, cutaneous or intraocular malignant melanoma, uterine cancer, ovarian cancer, rectal cancer, cancer of the anal region, stomach cancer, testicular cancer, uterine cancer, thyroid cancer, cancer of the esophagus, cancer of the small intestine, or any combination thereof.

22. An immunoresponsive cell comprising:
(a) a chimeric NKG2D polypeptide comprising a human NKG2D extracellular domain or a variant thereof and a murine NKG2D transmembrane domain or a variant thereof;
(b) a CXCR2 polypeptide; and
(c) at least one DNAX-activating protein 12 (DAP12) polypeptide or a variant thereof and at least one DNAX-activating protein 10 (DAP10) polypeptide or variant thereof.

23. The immunoresponsive cell of claim 22, wherein (i) the human NKG2D extracellular domain has the sequence set forth in SEQ ID NO: 95, SEQ ID NO: 96, or SEQ ID NO: 97, or is a variant having at least 80% sequence identity to any of SEQ ID NOs: 95, 96, or 97; and/or (ii) the murine NKG2D transmembrane domain has the sequence set forth in SEQ ID NO: 98, SEQ ID NO: 99, SEQ ID NO: 100, or SEQ ID NO: 101, or is a variant having at least 80% sequence identity to any of SEQ ID NOs: 98, 99, 100, or 101.

24. The immunoresponsive cell of claim 22 or claim 23, wherein the cell comprises a polypeptide that has at least 90% sequence identity to the sequence set forth in SEQ ID NO: 102.

## Patentansprüche

1. Immunresponsive Zelle, umfassend:
(a) ein NKG2D-Polypeptid;
(b) ein Fusionspolypeptid, umfassend (i) ein DNAX-aktivierendes 10(DAP10-)Polypeptid oder eine funktionelle Variante davon und (ii) ein DNAX-aktivierendes Protein 12(DAP12-)Polypeptid oder eine funktionelle Variante davon; und
(c) ein CXCR2-Polypeptid.

2. Immunresponsive Zelle nach Anspruch 1, wobei jedes von dem NKG2D-Polypeptid, dem DAP10-Polypeptid, dem DAP12-Polypeptid und dem CXCR2-Polypeptid ein Säugerpolypeptid ist, wobei optional jedes von dem NKG2D-Polypeptid, dem DAP10-Polypeptid, dem DAP12-Polypeptid und dem CXCR2-Polypeptid ein menschliches Polypeptid ist.

3. Immunresponsive Zelle nach einem der vorhergehenden Ansprüche, wobei
(i) die Aminosäuresequenz des NKG2D-Polypeptids zumindest etwa 85 % Sequenzidentität mit der Sequenz von SEQ ID NO: 14 aufweist, und/oder
(ii) das NKG2D-Polypeptid eine funktionelle Variante des Polypeptids mit der Aminosäuresequenz von SEQ ID NO: 14 ist, wobei optional die funktionelle Variante
(a) eine oder mehrere Punktmutationen aufweist, die zumindest eine der Aminosäuren von SEQ ID NO: 14 hinzufügen, löschen oder ersetzen,
(b) eine verkürzte Version des Polypeptids mit der Aminosäuresequenz von SEQ ID NO: 14 ist, oder
(c) ein chimäres NKG2D-Polypeptid ist.

4. Immunresponsive Zelle nach einem der vorhergehenden Ansprüche, wobei das Fusionspolypeptid die folgende Formel von N-Terminus zu C-Terminus aufweist:
A-B-C-D-E,
wobei
A eine optionale N-terminale Sequenz ist;
B ein DAP10-Polypeptid oder eine funktionelle Variante davon ist;
C eine optionale Linkersequenz ist;
D ein DAP12-Polypeptid oder eine funktionelle Variante davon ist; und
E eine optionale C-terminale Sequenz ist.

5. Immunresponsive Zelle nach einem der vorhergehenden Ansprüche, umfassend die funktionelle Variante von DAP10-Polypeptid, wobei die funktionelle Variante von DAP10-Polypeptid
(i) eine Aminosäuresequenz mit zumindest etwa 80 %, zumindest etwa 85 %, zumindest etwa 90 %, zumindest etwa 95 %, zumindest etwa 97 %, zumindest etwa 98 % oder zumindest etwa 99 % Sequenzidentität mit dem DAP10-Polypeptid von SEQ ID NO: 1 umfasst;
(ii) eine funktionelle Variante von SEQ ID NO: 1 mit einer oder mehreren (d. h. 1, 2, 3, 4, 5, 6, 7, 8, 9, 10 oder mehr) Punktmutationen ist, die eine beliebige der Aminosäuren des DAP10-Polypeptids von SEQ ID NO: 1 hinzufügen, löschen oder ersetzen; oder
(iii) eine verkürzte Version des Polypeptids mit der Aminosäuresequenz von SEQ ID NO: 1 ist, wobei optional die verkürzte Version von DAP10 Aminosäuren 19-93, 19-69, 1-71, 19-71, 19-48, 49-69, 49-93 oder 70-93 von SEQ ID NO: 1 umfasst oder daraus besteht.

6. Immunresponsive Zelle nach einem der Ansprüche 1-4, wobei das DAP10-Polypeptid oder die funktionelle Variante davon eine beliebige der SEQ ID NOs: 1-8 umfasst oder daraus besteht.

7. Immunresponsive Zelle nach einem der vorhergehenden Ansprüche, umfassend die funktionelle Variante von DAP12-Polypeptid, wobei die funktionelle Variante von DAP12-Polypeptid
(i) eine Aminosäuresequenz mit zumindest etwa 80 %, zumindest etwa 85 %, zumindest etwa 90 %, zumindest etwa 95 %, zumindest etwa 97 %, zumindest etwa 98 % oder zumindest etwa 99 % Sequenzidentität mit dem DAP12-Polypeptid von SEQ ID NO: 9 umfasst;
(ii) eine funktionelle Variante von SEQ ID NO: 9 mit einer oder mehreren (d. h. 1, 2, 3, 4, 5, 6, 7, 8, 9, 10 oder mehr) Punktmutationen ist, die eine beliebige der Aminosäuren des DAP12-Polypeptids von SEQ ID NO: 9 hinzufügen, löschen oder ersetzen; oder
(iii) eine verkürzte Version des Polypeptids mit der Aminosäuresequenz von SEQ ID NO: 9 ist, wobei optional die verkürzte Version von DAP12 Aminosäuren 22-113, 62-113, 22-61 oder 41-61 von SEQ ID NO: 9 umfasst oder daraus besteht.

8. Immunresponsive Zelle nach einem der Ansprüche 1-6, wobei das DAP12-Polypeptid oder die funktionelle Variante davon eine beliebige der SEQ ID NOs: 9-13 umfasst oder daraus besteht.

9. Immunresponsive Zelle nach einem der vorhergehenden Ansprüche, wobei das DAP10-Polypeptid oder die funktionelle Variante davon und das DAP12-Polypeptid oder die funktionelle Variante davon durch einen Linker verbunden sind, wobei optional der Linker die Aminosäuresequenz, die in einer beliebigen von SEQ ID NOs: 18-46 genannt ist, umfasst oder daraus besteht, wobei bevorzugt der Linker die Aminosäuresequenz, die in einer beliebigen von SEQ ID NOs: 33 oder 38-44 genannt ist, umfasst oder daraus besteht.

10. Immunresponsive Zelle nach einem der vorhergehenden Ansprüche, wobei das Fusionspolypeptid eine N-terminale Sequenz und/oder eine C-terminale Sequenz umfasst, wobei optional die N-terminale oder C-terminale Sequenz eines oder mehrere von einem His-Tag, FLAG-Tag, Arg-Tag, T7-Tag, Strep-Tag, S-Tag, einem AviTag^{™}, einem Aptamer-Tag, einem myc-Tag, CD8α-Leadersequenz, einer 4-1BB-Endodomäne, einem V5-Tag oder einer CD27-Endodomäne umfasst, wobei bevorzugt die N-terminale oder C-terminale Sequenz eines oder mehrere von einer CD8α-Leadersequenz, einer 4-1BB-Endodomäne oder einer CD27-Endodomäne umfasst.

11. Immunresponsive Zelle nach einem der vorhergehenden Ansprüche, wobei das Fusionspolypeptid die Sequenz von einer beliebigen von SEQ ID NOs: 60 bis 63 umfasst oder daraus besteht.

12. Immunresponsive Zelle nach einem der Ansprüche 1-3, wobei das Fusionspolypeptid:
(a) nicht SEQ ID NO: 84 umfasst; und/oder
(b) kein Anti-EpCAM-Peptid umfasst; und/oder
(c) nicht SEQ ID NO: 85 umfasst; und/oder
(d) nicht SEQ ID NO: 86 umfasst; und/oder
(e) nicht sowohl SEQ ID NO: 85 als auch SEQ ID NO: 86 umfasst.

13. Immunresponsive Zelle nach einem der vorhergehenden Ansprüche, wobei das Fusionspolypeptid menschliches DAP10 voller Länge umfasst oder daraus besteht, das an seinem C-Terminus mit der Endodomäne des menschlichen DAP12-Polypeptids kondensiert ist, wobei die Endodomäne durch Aminosäuren 62-113 des menschlichen DAP12 codiert ist und/oder wobei das Fusionspolypeptid die Sequenz von SEQ ID NO: 60 aufweist.

14. Immunresponsive Zelle nach einem der vorhergehenden Ansprüche, wobei die Aminosäuresequenz des CXCR2-Polypeptids zumindest etwa 85 % Sequenzidentität mit der Sequenz von SEQ ID NO: 87 aufweist, wobei bevorzugt das CXCR2-Polypeptid die Aminosäuresequenz von SEQ ID NO: 87 aufweist.

15. Immunresponsive Zelle nach einem der vorhergehenden Ansprüche, wobei die immunresponsive Zelle eine T-Zelle oder eine natürliche Killer(NK-)Zelle ist, wobei bevorzugt die immunresponsive Zelle eine aβ-T-Zelle, eine γδ-T-Zelle, eine CD4⁺-T-Zelle, eine CD8⁺-T-Zelle oder eine natürliche Killer-T(NKT-)Zelle ist.

16. Nukleinsäuremolekül, codierend:
(a) ein NKG2D-Polypeptid;
(b) ein Fusionspolypeptid, umfassend (i) ein DNAX-aktivierendes 10(DAP10-)Polypeptid oder eine funktionelle Variante davon und (ii) ein DNAX-aktivierendes Protein 12(DAP12-)Polypeptid oder eine funktionelle Variante davon; und
(c) ein CXCR2-Polypeptid.

17. Nukleinsäuremolekül nach Anspruch 16, wobei das Nukleinsäuremolekül ein chimäres Polynukleotid ist, wobei optional das Nukleinsäuremolekül von 5' bis 3' (i) menschliches DAP10 im Leseraster mit der Endodomäne von menschlichem DAP12; (ii) eine erste Protease-Spaltstelle; (iii) einen ersten Linker; (iv) ein erstes ribosomales Skip-Peptid; (v) menschliches NKG2D; (vi) eine zweite Protease-Spaltstelle; (vii) einen zweiten Linker; (viii) ein zweites ribosomales Skip-Peptid; und (ix) menschliches CXCR2 codiert.

18. Nukleinsäuremolekül nach Anspruch 16 oder Anspruch 17, wobei das Nukleinsäuremolekül die Nukleotidsequenz von SEQ ID NO: 88 umfasst oder die Nukleotidsequenz von SEQ ID NO: 91 aufweist.

19. Vektor, der die Nukleinsäuresequenz nach einem der Ansprüche 16-18 umfasst, wobei optional der Vektor ein lentiviraler Vektor oder ein retroviraler Vektor ist, wobei bevorzugt der Vektor ein retroviraler SFG-Vektor ist.

20. Verfahren zur Herstellung der immunresponsiven Zelle nach einem der Ansprüche 1-15, umfassend die folgenden Schritte:
(a) Transduzieren einer T-Zelle oder einer natürlichen Killerzelle mit dem Nukleinsäuremolekül nach einem der Ansprüche 16-18 oder einem Vektor nach Anspruch 19; und
(b) Kultivieren der T-Zelle oder der natürlichen Killerzelle, sodass die transduzierte Zelle ein NKG2D-Polypeptid, ein DAP10/DAP12-Fusionspolypeptid und ein CXCR2-Polypeptid exprimiert, wobei das NKG2D-Polypeptid und das DAP10/DAP12-Fusionspolypeptid in der Zellmembran assoziieren.

21. Immunresponsive Zelle nach einem der Ansprüche 1-15 zur Verwendung bei der Behandlung oder Prophylaxe von Krebs, wobei optional der Krebs ein solider Tumorkrebs ist, bevorzugt ausgewählt aus Leberkrebs, Lungenkrebs, Brustkrebs, Prostatakrebs, Lymphdrüsenkrebs, Dickdarmkrebs, Nierenkrebs, Knochenkrebs, Bauchspeicheldrüsenkrebs, Hautkrebs, Krebs von Kopf und Hals, kutanem oder intraokularem malignen Melanom, Gebärmutterkrebs, Eierstockkrebs, Rektumkrebs, Krebs der Analregion, Magenkrebs, Hodenkrebs, Gebärmutterkrebs, Schilddrüsenkrebs, Krebs der Speiseröhre, Krebs des Dünndarms oder einer beliebigen Kombination davon.

22. Immunresponsive Zelle, umfassend:
(a) ein chimäres NKG2D-Polypeptid, das eine menschliche extrazelluläre NKG2D-Domäne oder eine Variante davon und eine murine NKG2D-Transmembrandomäne oder eine Variante davon umfasst;
(b) ein CXCR2-Polypeptid; und
(c) zumindest ein DNAX-aktivierendes Protein 12(DAP12-)Polypeptid oder eine Variante davon und zumindest ein DNAX-aktivierendes Protein 10(DAP10-)Polypeptid oder eine Variante davon.

23. Immunresponsive Zelle nach Anspruch 22, wobei (i) die menschliche extrazelluläre NKG2D-Domäne die Sequenz aufweist, die in SEQ ID NO: 95, SEQ ID NO: 96 oder SEQ ID NO: 97 dargelegt ist, oder eine Variante mit zumindest 80 % Sequenzidentität zu einer beliebigen von SEQ ID NOs: 95, 96 oder 97 ist;
und/oder (ii) die murine NKG2D-Transmembrandomäne die Sequenz aufweist, die in SEQ ID NO: 98, SEQ ID NO: 99, SEQ ID NO: 100 oder SEQ ID NO: 101 dargelegt ist, oder eine Variante mit zumindest 80 % Sequenzidentität zu einer beliebigen von SEQ ID NOs: 98, 99, 100 oder 101 ist.

24. Immunresponsive Zelle nach Anspruch 22 oder Anspruch 23, wobei die Zelle ein Polypeptid umfasst, das zumindest 90 % Sequenzidentität mit der Sequenz aufweist, die in SEQ ID NO: 102 dargelegt ist.

## Revendications

1. Cellule immunoréactive comprenant :
(a) un polypeptide NKG2D ;
(b) un polypeptide de fusion comprenant (i) un polypeptide d'activation de DNAX 10 (DAP10), ou un variant fonctionnel de celui-ci et (ii) un polypeptide de la protéine 12 d'activation de DNAX (DAP12), ou un variant fonctionnel de celui-ci ; et
(c) un polypeptide CXCR2.

2. Cellule immunoréactive selon la revendication 1, chaque polypeptide parmi le polypeptide NKG2D, le polypeptide DAP10, le polypeptide DAP12 et le polypeptide CXCR2 étant un polypeptide mammifère, éventuellement chaque polypeptide parmi le polypeptide NKG2D, le polypeptide DAP 10, le polypeptide DAP 12 et le polypeptide CXCR2 étant un polypeptide humain.

3. Cellule immunoréactive selon l'une quelconque des revendications précédentes,
(i) la séquence d'acides aminés du polypeptide NKG2D comportant une identité de séquence d'au moins 85 % avec la SEQ ID n° : 14, et/ou
(ii) le polypeptide NKG2D étant un variant fonctionnel du polypeptide comportant la séquence d'acides aminés de SEQ ID n° : 14, éventuellement ledit variant fonctionnel
(a) comportant une ou plusieurs mutations ponctuelles qui ajoutent, suppriment ou substituent au moins l'un des acides aminés de SEQ ID n° : 14,
(b) étant une version tronquée du polypeptide comportant la séquence d'acides aminés de SEQ ID n° : 14, ou
(c) étant un polypeptide NKG2D chimérique.

4. Cellule immunoréactive selon l'une quelconque des revendications précédentes, ledit polypeptide de fusion comportant la formule de la terminaison N à la terminaison C :
A-B-C-D-E,
dans laquelle
A représente une séquence N-terminale éventuelle ;
B représente un polypeptide DAP10 ou un variant fonctionnel de celui-ci ;
C représente une séquence de lieur éventuelle ;
D représente un polypeptide DAP12 ou un variant fonctionnel de celui-ci ; et
E représente une séquence C-terminale éventuelle.

5. Cellule immunoréactive selon l'une quelconque des revendications précédentes, comprenant le variant fonctionnel du polypeptide DAP10, ledit variant fonctionnel du polypeptide DAP10
(i) comprenant une séquence d'acides aminés comportant une identité de séquence d'au moins environ 80 %, d'au moins environ 85 %, d'au moins environ 90 %, d'au moins environ 95 %, d'au moins environ 97 %, d'au moins environ 98 %, ou d'au moins environ 99 % avec le polypeptide DAP10 de SEQ ID n° : 1 ;
(ii) étant un variant fonctionnel de SEQ ID n° : 1 comportant une ou plusieurs (c.-à-d. 1, 2, 3, 4, 5, 6, 7, 8, 9, 10 ou plus) mutations ponctuelles qui ajoutent, suppriment ou substituent l'un quelconque des acides aminés du polypeptide DAP10 de SEQ ID n° : 1 ; ou
(iii) une version tronquée du polypeptide comportant la séquence d'acides aminés de SEQ ID n° : 1, éventuellement ladite version tronquée de DAP10 comprenant ou étant constitué des acides aminés 19 à 93, 19 à 69, 1 à 71, 19 à 71, 19 à 48, 49 à 69, 49 à 93, ou 70 à 93 de SEQ ID n° : 1.

6. Cellule immunoréactive selon l'une quelconque des revendications 1 à 4, ledit polypeptide DAP10 ou ledit variant fonctionnel de celui-ci comprenant ou étant constitué de l'une quelconque des SEQ ID n° : 1 à 8.

7. Cellule immunoréactive selon l'une quelconque des revendications précédentes, comprenant le variant fonctionnel du polypeptide DAP12, ledit variant fonctionnel du polypeptide DAP12
(i) comprenant une séquence d'acides aminés comportant une identité de séquence d'au moins environ 80 %, d'au moins environ 85 %, d'au moins environ 90 %, d'au moins environ 95 %, d'au moins environ 97 %, d'au moins environ 98 %, ou d'au moins environ 99 % avec le polypeptide DAP12 de SEQ ID n° : 9 ;
(ii) étant un variant fonctionnel de SEQ ID n° : 9 comportant une ou plusieurs (c.-à-d. 1, 2, 3, 4, 5, 6, 7, 8, 9, 10 ou plus) mutations ponctuelles qui ajoutent, suppriment ou substituent l'un quelconque des acides aminés du polypeptide DAP12 de SEQ ID n° : 9 ; ou
(iii) étant une version tronquée du polypeptide comportant la séquence d'acides aminés de SEQ ID n° : 9, éventuellement ladite version tronquée de DAP12 comprenant ou étant constituée des acides aminés 22 à 113, 62 à 113, 22 à 61, ou 41 à 61 de SEQ ID n° : 9.

8. Cellule immunoréactive selon l'une quelconque des revendications 1 à 6, ledit polypeptide DAP12 ou ledit variant fonctionnel de celui-ci comprenant ou étant constitué de l'une quelconque des SEQ ID n° : 9 à 13.

9. Cellule immunoréactive selon l'une quelconque des revendications précédentes, ledit polypeptide DAP10 ou ledit variant fonctionnel de celui-ci et ledit polypeptide DAP12 ou ledit variant fonctionnel de celui-ci étant joints par un lieur, éventuellement ledit lieur comprenant ou étant constitué de la séquence d'acides aminés énumérée dans l'une quelconque des SEQ ID n° : 18 à 46, de préférence ledit lieur comprenant ou étant constitué de la séquence d'acides aminés énumérée dans l'une quelconque des SEQ ID n° : 33 ou 38 à 44.

10. Cellule immunoréactive selon l'une quelconque des revendications précédentes, ledit polypeptide de fusion comprenant une séquence N-terminale et/ou une séquence C-terminale, éventuellement ladite séquence N-terminale ou C-terminale comprenant un ou plusieurs éléments parmi une étiquette His, une étiquette FLAG, une étiquette Arg, une étiquette T7, une étiquette Strep, une étiquette S, un AviTag^{™}, une étiquette aptamère, une étiquette myc, une séquence de tête de CD8α, un endodomaine de 4-1BB, une étiquette V5 ou endodomaine de CD27, de préférence ladite séquence N-terminale ou C-terminale comprenant un ou plusieurs éléments parmi une séquence de tête de CD8α, un endodomaine de 4-1BB ou un endodomaine de CD27.

11. Cellule immunoréactive selon l'une quelconque des revendications précédentes, ledit polypeptide de fusion comprenant ou étant constitué de l'une quelconque des SEQ ID n° : 60 à 63.

12. Cellule immunoréactive selon l'une quelconque des revendications 1 à 3, ledit polypeptide de fusion :
(a) ne comprenant pas la SEQ ID n° : 84 ; et/ou
(b) ne comprenant pas un peptide anti-EpCAM ; et/ou
(c) ne comprenant pas la SEQ ID n° : 85 ; et/ou
(d) ne comprenant pas la SEQ ID n° : 86 ; et/ou
(e) ne comprenant pas la SEQ ID n° : 85 et la SEQ ID n° : 86.

13. Cellule immunoréactive selon l'une quelconque des revendications précédentes, ledit polypeptide de fusion comprenant ou étant constitué d'un DAP10 humain pleine longueur fusionné au niveau de sa terminaison C à l'endodomaine du polypeptide DAP12 humain, ledit endodomaine étant codé par les acides aminés 62 à 113 du DAP12 humain, et/ou ledit polypeptide de fusion comportant la séquence de SEQ ID n° : 60.

14. Cellule immunoréactive selon l'une quelconque des revendications précédentes, ladite séquence d'acides aminés du polypeptide CXCR2 comportant une identité de séquence d'au moins environ 85 % avec la SEQ ID n° : 87, de préférence ledit polypeptide CXCR2 comportant la séquence d'acides aminés de SEQ ID n° : 87.

15. Cellule immunoréactive selon l'une quelconque des revendications précédentes, ladite cellule immunoréactive étant un lymphocyte T ou une cellule NK (Natural Killer), de préférence ladite cellule immunoréactive étant un lymphocyte T αβ, un lymphocyte T γδ, un lymphocyte T CD4⁺, un lymphocyte T CD8⁺, ou un lymphocyte T Natural Killer (NKT).

16. Molécule d'acide nucléique codant :
(a) un polypeptide NKG2D ;
(b) un polypeptide de fusion comprenant (i) un polypeptide d'activation de DNAX 10 (DAP10), ou un variant fonctionnel de celui-ci et (ii) un polypeptide de la protéine 12 d'activation de DNAX (DAP12), ou un variant fonctionnel de celui-ci ; et
(c) un polypeptide CXCR2.

17. Molécule d'acide nucléique selon la revendication 16, ladite molécule d'acide nucléique étant un polynucléotide chimérique, éventuellement ladite molécule d'acide nucléique codant, de 5' à 3', (i) le DAP10 humain dans le cadre avec l'endodomaine du DAP12 humain ; (ii) un premier site de clivage de protéase ; (iii) un premier lieur ; (iv) un premier peptide skip ribosomique ; (v) un NKG2D humain ; (vi) un second site de clivage de protéase ; (vii) un second lieur ; (viii) un second peptide skip ribosomique ; et (ix) un CXCR2 humain.

18. Molécule d'acide nucléique selon la revendication 16 ou la revendication 17, ladite molécule d'acide nucléique comprenant la séquence nucléotidique de SEQ ID n°: 88, ou comportant la séquence nucléotidique de SEQ ID n° : 91.

19. Vecteur comprenant la séquence d'acide nucléique selon l'une quelconque des revendications 16 à 18, éventuellement ledit vecteur étant un vecteur lentiviral ou un vecteur rétroviral, de préférence ledit vecteur étant un vecteur rétroviral SFG.

20. Procédé de fabrication de la cellule immunoréactive selon l'une quelconque des revendications 1 à 15, comprenant les étapes de :
(a) transduction d'un lymphocyte T ou d'une cellule Natural Killer avec la molécule d'acide nucléique selon l'une quelconque des revendications 16 à 18 ou un vecteur selon la revendication 19 ; et
(b) culture du lymphocyte T ou de la cellule Natural Killer de sorte que la cellule transduite exprime un polypeptide NKG2D, un polypeptide de fusion DAP10/DAP12 et un polypeptide CXCR2, ledit polypeptide NKG2D et ledit polypeptide de fusion DAP10/DAP12 s'associant dans la membrane cellulaire.

21. Cellule immunoréactive selon l'une quelconque des revendications 1 à 15 destinée à être utilisée dans le traitement ou la prophylaxie d'un cancer, éventuellement ledit cancer étant un cancer à tumeur solide, de préférence choisi parmi le cancer du foie, le cancer du poumon, le cancer du sein, le cancer de la prostate, un cancer lymphoïde, le cancer du côlon, le cancer du rein, le cancer des os, le cancer du pancréas, le cancer de la peau, le cancer de la tête et du cou, un mélanome malin cutané ou intraoculaire, le cancer de l'utérus, le cancer de l'ovaire, le cancer du rectum, le cancer de la région anale, le cancer de l'estomac, le cancer des testicules, le cancer de l'utérus, le cancer de la thyroïde, le cancer de l'oesophage, le cancer de l'intestin grêle ou toute combinaison de ceux-ci.

22. Cellule immunoréactive comprenant :
(a) un polypeptide NKG2D chimérique comprenant un domaine extracellulaire de NKG2D humain ou un variant de celui-ci et un domaine transmembranaire de NKG2D murin ou un variant de celui-ci ;
(b) un polypeptide CXCR2 ; et
(c) au moins un polypeptide de la protéine 12 d'activation de DNAX (DAP12), ou un variant de celui-ci et au moins un polypeptide de la protéine 10 d'activation de DNAX (DAP10), ou un variant de celui-ci.

23. Cellule immunoréactive selon la revendication 22, (i) ledit domaine extracellulaire de NKG2D humain comportant la séquence décrite dans SEQ ID n° : 95, SEQ ID n° : 96 ou SEQ ID n° : 97, ou étant un variant comportant une identité de séquence d'au moins 80 % avec la SEQ ID n° : 95, 96 ou 97 ;
et/ou (ii) ledit domaine transmembranaire de NKG2D murin comportant la séquence décrite dans SEQ ID n° : 98, SEQ ID n° : 99, SEQ ID n° : 100 ou SEQ ID n° : 101, ou étant un variant comportant une identité de séquence d'au moins 80 % avec la SEQ ID n° : 98, 99, 100 ou 101.

24. Cellule immunoréactive selon la revendication 22 ou la revendication 23, ladite cellule comprenant un polypeptide qui comporte une identité de séquence d'au moins 90% avec la séquence décrite dans la SEQ ID n° : 102.
